# EUROPEAN PATENT SPECIFICATION

(11) **EP 3 641 811 B1**
(45) Date of publication and mention of the grant of the patent: **26.02.2025**
(21) Application number: 18821467.0
(22) Date of filing: 21.06.2018
(51) Int. Cl.: C12N 5/071, C12N 5/10, A61K 35/22, A61K 9/00, A61K 47/42, A61P 13/12

(54) **IMMUNOPRIVILEGED BIOACTIVE RENAL CELLS FOR THE TREATMENT OF KIDNEY DISEASE**
IMMUNPRIVILEGIERTE BIOAKTIVE NIERENZELLEN ZUR BEHANDLUNG VON NIERENKRANKHEIT
CELLULES RÉNALES BIOACTIVES À IMMUNO-PRIVILÈGE POUR LE TRAITEMENT D'UNE MALADIE RÉNALE

(30) Priority: 21.06.2017 US 201762523241 P
(43) Date of publication of application: 29.04.2020
(60) Divisional application: 25153615.7
(73) Proprietor: ProKidney, Grand Cayman KY1-9006 (KY)
(72) Inventor: BASU, Joydeep, Morrisville, NC 27560 (US); LUDLOW, John, W., Chapel Hill, NC 27517 (US); BERTRAM, Timothy, A., Camana Bay Grand Cayman KY1-9006/KY (KY); JAIN, Deepak, Winston-Salem, NC 27103 (US)
(74) Representative: Mewburn Ellis LLP
(86) International application number: PCT/US2018/038801
(87) International publication number: WO 2018/237170

(56) References cited:
- WO-A1-2011/143499
- WO-A1-2016/094679
- WO-A1-2016/201047
- WO-A1-2017/173043
- WO-A2-2016/073955
- US-A1- 2015 246 073
- US-A1- 2016 206 659

## Description

This application claims benefit of priority to U.S. Provisional Application No. 62/523,241, filed June 21, 2017.

### FIELD OF THE INVENTION

The present invention relates to methods for developing a genomically modified, bioactive renal cell and its use for treating subjects with kidney disease,

### BACKGROUND

Renal cell-based therapy has attracted recent interest as regeneration of tissues and organs is now within the technological reach of modern medicine (Ludlow et al. The Future of Regenerative Medicine: Urinary System. Tissue Engineering. 2012; 18:218-24). New treatment paradigms involving tissue engineering and cellular-based applications have been described that provide substantial and durable augmentation of kidney functions, slow progression of disease and improve quality of life in this patient population. These next-generation regenerative medicine technologies provide isolated renal cells as a therapeutic option for chronic kidney disease (CKD). Presnell et al. WO/2010/056328 and Ilagan et al. PCT/US2011/036347 describe isolated bioactive renal cells, and methods of isolating and culturing the same, as well as methods of treatment with the cell populations. Injection of these bioactive renal cells into recipient kidneys has resulted in significant improvement in animal survival and kidney function, as evidenced for example by urine concentration and filtration functions, in nonclinical studies.

The current protocol for treatment of patients using selected renal cells is based on autologous cell transfer. In this approach, bioactive renal cells are recovered from patients, selected *ex vivo,* cultivated *in vitro* in order to amplify the number of cells if necessary and finally infused into the patient. Each patient receives an individually fabricated treatment, using the patient's own renal cells (*i.e.* an autologous therapy). Autologous therapies face substantial technical and logistic hurdles to practical application, their generation requires expensive dedicated facilities and expert personnel, they must be generated in a short time following a patient's diagnosis, and in some cases, depending on the extent of disease progression, the patient's cells may be poorly functional and/or present in very low numbers. Because of these hurdles, autologous cell preparations can have substantial variations in efficacy and safety.

### SUMMARY OF THE INVENTION

Provided herein are, methods for producing bioactive renal cells with reduced immunogenicity and their use in treating subjects with kidney disease. In certain cases cells derived from a donor that would not normally be administered to a patient (*e.g.,* due to likely inflammation and immune system rejection) are genetically modified to be useful (*e.g.,* such that the cells will persist and promote improved kidney function) in the patient. Also described are bioactive renal cells produced by the methods herein. The invention is defined by the appended claims. The invention includes is a method of producing a genomically modified bioactive renal cell (BRC) (*i.e.,* a BRC with a genetic modification in the genome thereof). In certain embodiments, the genetically modified BRC comprises an exogenous polynucleotide (such as a plasmid or a viral vector) that expresses an RNA interference (RNAi) molecule that reduces expression of a genomic immunogenicity gene in a BRC. In certain embodiments, the RNAi molecule is a short interfering or a short hairpin RNA the invention molecule. In the invention the method includes genetically modifying a genomic immunogenicity gene in a BRC wherein

the gene encodes a protein within a major histocompatibility complex (MHC) class I molecule and/or a MHC class II molecule. In certain embodiments, the gene is a beta-2 microglobulin (B2M also known as β2M), human leukocyte antigen (HLA)-A, HLA-B, HLA-C, HLA-DRA, HLA-DRB1, HLA-DRB3, HLA-DRB4, HLA-DRB5, HLA-DPA1, HLA-DPA2, HLA-DQA1, or HLA-DQB1 gene.

In certain embodiments, the gene encodes a minor histocompatibility antigen (MiHA or mHA). In certain embodiments, the gene is a HA-1, HA-2, HA-8, HB-1, HY-A1, HY-A2, HY-B7, HY-B8, HY-B60, or HY-DQ5 gene.

In embodiments, any allelic variant of a gene mentioned herein (*e.g.,* an HLA gene, B2M, or an mHA gene) may be modified (*e.g.,* deleted) or targeted with RNA interference.

In certain embodiments, genetically modifying the gene comprises mutating the gene. In certain embodiments, mutating the gene comprises deleting the gene or a portion thereof.

In certain embodiments, genetically modifying a cell includes mutating any combination of two or more of a B2M, HLA-A, HLA-B, HLA-C, HLA-DRA, HLA-DRB1, HLA-DRB3, HLA-DRB4, HLA-DRB5, HLA-DPA1, HLA-DPA2, HLA-DQA1, and/or HLA-DQB1 gene.

In certain embodiments, the genetically modified BRC is a genetically modified primary renal cell. In certain embodiments, the genetically modified primary renal cell has been passaged at least about 1, 2, 3, 4, 5 or more times before or after genetic modification. In certain embodiments, the method further includes obtaining SRCs from the genetically modified BRCs. In certain embodiments, SRCs are obtained, and then genetically modified.

In certain embodiments, the BRC is in a population of BRCs. In certain embodiments, the BRC is a selected renal cell (SRC). In certain embodiments, the SRC is in a population of SRCs. Various non-limiting examples of SRCs are disclosed herein. In certain embodiments, the SRC is a tubule cell. In certain embodiments, tubule cell is a proximal tubule cell. In certain embodiments, the SRC is an endocrine, vascular, or glomerular cell. In certain embodiments, the population of SRCs comprise hypoxia-resistant and iodixanol-resistant cells. In certain embodiments, the population of SRCs comprise cells that express hyaluronic synthase-2. In certain embodiments, the population of SRCs comprise cells that are capable of receptor-mediated albumin transport. In certain embodiments, the SRC is characterized expresses CK18 and GGT1. In certain embodiments, the metabolism of PrestoBlue and production of VEGF and KIM-1 are used as markers for the presence of viable and functional progeny. In certain embodiments, the BRC is obtained by kidney biopsy.

In certain embodiments, the BRC is genetically modified while within a population of BRCs, wherein fewer than all of the cells in the population of BRCs becomes genetically modified. In certain cases, the method further comprises isolating or enriching a genetically modified BRC from a population of BRCs. In certain cases, the method further comprises isolated or enriching a genetically modified SRC from a population of SRCs. In certain cases, a population of BRCs (*e.g.* SRCs) is subjected to genetic modification to yield a population of BRCs in which some cells are genetically modified and other cells are not. In certain cases some of the genetically modified cells are homozygous for the modification. In certain cases, some of the genetically modified cells are heterozygous for the modification. In certain cases, cells that are homozygous for the modification are enriched or selected. In certain cases, cells that are heterozygous for the modification are enriched or selected. In certain cases, cells that are homozygous or heterozygous for the modification are enriched or selected. In certain cases, the modification is a mutation that reduces the expression of a protein encoded by the gene. In certain cases, the mutation reduces the level of the protein on the surface of modified cells. In certain cases, cells that express the protein are depleted or excluded. In certain cases, the mutation reduces the level of a MHC class I molecule and/or a MHC class II molecule on the surface of a cell. In certain cases, cells with the mutation do not have a MHC class I molecule and/or a MHC class II molecule on the surface thereof. In certain cases, cells that express a MHC class I molecule on the surfaces thereof are depleted or excluded. In certain cases, cells that express a MHC class II molecule on the surfaces thereof are depleted or excluded. In certain cases a cell sorting method is used to remove cells that express the protein, a MHC class I molecule, and/or a MHC class II molecule from the population. In certain cases, the cell sorting method comprises an agent (such as an antibody) that binds to the protein, a MHC class I molecule, and/or a MHC class II molecule. In certain cases depletion or selection of cells comprises bead/antibody coupling to pull out cells with certain proteins on their cell surface. In certain cases, the cell sorting method is magnetic activated cell sorting (MACS) or fluorescence-activated cell sorting (FACS). In certain cases, the gene chosen for genetic modification is one whose protein is expressed on the cell surface, so FACS and/or MACS technology can differentiate *(e.g.,* based on antibody binding onto the surface) of the live cell. In cases, MACs is used to remove cells that express a MHC molecule (such as a MHC class I molecule or a MHC class II molecule) from cells that do not. In certain cases, an integrating or non-integrating vector may be used to express another HLA component polypeptide to further modify or modulate the adaptive or innate immune system as for example, to prevent targeting and lysis by Natural-Killer (NK) cells.

In certain embodiments, genetically modifying (*e.g*., mutating) the gene comprises (i) expressing a gene editing protein in the BRC; or (ii) delivering a gene editing protein across the cell membrane of the BRC. In certain embodiments, the gene editing protein is a zinc finger nuclease (ZFN), a transcription activator-like effector nuclease (TALEN), a megaTAL, or an RNA-guided endonuclease. In certain embodiments, the RNA-guided endonuclease is a Cas protein. In certain embodiments, the Cas protein is a Cas9 protein. In certain cases, genetically modifying the gene further comprises (i) expressing a guide single-guide RNA (gRNA) in the BRC; or (ii) delivering a guide single-guide RNA (gRNA) across the cell membrane of the BRC. In certain cases, the Cas9 protein and the gRNA are part of a ribonucleoprotein complex.

In certain cases, a method of producing a genomically modified BRC comprises genetically modifying genomic immunogenicity gene in a BRC to produce a genomically modified BRC and then culturing the modified BRC to produce progeny with a genetic modification in the gene. In certain cases, the progeny have reduced potential for immune rejection compared to corresponding cells that do not contain the genetic modification in the gene. In certain cases, the method comprises expanding the progeny. In certain cases, expanding the progeny comprises passaging the progeny at least 1, 2, 3, 4, or 5 times. In certain cases, cell growth kinetics and/or viability are monitored at each cell passage. In certain cases, viability of the expanded progeny is assaed. In certain cases, the number of expanded cells is determined. In certain cases, cell counts and viability of the progeny are monitored by Trypan Blue dye exclusion and metabolism of PrestoBlue. In certain cases, BRC or SRC functionality is assessed by gene expression profiling or measurement of enzymatic activities. In certain cases, enzymatic activity is measured for LAP and/or GGT.

In certain embodiments, the gene editing protein is expressed from transfected mRNA. In certain embodiments, the Cas protein is expressed from transfected mRNA and the gRNA is expressed from a plasmid DNA. In certain embodiments, the transfected mRNA is stabilized by including modified nucleic acid bases or polyadenylation sequences. In certain embodiments, the transfected mRNA is coupled with at least one cell-penetrating peptide. In certain embodiments, the Cas protein is expressed from a DNA vector. In certain embodiments, the expression of the Cas protein is induced during at least part of the time that the gRNA is expressed in the cell. In certain embodiments, the DNA vector does not integrate in the genome. In certain embodiments, the DNA vector is an episomal vector. In certain embodiments, the DNA vector is a transposon. In certain embodiments, the gRNA is a transcript from a DNA vector. In certain embodiments, the gene editing protein is a recombinant protein and is complexed with a gRNA *ex vivo.* In certain embodiments, the gene editing protein is a recombinant protein and is complexed with a gRNA *ex vivo* and the protein/gRNA complex is delivered to the cell by transfection, lipofection, electroporation, microinjection or other methodology known to those of skill in the art. In certain cases, additional nucleic acid elements encoding a selectable marker and/or specific mutations to the targeted gene are introduced into the cell together with the Cas9 protein/gRNA complex.

In certain embodiments, genetically modifying the gene reduces the amount of MHC class I on the surface of the cell. In certain embodiments, genetically modifying the gene reduces the amount of MHC class II on the surface of the cell. In certain embodiments, the method comprises genetically modifying at two or more genes, wherein at least one of the genes encodes a protein within a MHC class I molecule and at least one of the genes is encodes a protein within a MHC class II molecule. In certain embodiments, at least one of the genes is an HLA gene.

In an aspect, provided herein is a genomically modified BRC population obtainable by a method disclosed herein. In certain cases, the population comprises both genetically unmodified and genetically modified cells. In certain cases, the population is enriched for the engineered BRC. In certain cases, the engineered BRC are homozygous for a genetic modification.

In an aspect, provided herein is a population of genomically modified BRCs obtainable by the method of claim 1, and their use in treating a kidney disease in a patient.

In certain cases, the kidney disease is chronic kidney disease.

In an aspect, provided herein is an injectable formulation comprising a) a temperature-sensitive cell-stabilizing biomaterial, and b) a BRC population, obtainable by the method according to claim 1, wherein the temperature-sensitive cell-stabilizing biomaterial is a hydrogel that (i) maintains a substantially solid state at about 8°C or below, wherein the substantially solid state is a gel state, (ii) maintains a substantially liquid state at about ambient temperature or above, and (iii) has a solid-to-liquid transitional state between about 8°C and about ambient temperature or above.

In certain cases, the hydrogel comprises an extracellular matrix protein of recombinant origin, is derived from extracellular matrix sourced from kidney or another tissue or organ, or comprises gelatin. In certain cases, the hydrogel comprises gelatin. In certain cases, the gelatin is derived from Type I, alpha I collagen. In certain cases, the gelatin is derived from porcine Type I, alpha I collagen or recombinant human Type I, alpha I collagen. In certain cases, the BRC population is a selected renal cell (SRC) population and the engineered BRCs are engineered SRCs.

The present disclosure if of methods to genetically engineer BRCs, into immunogenically transparent allogeneic BRCs obtainable from donors, to make them suitable for therapeutic purposes, by using RNA-guided endonucleases such as Cas9/clustered regularly interspaced short palindromic repeat (CRISPR). Methods provided herein more particularly allow the precise modification of the genome of BRCs relevant for kidney tissue regeneration by inactivating and/or replacing genes involved in the immunological recognition of BRCs *(e.g.* MHC recognition and/or immune checkpoint proteins) so as to allow for the incorporation of these cells as an allogeneic implant without immune clearance. herein is a method of preparing a genomically modified immunoprivileged bioactive renal cell population comprising the step of (a) genetically modifying BRC or SRC comprising the introduction and/or expression within the cells an RNA-guided endonuclease; and a specific guide RNA that directs the endonuclease to at least one targeted immunogenicity gene in the BRC or SRC genome; and (b) expanding the resulting cells that are immune-privileged with low or reduced potential for immune rejection. In certain embodiments, the RNA-guided endonuclease is Cas9. The RNA-guided endonuclease may be expressed from transfected mRNA or the RNA-guided endonuclease is expressed from transfected mRNA and the gRNA is expressed from a plasmid DNA. In certain cases, the transfected mRNA is stabilized by including modified nucleic acid bases or polyadenylation sequences. In certain cases, the transfected mRNA is coupled with at least one cell-penetrating peptide. In certain embodiments of the method, the endonuclease is expressed from a DNA vector. The expression of the endonuclease may be induced at the time the guide RNA is produced into the cell. In certain cases, the DNA vector does not integrate in the genome. The DNA vector may be an episomal vector, an artificial chromosome, or a transposon. In certain cases, the guide RNA is a transcript from a DNA vector. In certain embodiments, the Cas protein is a purified recombinant protein complexed *ex vivo* with a guide RNA and/or DNA targeting construct containing defined mutations. In certain embodiments, the Cas/gRNA complex or plasmid is introduced into the cell by electroporation.

In certain embodiments, the at least one targeted immunogenicity gene encodes one or more genes controlling MHC class I. In certain embodiments, the at least one targeted immunogenicity gene encodes one or more genes controlling MHC class II. In certain embodiments, the at least one targeted immunogenicity gene encodes one or more genes controlling MHC class I and MHC class II. In certain embodiments, the at least one targeted locus may encode an HLA gene. In certain embodiments, the HLA gene is selected from the group consisting of HLA-A, HLA-B, HLA-C, HLA-DRB1, HLA-DRB3/4/5, a HLA-DQ family gene *(e.g.,* HLA-DQA1, and/or HLA-DQB1), and a HLA-DP family gene *(e.g.,* HLA-DPA1 and/or HLA-DPA2).

In an aspect, the bioactive renal cell population is obtained from isolation and expansion of renal cells from kidney tissue under culturing conditions that enrich for cells capable of kidney regeneration. In certain embodiments, the bioactive renal cell population is obtained after exposure to hypoxic culture conditions. In certain embodiments, the BRC population is a SRC population obtained after density gradient separation of the expanded renal cells. In certain embodiments, the SRCs exhibit a buoyant density greater than about 1.04, 1.0419, or 1.045 g/mL. In certain embodiments, the BRC or SRC population contains a greater percentage of one or more cell populations and lacks or is deficient in one or more other cell populations, as compared to a starting kidney cell population. In certain embodiments, cell growth kinetics of the BRC or SRC population may be monitored at each cell passage. For example, BRC or SRC counts and viability may be monitored by Trypan Blue dye exclusion and metabolism of PrestoBlue. In certain embodiments, the BRC or SRC may be characterized by phenotypic expression of specific viability and functionality markers, for example CK18 and GGT1. In certain embodiments, the production of VEGF and KIM-1 may be used as markers for the presence of viable and functional BRC or SRC. In certain embodiments, viability and functionality may be established with gene expression profiling or measurement of enzymatic activity. In certain embodiments, the BRCs or SRCs are characterized by measurement of enzymatic activity for LAP and/or GGT.

### BRIEF DESCRIPTION OF THE DRAWINGS

FIG. 1: Flow diagram representation of a manufacturing plan for generating engineered human allogenic renal cells to be used in treating chronic kidney disease.

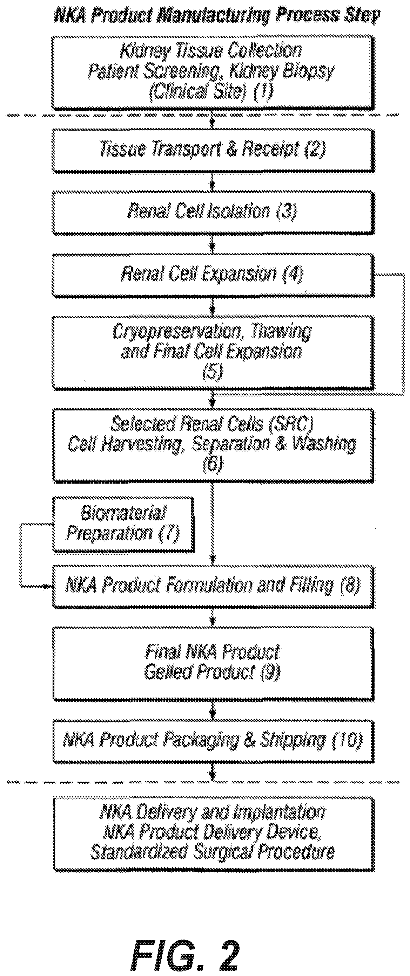

FIG. 2: Flow diagram of a non-limiting example of an overall NKA manufacturing process.
FIGs. 3A-D: Flow diagrams providing further details of the non-limiting example process depicted in FIG. 2.

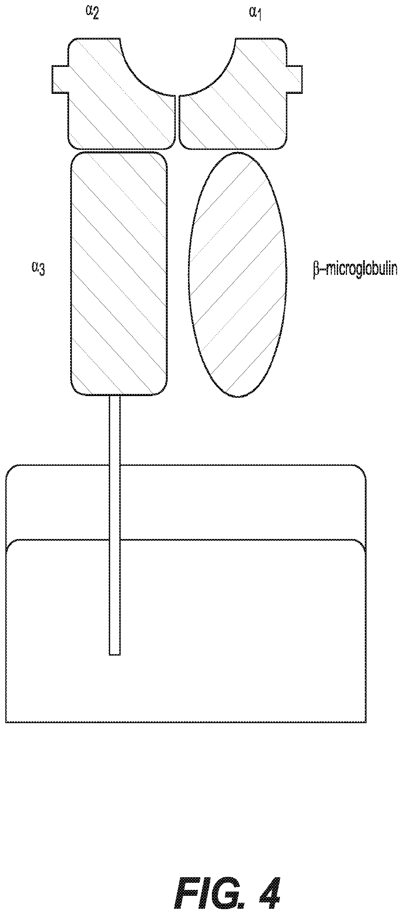

FIG. 4: A cartoon of a MHC class I complex.

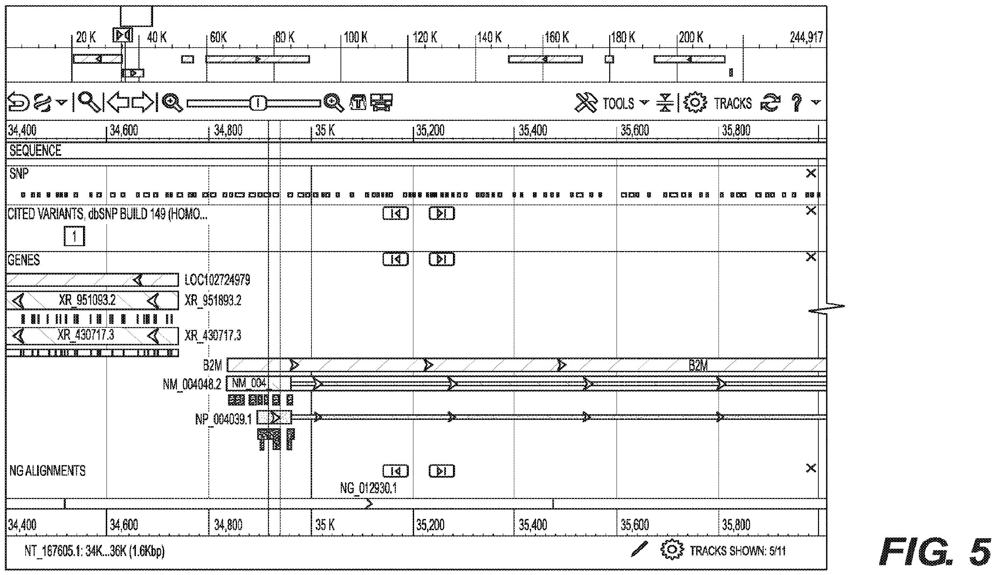

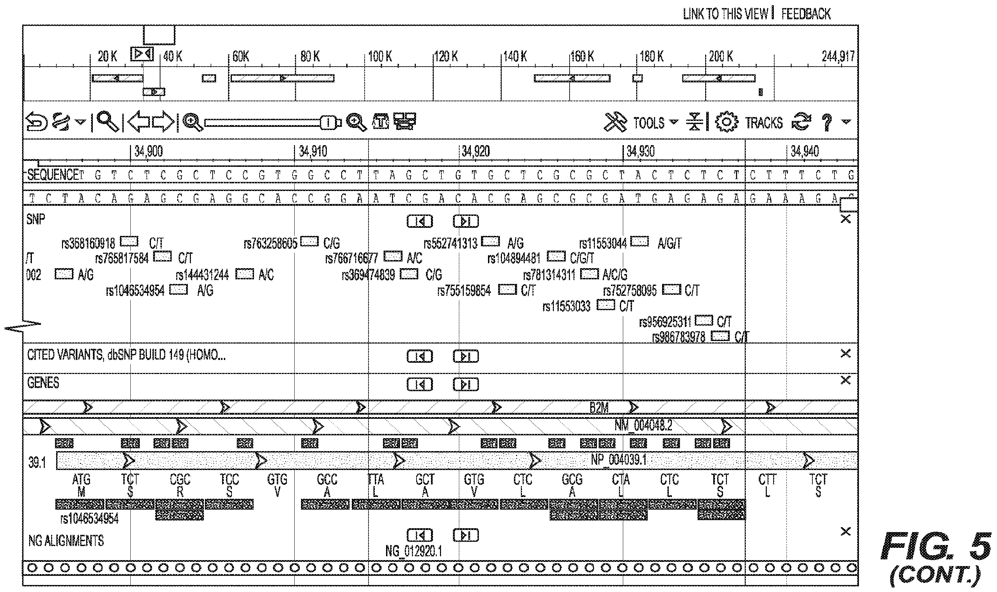

FIG. 5: A genomic scaffold at the B2M gene showing site of guide RNA binding.
FIG. 6: FACS showing CRISPR/Cas9 mediated knockdown of B2M (invariable subunit of MHC-I) in human primary kidney cells.
FIG. 7: FACS showing CRISPR/Cas9 mediated knockdown of B2M (invariable subunit of MHC-I) in human primary kidney cells.
FIG. 8: An image from a genomic cleavage assay demonstrating successful gene editing at the B2M locus. The blot shown is an ethidium bromide-stained DNA gel with the white/black inverted for clarity. In non-limiting examples, the genome cleavage assay or direct sequencing of the targeted locus may be used to confirm a targeted genome modification.
FIG. 9: A FACS output demonstrating CRISPR mediated editing of the B2M locus in SRC. Note different donor ABO haplotypes, showing ability to execute gene editing of MHC-I is independent of ABO haplotype:

| **donor** | **blood type** |
|---|---|
| TCHK006 | O(+) |
| TCHK011 | AB(-) |
| TCHK004 | O(-) |

FIG. 10: A graph from a lymphocyte proliferation assay demonstrating that B2M CRISPR modified SRCs decrease lympohocyte profileration relative to unmodified control SRCs, providing evidence that CRISPR targeting of B2M lowers immunogenicity of CRISPR modified SRCs.
FIG. 11: A graph from potency assay testing of B2M CRISPR modified SRC, showing no significant differences in secretion of VEGF and KIM1 between modified and unmodified SRC. CRISPR modification does not affect functionality of SRCs.

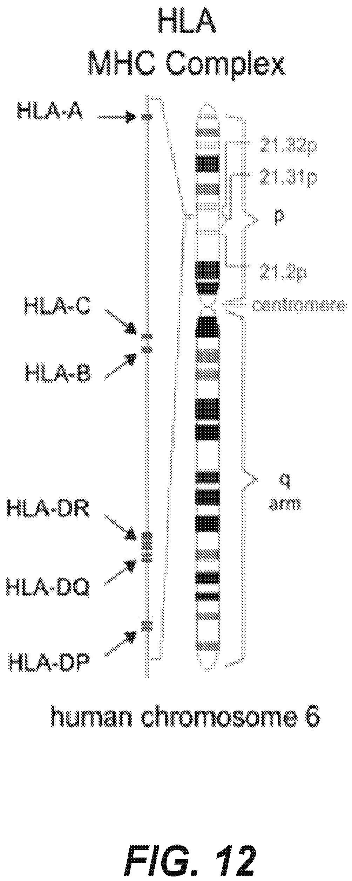

FIG. 12: A cartoon showing the HLA MHC Complex on human chromosome 6.
FIG. 13: An image showing exemplary human renal cell morphology in culture.
FIG. 14: An image showing exemplary SRC banding by centrifugation across a density boundary.
FIG. 15: A graph showing an exemplary gelatin solution temperature profile for gelation.
FIG. 16: A graph showing exemplary cell distribution as a function of rotation time during NKA gelation.

### DETAILED DESCRIPTION OF THE INVENTION

Provided herein are, *inter alia,* formulations and methods for obtaining renal cell populations that can be used in numerous patients. In certain cases, the popultions are less immunogenic than populations from which they are derived. In certain cases, gene editing is used to generate an allogeneic renal cell population to be administered to patients without immunosuppression. Included herein are suitable donor cells from different tissue types or donors that can be successfully transplanted into recipient subjects regardless of immunogenicity gene haplotype differences.

Ideally, one would like to use a standardized therapy in which allogeneic therapeutic cells could be pre-manufactured, characterized in detail, and available for immediate administration to patients. Unfortunately, the availability of suitable donor cells with matching alleles at one or more immunogenicity gene loci is limited because of haplotype heterogeneity in human populations. For example, the HLAs are immunogenicity genes that were first identified during early bone marrow hematopoietic stem/progenitor cell transplantation (HSCT) clinic treatments. Mismatch of HLAs between a donor and a recipient subject can cause immune reactions where recipient T cells can recognize the incoming donor allogeneic tissue as foreign by recognizing HLA proteins or donor-specific antigens that are expressed or presented on the allogeneic donor cell surface, ultimately leading to graft rejection. (Bhatia S. Expert Rev Hematol. 2011; 4(4):437-452; Garnett C, et al. TherAdv Hematol. 4(6): 366-78 (2013)). Despite advances in the medical field to suppress immune responses against allogeneic transplanted donor cells, there remains a need for additional methods and compositions that can decrease rejection and/or improve the immunocompatibility of donor cells. Most notably, there remains a need to improve the availability of suitable donor cells that can be successfully transplanted into recipient subjects regardless of immunogenicity gene haplotype differences.

One approach to overcoming the immunological barrier to cell transplantation is genetic manipulation of HLA molecules. By using zinc finger nucleases, Torikai and colleagues selectively eliminated human leukocyte antigen (HLA) class I in embryonic stem cells (ESCs) and demonstrated that HLA-A cells could escape lysis from HLA-restricted cytotoxic T lymphocytes. (Oberg L etc., Eur J Immunol., 2004, 34(6): 1646-1653). In another study, Riolobos and colleagues disrupted B2M which encodes the accessory chain of MHC class I molecules and is required for their surface expression (Laura Riolobos etc., 2013, 21(6): 1232-1241). The homozygous deletion of the B2M gene prevented the surface translocation of class I human leukocyte antigen (HLA) molecules and reduced immunogenicity. More recently, the discovery and application of the CRISPR/Cas9 system in mammalian cells has been shown to result in effective and precise editing of target genes, *e.g.,* through the non-homologous end joining pathway (NHEJ), homology directed repair (HDR), or other DNA repair pathways. (Gasiunas, Barrangou et al. 2012; Jinek, Chylinski et al. 2012) Co-delivery of a Cas9 molecule and a target-specific guide RNA (gRNA) molecule facilitates gene-editing of a target sequence in the genome. Thus, the use of the CRISPR/Cas9 system to modify genes in cells is also a promising strategy for increasing the immunocompatibility of donor cells for transplantation to a recipient subject. (WO2016201047 A1).

Experiments relating to the immunogenicity of one cell type (such as embryonic stem cells) cannot predictably be applied or translated to another (such as renal cells). Surprisingly, knocking out some B2M expression also knocked down the immunogenicity of a renal cell population. Moreover, decreased immunogenicity was observed despite some B2M expression being retained in the modified cell population. Included herein are gene-editing technologies to modify the adaptive immune system of BRCs (such as SRCs) to create a universal donor cell for treating of kidney disease. In certain cases, immunogenicity is the ability to trigger the adaptive (*e.g.* T-cell mediated)immune response.

Aspects of the present disclosure relate, at least in part, to the discovery that SRCs from a donor with one haplotype can be genetically modified to render them less immunogenic in subjects with a different haplotype. Included herein are genetically modified SRCs that can be successfully transplanted into recipient subjects regardless of immunogenicity gene haplotype differences, as well as methods and formulations for treating kidney diseases with such modified SRCs.

Reference will now be made in detail to certain embodiments of the invention.

### Definitions

Unless defined otherwise, technical and scientific terms used herein have the same meaning as commonly understood by one of ordinary skill in the art to which this invention belongs. Principles of Tissue Engineering, 3rd Ed. (Edited by R Lanza, R Langer, & J Vacanti), 2007 provides one skilled in the art with a general guide to many of the terms used in the present application. One skilled in the art will recognize many methods and materials similar or equivalent to those described herein, which could be used in the practice of the present invention. Indeed, the present invention is in no way limited to the methods and materials described.

As used herein, the singular forms "a," "an," and "the" are intended to include the plural forms as well, unless the context clearly indicates otherwise. As used herein, the term "and/or" includes any and all combinations of one or more of the associated listed items.

The words "comprise," "comprising," "include," "including," and "includes" when used in this specification and claims are intended to specify the presence of stated features, integers, components, or steps, but they do not preclude the presence or addition of one or more other features, integers, components, steps, or groups thereof.

As used herein, the term "about" in the context of a numerical value or range means ±10% of the numerical value or range recited or claimed, unless the context requires a more limited range.

The term "cell population" as used herein refers to a number of cells obtained by isolation directly from a suitable tissue source, usually from a mammal. For example, a cell population may comprise populations of kidney cells, and admixtures thereof. The isolated cell population may be subsequently cultured *in vitro.* Those of ordinary skill in the art will appreciate that various methods for isolating and culturing cell populations for use with the present disclosure and various numbers of cells in a cell population that are suitable for use in the present disclosure. A cell population may be an unfractionated, heterogeneous cell population or an enriched homogeneous cell population derived from an organ or tissue, e.g., the kidney. For example, a heterogeneous cell population may be isolated from a tissue biopsy or from whole organ tissue. Alternatively, the heterogeneous cell population may be derived from in vitro cultures of mammalian cells, established from tissue biopsies or whole organ tissue. An unfractionated heterogeneous cell population may also be referred to as a non-enriched cell population. In certain embodiments, the cell populations contain bioactive cells. Homogenous cell populations comprise a greater proportion of cells of the same cell type, sharing a common phenotype, or having similar physical properties, as compared to an unfractionated, heterogeneous cell population. For example, a homogeneous cell population may be isolated, extracted, or enriched from heterogeneous kidney cell population. In certain embodiments, a homogeneous cell population is obtained as a cell fraction using separation by centrifugation across a density boundary, barrier, or interface of a heterogeneous cell suspension. In certain embodiments, a homogeneous cell population is obtained as a cell fraction using continuous or discontinuous (single step or multi-step) density gradient separation of a heterogeneous cell suspension. In certain embodiments, a homogenous or heterogeneous cell population sourced from the kidney is admixed with a homogenous or heterogeneous cell population sourced from a tissue or organ other than the kidney, without further limitation.

As used herein, the term "bioactive" means "possessing biological activity," such as a pharmacological or a therapeutic activity. In certain embodiments, the bioactivity is enhancement of renal function and/or effect on renal homeostasis. In certain embodiments, the biological activity is, without limitation, analgesic; antiviral; anti-inflammatory; antineoplastic; immune stimulating; immune modulating; enhancement of cell viability, antioxidation, oxygen carrier, cell recruitment, cell attachment, immunosuppressant, angiogenesis, wound healing activity, mobilization of host stem or progenitor cells, cellular proliferation, stimulation of cell migration to injury sites, amelioration of cell and tissue fibrosis, interference with the epithelial-mesenchymal signaling cascade, secretion of cytokines, growth factors, proteins, nucleic acids, exosomes, microvesicles or any combination thereof.

The term "bioactive renal cells" or "BRCs" as used herein refers to renal cells having one or more of the following properties when administered into the kidney of a subject: capability to reduce (*e.g.,* slow or halt) the worsening or progression of chronic kidney disease or a symptom thereof, capability to enhance renal function, capability to affect (improve) renal homeostasis, and capability to promote healing, repair and/or regeneration of renal tissue or kidney. In certain embodiments, the BRCs are genetically modified (*e.g.,* genomically modified and/or modified via RNAi) such that they are less immunogenic in a patient than they would be without the genetic modification. In certain embodiments, the BRCs may be administered to a patient regardless of the patient's haplotype. In certain embodiments, the BRCs are genetically modified (*e.g*., genomically modified and/or modified via RNAi) immunoprivileged cells capable of enhancing renal function, affect (improve) renal homeostasis, and/or promote healing, repair and/or regeneration of renal tissue or kidney without immunologic rejection. In certain embodiments, these cells may include functional tubular cells (*e.g.,* based on improvements in creatinine excretion and protein retention), glomerular cells (*e.g.,* based on improvement in protein retention), vascular cells and other cells of the corticomedullary junction. In certain embodiments, BRCs are obtained from isolation and expansion of renal cells from kidney tissue. In certain embodiments, BRCs are obtained from isolation and expansion of renal cells from kidney tissue using methods that select for bioactive cells (*e.g.,* cells with regenerative capacity). In certain embodiments, the BRCs have a regenerative effect on the kidney. In certain embodiments, BRCs comprise, consist essentially of, or consist of selected renal cells (SRCs). In certain embodiments, BRCs are SRCs. Methods to obtain bioactive renal cells (BRC) are disclosed, for example, in Presnell et al. WO/2010/056328, Ilagan et al. PCT/US2011/036347, and Jain et al. PCT/US2016/044866.

In certain embodiments, SRCs are cells obtained from isolation and expansion of renal cells from a suitable renal tissue source, wherein the SRCs contain a greater percentage of one or more cell types and lacks or has a lower percentage of one or more other cell types, as compared to a starting kidney cell population. In certain embodiments, the SRCs contain an increased proportion of BRCs compared to a starting kidney cell population. In certain embodiments, an SRC population is an isolated population of kidney cells enriched for specific bioactive components and/or cell types and/or depleted of specific inactive and/or undesired components or cell types for use in the treatment of kidney disease, *i.e.,* providing stabilization and/or improvement and/or regeneration of kidney function. In certain embodiments, the SRCs are genetically modified (*e.g*., genomically modified and/or modified via RNAi) such that the are less immunogenic in a patient than they would be without the genetic modification. In certain embodiments, the SRCs may be administered to a patient regardless of the patient's haplotype. In certain embodiments, the SRC have been genetically modified (*e.g*., genomically modified and/or modified via RNAi) to render them immunoprivileged or incapable of rejection and are capable of providing stabilization and/or improvement and/or regeneration of kidney function. SRCs provide superior therapeutic and regenerative outcomes as compared with the starting population. In certain embodiments, SRCs are obtained from the patient's renal cortical tissue via a kidney biopsy. In certain embodiments, SRCs are selected (*e.g.,* by MACS or FACS) based on their expression of one or more markers. In certain embodiments, SRCs are depleted (*e.g.,* by MACS or FACS) of one or more cell types based on the expression of one or more markers on the cell types. In certain embodiments, depletion or selection of cells comprises bead/antibody coupling to pull out cells with certain proteins on their cell surface. In certain embodiments, SRCs are selected from a population of bioactive renal cells. In certain embodiments, SRCs are selected by density gradient separation of expanded renal cells. In certain embodiments, SRCs are selected by separation of expanded renal cells by centrifugation across a density boundary, barrier, or interface, or single step discontinuous step gradient separation. In certain embodiments, SRCs are selected by continuous or discontinuous density gradient separation of expanded renal cells that have been cultured under hypoxic conditions. In certain embodiments, SRCs are selected by density gradient separation of expanded renal cells that have been cultured under hypoxic conditions for at least about 8, 12, 16, 20, or 24 hours. In certain embodiments, SRCs are selected by separation by centrifugation across a density boundary, barrier, or interface of expanded renal cells that have been cultured under hypoxic conditions. In certain embodiments, SRCs are selected by separation of expanded renal cells that have been cultured under hypoxic conditions for at least about 8, 12, 16, 20, or 24 hours by centrifugation across a density boundary, barrier, or interface (*e.g*., single-step discontinuous density gradient separation). In certain embodiments, SRCs are composed primarily of renal tubular cells. In certain embodiments, other parenchymal (*e.g*., vascular) and stromal (*e.g*., collecting duct) cells may be present in SRCs. In certain embodiments, less than about 10%, 9%, 8%, 7%, 6%, 5%, 4%, 3%, 2%, or 1% of the cells in a population of SRCs are vascular cells. In certain embodiments, less than about 10%, 9%, 8%, 7%, 6%, 5%, 4%, 3%, 2%, or 1% of the cells in a population of SRCs are collecting duct cells. In certain embodiments, less than about 10%, 9%, 8%, 7%, 6%, 5%, 4%, 3%, 2%, or 1% of the cells in a population of SRCs are vascular or collecting duct cells. Methods to obtain SRCs are disclosed, for example, in Example 1 herein, Presnell et al. WO/2010/056328, Ilagan et al. PCT/US2011/036347, and Jain et al. PCT/US2016/044866.

The term "native organ" shall mean the organ of a living subject. The subject may be healthy or unhealthy. An unhealthy subject may have a disease associated with that particular organ.

The term "native kidney" shall mean the kidney of a living subject. The subject may be healthy or unhealthy. An unhealthy subject may have a kidney disease.

The term "regenerative effect" shall mean an effect which provides a benefit to a native organ, such as the kidney. The effect may include, without limitation, a reduction in the degree of injury to a native organ or an improvement in, restoration of, or stabilization of a native organ function or structure. Renal injury may be in the form of fibrosis, inflammation, glomerular hypertrophy, atrophy, etc. and related to a disease associated with the native organ in the subject.

The term "admixture" as used herein in the context of a cell population refers to a combination of two or more isolated, enriched cell population phenotypes derived from an unfractionated, heterogeneous cell population. According to certain embodiments, the cell populations of the present invention are renal cell populations.

An "enriched" cell population or preparation refers to a cell population derived from a starting organ cell population (*e.g*., an unfractionated, heterogeneous cell population) that contains a greater percentage of a specific cell type than the percentage of that cell type in the starting population. For example, a starting kidney cell population can be enriched for a first, a second, a third, a fourth, a fifth, and so on, cell population of interest. As used herein, the terms "cell population", "cell preparation" and "cell phenotype" are used interchangeably.

The term "hypoxic" culture conditions as used herein refers to culture conditions in which cells are subjected to a reduction in available oxygen levels in the culture system relative to standard culture conditions in which cells are cultured at atmospheric oxygen levels (about 21%). Non-hypoxic conditions are referred to herein as normal or normoxic culture conditions.

Provided herein are BRCs (*e.g.,* SRCs) that are "genetically modified" or "genetically engineered" or have received "genomic modification." In certain embodiments, such cells may be obtained by using a gene editing technique to remove one or more specific cell surface antigens, such as major histocompatibility antigens, from SRC/BRC donor populations. A variety of techniques may be used to engineer such cells. For example, cloned or synthetically engineered DNA can be inserted, replaced, or removed to generate engineered SRC/BRC by genome editing methods with engineered nucleases, such as: a) the CRISPR/Cas system; b) Transcription Activator-Like Effector Nucleases (TALENs); c) zinc finger nucleases (ZFNs); d) and engineered meganuclease homing endonucleases. Various other polynucleotide delivery methods known in the art may be suitable for the engineering of SRC/BRC such as transfection, electroporation, transduction, lipofection, nanoengineered substances, such as organically modified silica or organically modified silicate (*e.g.,* Ormosil), viral delivery methods, including adenoviruses, retroviruses, lentiviruses, adeno-associated viruses, or another suitable method.

By "RNA-guided endonuclease" is meant a polypeptide the endonuclease activity and specificity of which depend on its association with an RNA molecule. The full sequence of this RNA molecule or more generally a fragment of this RNA molecule, which is a sequence preferably longer than 8 nucleic acid bases, more preferably longer than 10 nucleic acid bases, even more preferably longer than 12 nucleic acid bases, has the ability to specify a target sequence in the genome. In general, this RNA molecule has the ability to hybridize the target sequence and to mediate the endonuclease activity of such endonuclease. An example of RNA-guided endonuclease is Cas9 (CRISPR associated protein 9) as part of the Cas9/CRISPR system. For further details see, e.g. Sanders and Joung, Nature Biotechnology 32, 347-355 (2014).

As used herein, "immunogenicity" refers to property that allows a substance to induce a detectable immune response (humoral or cellular) when introduced into a subject (*e.g.,* a human subject).

The term "immunogenicity gene" includes genes encoding a major histocompatibility antigen complex protein or a minor histocompatibility antigen. In certain embodiments, the immunogenicity gene is any one of, or any combination of, a B2M, HLA-A, HLA-B, HLA-C, HLA-DRA, HLA-DRB1, HLA-DRB3, HLA-DRB4, HLA-DRB5, HLA-DPA1, HLA-DPA2, HLA-DQA1, and/or HLA-DQB1gene. In certain embodiments, the immunogenicity gene is a gene encoding a protein selected from the group consisting of B2M, HLA-A, HLA-B, HLA-C, a HLA-DR family gene (*e.g.,* HLA-DRA, HLA-DRB1, HLA-DRB3, HLA-DRB4, and/or HLA-DRB5), a HLA-DP family gene (*e.g.,* HLA-DPA1 and/or HLA-DPA2), and a HLA-DQ family gene (*e.g.,* HLA-DQA1, and/or HLA-DQB1).

The term "immune-compatible renal cell" or "immune-compatible renal cell population" includes a renal cell or population lacking one or more alleles of a gene encoding a major histocompatibility antigen complex protein and/or minor histocompatibility antigen that renders the cell immunologically recognized. In certain embodiments, an immune-compatible renal cell or immune-compatible renal cell population is a renal cell or population lacking one or more alleles of a gene encoding B2M. In certain embodiments, an immune-compatible renal cell or immune-compatible renal cell population is a renal cell or population lacking one or more alleles of any one of, or any combination of, a B2M, HLA-A, HLA-B, HLA-C, HLA-DRA, HLA-DRB1, HLA-DRB3, HLA-DRB4, HLA-DRB5, HLA-DPA1, HLA-DPA2, HLA-DQA1, and/or HLA-DQB1 gene. In certain embodiments, the administration of immune-compatible renal cells to a recipient subject is immunologically privileged and does not induce an immune response in the recipient subject.

The term "biomaterial" as used herein refers to a natural or synthetic biocompatible material that is suitable for introduction into living tissue supporting the selected bioactive cells in a viable state. A natural biomaterial is a material that is made by or originates from a living system. Synthetic biomaterials are materials which are not made by or do not originate directly from a living system, but are instead synthesized or composed by specific chemical procedures and protocols well known to those of ordinary skill in the art. The biomaterials disclosed herein may be a combination of natural and synthetic biocompatible materials. As used herein, biomaterials include, for example, polymeric matrices and scaffolds. Those of ordinary skill in the art will appreciate that the biomaterial(s) may be configured in various forms, for example, as porous foam, gels, liquids, beads, solids, and may comprise one or more natural or synthetic biocompatible materials. In certain embodiments, the biomaterial is the liquid form of a solution that is capable of becoming a hydrogel.

The term "hydrogel" is used herein to refer to a substance formed when an organic polymer (natural or synthetic) is cross-linked via covalent, ionic, or hydrogen bonds to create a three-dimensional open-lattice structure which entraps water molecules to form a gel. Examples of materials which can be used to form a hydrogel include polysaccharides such as alginate, polyphosphazines, and polyacrylates, which are crosslinked tonically, or block copolymers such as Pluronics^{™} or Tetronics^{™}, polyethylene oxide-polypropylene glycol block copolymers which are crosslinked by temperature or pH, respectively. The hydrogel used herein is preferably a biodegradable gelatin-based hydrogel.

As used herein, biomaterials include, for example, extracellular matrix derived from an existing kidney of human or animal origin, wherein the native cell population has been eliminated through application of detergents and/or other chemical agents known to those of ordinary skill in the art. In certain embodiments, the biomaterial is a liquid form of a solution that is capable of becoming a hydrogel and is layered with or without certain cell populations by application of three-dimensional bioprinting methodologies known to those skilled in the art. In certain embodiments, the biomaterial is configured to mimic the three dimensional fractal organization of decellurized kidney.

The term "modified release" or the equivalent terms "controlled release", "delayed release", or "slow release" refer to formulations that release an active agent, such as bioactive cells, over time or at more than one point in time following administration to an individual. Modified release of an active agent, which can occur over a range of desired times, *e.g.,* minutes, hours, days, weeks, or longer, depending upon the formulation, is in contrast to standard formulations in which substantially the entire dosage unit is available immediately after administration. For tissue engineering and regenerative medicine applications, preferred modified release formulations provide for the release of an active agent at multiple time points following local administration (*e.g*., administration of an active agent directly to a solid organ). For example, a modified release formulation of bioactive cells would provide an initial release of cells immediately at the time of administration and a later, second release of cells at a later time. The time delay for the second release of an active agent may be minutes, hours, or days after the initial administration. In general, the period of time for delay of release corresponds to the period of time that it takes for a biomaterial carrier of the active agent to lose it structural integrity. The delayed release of an active agent begins as soon as such integrity begins to degrade and is completed by the time integrity fails completely. Those of ordinary skill in the art will appreciate other suitable mechanisms of release.

The term "ambient temperature" refers to the temperature at which the formulations of the present disclosure will be administered to a subject. Generally, the ambient temperature is the temperature of a temperature-controlled environment. Ambient temperature ranges from about 18°C to about 30°C. In certain embodiments, ambient temperature is about 18°C, about 19°C, about 20°C, about 21°C, about 22°C, about 23°C, about 24°C, about 25°C, about 26°C, about 27°C, about 28°C, about 29°C, or about 30°C.

The term "kidney disease" as used herein refers to disorders associated with any stage or degree of acute or chronic renal failure that results in a loss of the kidney's ability to perform the function of blood filtration and elimination of excess fluid, electrolytes, and wastes from the blood. Kidney disease may also include endocrine dysfunctions such as anemia (erythropoietin-deficiency), and mineral imbalance (Vitamin D deficiency). Kidney disease may originate in the kidney or may be secondary to a variety of conditions, including (but not limited to) heart failure, hypertension, diabetes, autoimmune disease, or liver disease. Kidney disease may be a condition of chronic renal failure that develops after an acute injury to the kidney. For example, injury to the kidney by ischemia and/or exposure to toxicants may cause acute renal failure; incomplete recovery after acute kidney injury may lead to the development of chronic renal failure.

The term "treatment" refers to both therapeutic treatment and prophylactic or preventative measures for kidney disease, anemia, tubular transport deficiency, or glomerular filtration deficiency wherein the object is to reverse, prevent or slow down (lessen) the targeted disorder. Those in need of treatment include those already having a kidney disease, anemia, tubular transport deficiency, or glomerular filtration deficiency as well as those prone to having a kidney disease, anemia, tubular transport deficiency, or glomerular filtration deficiency or those in whom the kidney disease, anemia, tubular transport deficiency, or glomerular filtration deficiency is to be prevented. The term "treatment" as used herein includes the stabilization and/or improvement of kidney function.

The term "in vivo contacting" as used herein refers to direct contact in vivo between products secreted by an enriched population of cells and a native organ. For example, products secreted by an enriched population of renal cells (or an admixture or construct containing renal cells/renal cell fractions) may in vivo contact a native kidney. The direct in vivo contacting may be paracrine, endocrine, or juxtacrine in nature. The products secreted may be a heterogeneous population of different products described herein.

The terms "construct" or "formulation" refer to one or more cell populations deposited on or in a surface of a scaffold or matrix made up of one or more synthetic or naturally-occurring biocompatible materials. The one or more cell populations may be coated with, deposited on, embedded in, attached to, seeded, or entrapped in a biomaterial made up of one or more synthetic or naturally-occurring biocompatible biomaterials, polymers, proteins, or peptides. In certain embodiments, the naturally occurring biomaterial is decellularized kidney of human or animal origin. In certain embodiments, the biomaterial has been structurally engineered through three dimensional bioprinting. The one or more cell populations may be combined with a biomaterial or scaffold or matrix in vitro or in vivo. The one or more biomaterials used to generate the construct or formulation may be selected to direct, facilitate, or permit dispersion and/or integration of the cellular components of the construct with the endogenous host tissue, or to direct, facilitate, or permit the survival, engraftment, tolerance, or functional performance of the cellular components of the construct or formulation. In certain embodiments, the one or more biocompatible materials used to form the scaffold/biomaterial is selected to direct, facilitate, or permit the formation of multicellular, three-dimensional, organization of at least one of the cell populations deposited thereon. In certain embodiments, the biomaterials direct the assembly of defined three dimensional cellular aggregrates or organoids that recapitulate aspects of native kidney tissue, including but not limited to organizational polarity. In certain embodiments, the biomaterials direct the assembly of defined tubular structures that recapitulate aspects of native kidney tissue, including lumens. In certain embodiments, the biomaterials promote or facilitate the secretion of proteins, nucleic acids and membrane-bound vesicles from the cell populations deposited herein. In general, the one or more biomaterials used to generate the construct may also be selected to mimic or recapitulate aspects of the specific three dimensional organization or environmental niche within native kidney or renal parenchyma representing the original biological environment from which these cell populations were derived. Recreation of the original biological niche from which these cell populations were sourced is believed to further promote or facilitate cell viability and potency.

The term "cellular aggregate" or "spheroid" refers to an aggregate or assembly of cells cultured to allow 3D growth as opposed to growth as a monolayer. It is noted that the term "spheroid" does not imply that the aggregate is a geometric sphere. The aggregate may be highly organized with a well defined morphology and polarity or it may be an unorganized mass; it may include a single cell type or more than one cell type. The cells may be primary isolates, or a permanent cell line, or a combination of the two. Included in this definition are organoids and organotypic cultures. In certain embodiments, the spheroids (*e.g*., cellular aggregates or organoids) are formed in a spinner flask. In certain embodiments, the spheroids (*e.g.,* cellular aggregates or organoids) are formed in a 3-dimensional matrix.

The term "Neo-Kidney Augment (NKA)" refers to a bioactive cell formulation which is an injectable product composed of selected renal cells (SRC) formulated in a biomaterial comprised of a gelatin-based hydrogel. The term "Advance Cell Therapy (ACT)" is also used in reference to treatment with NKA. In certain embodiments, NKA is an injectable product comprising an immune-compatible renal cell population (*e.g*., immune-compatible SRCs) formulated in a biomaterial comprised of a gelatin-based hydrogel. In certain embodiments, NKA is an injectable product composed of genomically modified immunoprivileged, homologous SRCs that are incapable of immune rejection formulated in a biomaterial comprised of a gelatin-based hydrogel.

The term "subject" shall mean any single human subject, including a patient, eligible for treatment, who is experiencing or has experienced one or more signs, symptoms, or other indicators of a kidney disease. Such subjects include without limitation subjects who are newly diagnosed or previously diagnosed and are now experiencing a recurrence or relapse, or are at risk for a kidney disease, no matter the cause. The subject may have been previously treated for a kidney disease, or not so treated.

The term "patient" refers to any single animal, more preferably a mammal (including such non-human animals as, for example, dogs, cats, horses, rabbits, zoo animals, cows, pigs, sheep, and non-human primates) for which treatment is desired. Most preferably, the patient herein is a human.

The term "sample" or "patient sample" or "biological sample" shall generally mean any biological sample obtained from a subject or patient, body fluid, body tissue, cell line, tissue culture, or other source. The term includes tissue biopsies such as, for example, kidney biopsies. The term includes cultured cells such as, for example, cultured mammalian kidney cells. Methods for obtaining tissue biopsies and cultured cells from mammals are well known in the art. If the term "sample" is used alone, it shall still mean that the "sample" is a "biological sample" or "patient sample", i.e., the terms are used interchangeably.

The term "test sample" refers to a sample from a subject that has been treated by a method of the present disclosure. The test sample may originate from various sources in the mammalian subject including, without limitation, blood, semen, serum, urine, bone marrow, mucosa, tissue, etc.

The term "control" or "control sample" refers a negative or positive control in which a negative or positive result is expected to help correlate a result in the test sample. Controls that are suitable for the present disclosure include, without limitation, a sample known to exhibit indicators characteristic of normal kidney function, a sample obtained from a subject known not to have kidney disease, and a sample obtained from a subject known to have kidney disease. In addition, the control may be a sample obtained from a subject prior to being treated by a method of the present disclosure. An additional suitable control may be a test sample obtained from a subject known to have any type or stage of kidney disease, and a sample from a subject known not to have any type or stage of kidney disease. A control may be a normal healthy matched control. Those of skill in the art will appreciate other controls suitable for use in the present disclosure.

"Regeneration prognosis", "regenerative prognosis", or "prognostic for regeneration" generally refers to a forecast or prediction of the probable regenerative course or outcome of the administration or implantation of a cell population, admixture or construct described herein. For a regeneration prognosis, the forecast or prediction may be informed by one or more of the following: improvement of a functional organ (e.g., the kidney) after implantation or administration, development of a functional kidney after implantation or administration, development of improved kidney function or capacity after implantation or administration, and expression of certain markers by the native kidney following implantation or administration.

"Regenerated organ" refers to a native organ after implantation or administration of a cell population, admixture, or construct as described herein. The regenerated organ is characterized by various indicators including, without limitation, development of function or capacity in the native organ, improvement of function or capacity in the native organ, the amelioration of certain markers and physiological indices associated with disease, and/or the expression of certain markers in the native organ. Those of ordinary skill in the art will appreciate that other indicators may be suitable for characterizing a regenerated organ.

"Regenerated kidney" refers to a native kidney after implantation or administration of a cell population, admixture, or construct as described herein. The regenerated kidney is characterized by various indicators including, without limitation, development of function or capacity in the native kidney, improvement of function or capacity in the native kidney, the amelioration of certain markers and physiological indices associated with renal disease and the expression of certain markers in the native kidney. Those of ordinary skill in the art will appreciate that other indicators may be suitable for characterizing a regenerated kidney.

### Cell populations

As disclosed, a therapeutic composition or formulation provided herein contains an isolated, heterogeneous population of kidney cells that is enriched for specific bioactive components or cell types and/or depleted of specific inactive or undesired components or cell types. In certain cases such compositions and formulations are used in the treatment of kidney disease, *e.g*., providing stabilization and/or improvement and/or regeneration of kidney function and/or structure. In certain cases, the compositions contain isolated renal cell fractions that lack cellular components as compared to a healthy individual yet retain therapeutic properties, *e.g*., provide stabilization and/or improvement and/or regeneration of kidney function. In certain cases, the cell populations described herein may be derived from healthy individuals, individuals with a kidney disease, or subjects as described herein.

Included herein are therapeutic compositions of selected renal cell populations that are to be administered to a target organ or tissue in a subject. As described, a bioactive selected renal cell population generally refers to a cell population potentially having therapeutic properties upon administration to a subject. In certain cases, upon administration to a subject in need, a bioactive renal cell population can provide stabilization and/or improvement and/or repair and/or regeneration of kidney function in the subject. In certain cases, the therapeutic properties may include a repair or regenerative effect.

In certain cases, the renal cell population is an unfractionated, heterogeneous cell population or an enriched homogeneous cell population derived from a kidney. In certain cases, the heterogeneous cell population is isolated from a tissue biopsy or from whole organ tissue. In certain cases, the renal cell population is derived from an *in vitro* culture of mammalian cells, established from tissue biopsies or whole organ tissue. In certain embodiments, a renal cell population comprises subfractions or subpopulations of a heterogeneous population of renal cells, enriched for bioactive components (*e.g*., bioactive renal cells) and depleted of inactive or undesired components or cells.

In certain cases, the renal cell population expresses GGT and a cytokeratin. In certain embodiments, the GGT has a level of expression greater than about 10%, about 15%, about 18%, about 20%, about 25%, about 30%, about 35%, about 40%, about 45%, about 50%, about 55%, or about 60%. In certain embodiments, the GGT is GGT-1. In certain embodiments, cells of the renal cell population expresses GGT-1, a cytokeratin, VEGF, and KIM-1. In certain cases, greater than 18% of the cells in the renal cell population express GGT-1. In certain cases, greater than 80% of the cells in the renal cell population express the cytokeratin. In certain cases, the cytokeratin is selected from CK8, CK18, CK19 and combinations thereof. In certain cases, the cytokeratin is CK8, CK18, CK19, CK8/CK18, CK8/CK19, CK18/CK19 or CK8/CK18/CK19, wherein the "/" refers to a combination of the cytokeratins adjacent thereto. In certain cases, the cytokeratin has a level of expression greater than about 80%, about 85%, about 90%, or about 95%. In certain cases, greater than 80% of the cells in the renal cell population express the cytokeratin. In certain cases, the renal cell population expresses AQP2. In certain embodiments, less than 40% of the cells express AQP2. In certain cases, at least 3% of the cells in the renal cell population express AQP2.

In certain cases, greater than 18% of the cells within the cell population express GGT-1 and greater than 80% of the cells within the cell population express a cytokeratin. In certain cases, the cytokeratin is CK18. In certain cases, 4.5% to 81.2% of the cells in the cell population express GGT-1, 3.0% to 53.7% of the cells within the cell population express AQP2, and 81.1% to 99.7% of the cells within the cell population express CK18.

In certain cases, the renal cell population comprises cells that express one or more of any combination of the biomarkers selected from AQP1, AQP2, AQP4, Calbindin, Calponin, CD117, CD133, CD146, CD24, CD31 (PECAM-1), CD54 (ICAM-1), CD73, CK18, CK19, CK7, CK8, CK8, CK18, CK19, combinations of CK8, CK18 and CK19, Connexin 43, Cubilin, CXCR4 (Fusin), DBA, E-cadherin (CD324), EPO (erythropoeitin) GGT1, GLEPP1 (glomerular epithelial protein 1) , Haptoglobulin, Itgbl (Integrin 01), KIM-1 (kidney injury molecule-1), T1M-1 (T-cell immunoglobulin and mucin-containing molecule), MAP-2(microtubule-associated protein 2), Megalin, N-cadherin, Nephrin, NKCC (Na-K-Cl-cotransporters), OAT-1 (organic anion transporter 1), Osteopontin, Pan-cadherin, PCLP1 (podocalyxin-like 1 molecule), Podocin, SMA (smooth muscle alpha-actin), Synaptopodin, THP (tamm-horsfall protein), Vinientin, and αGST-1 (alpha glutathione S-transferase).

In certain cases, the renal cell population is enriched for epithelial cells compared to a starting population, such as a population of cells in a kidney tissue biopsy or a primary culture thereof (*e.g.,* the renal cell population comprises at least about 5%, 10%, 15%, 20%, or 25% more epithelial cells than the starting population). In certain cases, the renal cell population is enriched for tubular cells compared to a starting population, such as a population of cells in a kidney tissue biopsy or a primary culture thereof (*e.g.,* the renal cell population comprises at least about 5%, 10%, 15%, 20%, or 25% more tubular cells than the starting population). In certain cases, the tubular cells comprise proximal tubular cells. In certain cases, the renal cell population has a lesser proportion of distal tubular cells, collecting duct cells, endocrine cells, vascular cells, or progenitor-like cells compared to the starting population. In certain cases, the renal cell population has a lesser proportion of distal tubular cells compared to the starting population. In certain cases, the renal cell population has a lesser proportion of collecting duct cells compared to the starting population. In certain cases, the renal cell population has a lesser proportion of endocrine cells compared to the starting population. In certain cases, the renal cell population has a lesser proportion of vascular cells compared to the starting population. In certain cases, the renal cell population has a lesser proportion of progenitor-like cells compared to the starting population. In certain cases, the renal cell population has a greater proportion of tubular cells and lesser proportions of EPO producing cells, glomerular cells, and vascular cells when compared to the non-enriched population (*e.g.,* a starting kidney cell population). In certain cases, the renal cell population has a greater proportion of tubular cells and lesser proportions of EPO producing cells and vascular cells when compared to the non-enriched population. In certain cases, the renal cell population has a greater proportion of tubular cells and lesser proportions of glomerular cells and vascular cells when compared to the non-enriched population.

In certain cases, cells of the renal cell population, express hyaluronic acid (HA). In certain cases, the size range of HA is from about 5 kDa to about 20000 kDa. In certain cases, the HA has a molecular weight of 5 kDa, 60 kDa, 800 kDa, and/or 3000 kDa. In certain cases, the renal cell population synthesizes and/or stimulate synthesis of high molecular weight HA through expression of Hyaluronic Acid Synthase-2 (HAS-2), especially after intra-renal implantation. In certain cases, cells of the renal cell population express higher molecular weight species of HA *in vitro* and/or *in vivo,* through the actions of HAS-2. In certain cases, cells of the renal cell population express higher molecular weight species of HA both *in vitro* and *in vivo,* through the actions of HAS-2. In certain cases, a higher molecular weight species of HA is HA having a molecular weight of at least 100 kDa. In certain cases, the higher molecular weight species of HA is HA having a molecular weight from about 800 kDa to about 3500 kDa. In certain cases the higher molecular weight species of HA is HA having a molecular weight from about 800 kDa to about 3000 kDa. In certain cases, the higher molecular weight species of HA is HA having a molecular weight of at least 800 kDa. In certain cases, the higher molecular weight species of HA is HA having a molecular weight of at least 3,000 kDa. In certain cases, the higher molecular weight species of HA is HA having a molecular weight of about 800 kDa. In certain cases, the higher molecular weight species of HA is HA having a molecular weight of about 3000 kDa. In certain cases, HAS-2 synthesizes HA with a molecular weight of 2×10⁵ to 2×10⁶ Da. In certain cases, smaller species of HA are formed through the action of degradative hyaluronidases. In certain cases, the higher molecular weight species of HA is HA having a molecular weight from about 200 kDa to about 2000 kDa. In certain cases, the higher molecular weight species of HA is HA having a molecular weight of about 200 kDa. In certain cases, the higher molecular weight species of HA is HA having a molecular weight of about 2000 kDa. In certain cases, the higher molecular weight species of HA is HA having a molecular weight of at least 200 kDa. In certain cases, the higher molecular weight species of HA is HA having a molecular weight of at least 2000 kDa. In certain cases, the higher molecular weight species of HA is HA having a molecular weight of at least 5000 kDa. In certain cases, the higher molecular weight species of HA is HA having a molecular weight of at least 10000 kDa. In certain cases embodiments, the higher molecular weight species of HA is HA having a molecular weight of at least 15000 kDa. In certain cases, the higher molecular weight species of HA is HA having a molecular weight of about 20000 kDa.

In certain cases, the population comprises cells that are capable of receptor-mediated albumin transport.

In certain cases, cells of the renal cell population are hypoxia resistant.

In certain cases, the renal cell population comprises one or more cell types that express one or more of any combination of: megalin, cubilin, N-cadherin, E-cadherin, Aquaporin-1, and Aquaporin-2.

In certain cases, the renal cell population comprises one or more cell types that express one or more of any combination of: megalin, cubilin, hyaluronic acid synthase 2 (HAS2), Vitamin D3 25-Hydroxylase (CYP2D25), N-cadherin (Ncad), E-cadherin (Ecad), Aquaporin-1 (Aqp1), Aquaporin-2 (Aqp2), RAB17, member RAS oncogene family (Rab17), GATA binding protein 3 (Gata3), FXYD domain-containing ion transport regulator 4 (Fxyd4), solute carrier family 9 (sodium/hydrogen exchanger), member 4 (S1c9a4), aldehyde dehydrogenase 3 family, member B1 (Aldh3b1), aldehyde dehydrogenase 1 family, member A3 (Aldh1a3), and Calpain-8 (Capn8).

In certain cases, the renal cell population comprises one or more cell types that express one or more of any combination of: megalin, cubilin, hyaluronic acid synthase 2 (HAS2), Vitamin D3 25-Hydroxylase (CYP2D25), N-cadherin (Ncad), E-cadherin (Ecad), Aquaporin-1 (Aqp1), Aquaporin-2 (Aqp2), RAB17, member RAS oncogene family (Rab17), GATA binding protein 3 (Gata3), FXYD domain-containing ion transport regulator 4 (Fxyd4), solute carrier family 9 (sodium/hydrogen exchanger), member 4 (S1c9a4), aldehyde dehydrogenase 3 family, member 81 (Aldh3b1), aldehyde dehydrogenase 1 family, member A3 (Aldh1a3), and Calpain-8 (Capn8), and Aquaporin-4 (Aqp4).

In certain cases, the renal cell population comprises one or more cell types that express one or more of any combination of: aquaporin 7 (Aqp7), FXYD domain-containing ion transport regulator 2 (Fxyd2), solute carrier family 17 (sodium phosphate), member 3 (Slc17a3), solute carrier family 3, member 1 (Slc3a1), claudin 2 (Cldn2), napsin A aspartic peptidase (Napsa), solute carrier family 2 (facilitated glucose transporter), member 2 (S1c2a2), alanyl (membrane) aminopeptidase (Anpep), transmembrane protein 27 (Tmem27), acyl-CoA synthetase medium-chain family member 2 (Acsm2), glutathione peroxidase 3 (Gpx3), fructose-1,6-biphosphatase 1 (Fbp1), alanine-glyoxylate aminotransferase 2 (Agxt2), platelet endothelial cell adhesion molecule (Pecam), and podocin (Podn).

In certain cases, the renal cell population comprises one or more cell types that express one or more of any combination of: PECAM, VEGF, KDR, HIF1a, CD31, CD146, Podocin (Podn), and Nephrin (Neph), chemokine (C-X-C motif) receptor 4 (Cxcr4), endothelin receptor type B (Ednrb), collagen, type V, alpha 2 (Col5a2), Cadherin 5 (Cdh5), plasminogen activator, tissue (Plat), angiopoietin 2 (Angpt2), kinase insert domain protein receptor (Kdr), secreted protein, acidic, cysteine-rich (osteonectin) (Sparc), serglycin (Srgn), TIMP metallopeptidase inhibitor 3 (Timp3), Wilms tumor 1 (Wt1), wingless-type MMTV integration site family, member 4 (Wnt4), regulator of G-protein signaling 4 (Rgs4), Erythropoietin (EPO).

In certain cases, the renal cell population comprises one or more cell types that express one or more of any combination of: PECAM, vEGF, KDR, HIF1a, podocin, nephrin, EPO, CK7, CK8/18/19.

In certain cases, the renal cell population comprises one or more cell types that express one or more of any combination of: PECAM, vEGF, KDR, HIF1a, CD31, CD146.

In certain cases, the renal cell population comprises one or more cell types that express one or more of any combination of: Podocin (Podn), and Nephrin (Neph).

In certain cases, the renal cell population comprises one or more cell types that express one or more of any combination of: PECAM, vEGF, KDR, HIF1a, and EPO.

In certain cases, the presence (*e.g*., expression) and/or level/amount of various biomarkers in a sample or cell population can be analyzed by a number of methodologies, many of which are known in the art and understood by the skilled artisan, including, but not limited to, immunohistochemical ("IHC"), Western blot analysis, immunoprecipitation, molecular binding assays, ELISA, ELIFA, fluorescence activated cell sorting ("FACS"), MassARRAY, proteomics, biochemical enzymatic activity assays, in situ hybridization, Southern analysis, Northern analysis, whole genome sequencing, polymerase chain reaction ("PCR") including quantitative real time PCR ("qRT-PCR") and other amplification type detection methods, such as, for example, branched DNA, SISBA, TMA and the like), RNA-Seq, FISH, microarray analysis, gene expression profiling, and/or serial analysis of gene expression ("SAGE"), as well as any one of the wide variety of assays that can be performed by protein, gene, and/or tissue array analysis. Non-limiting examples of protocols for evaluating the status of genes and gene products include Northern Blotting, Southern Blotting, Immunoblotting, and PCR Analysis. In certain cases, multiplexed immunoassays such as those available from Rules Based Medicine or Meso Scale Discovery may also be used. In certain cases, the presence (*e.g*., expression) and/or level/amount of various biomarkers in a sample or cell population can be analyzed by a number of methodologies, many of which are known in the art and understood by the skilled artisan, including, but not limited to, "-omics" platforms such as genome-wide transcriptomics, proteomics, secretomics, lipidomics, phospatomics, exosomics etc., wherein high-throughput methodologies are coupled with computational biology and bioinformatics techniques to elucidate a complete biological signature of genes, miRNA, proteins, secreted proteins, lipids, etc. that are expressed and not expressed by the cell population under consideration.

In certain cases, a method of detecting the presence of two or more biomarkers in a renal cell population comprises contacting the sample with an antibody directed to a biomarker under conditions permissive for binding of the antibody to its cognate ligand (*i.e.,* biomarker), and detecting the presence of the bound antibody, *e.g.,* by detecting whether a complex is formed between the antibody and the biomarker. In certain cases, the detection of the presence of one or more biomarkers is by immunohistochemistry. The term "detecting" as used herein encompasses quantitative and/or qualitative detection.

In certain cases, a renal cell population are identified with one or more reagents that allow detection of a biomarker disclosed herein, such as AQP1, AQP2, AQP4, Calbindin, Calponin, CD117, CD133, CD146, CD24, CD31 (PECAM-1), CD54 (ICAM-1), CD73, CK18, CK19, CK7, CK8, CK8/18, CK8/18/19, Connexin 43, Cubilin, CXCR4 (Fusin), DBA, E-cadherin (CD324), EPO (erythropoeitin), GGT1, GLEPP1 (glomerular epithelial protein 1), Haptoglobulin, Itgbl (Integrin p), KIM-1 (kidney injury molecule-1), T1M-1 (T-cell immunoglobulin and mucirs-containing molecule), MAP-2 (microtubule-associated protein 2), Megalin, N-cadherin, Nephrin, NKCC (Na-K-Cl-cotransporters), OAT-1 (organic anion transporter 1), Osteopontin, Pan-cadherin, PCLP1 (podocalyxin-like 1 molecule), Podocin, SMA (smooth muscle alpha-actin), Synaptopodin, THP (tamm-horsfall protein), Vimentin, and αGST-1 (alpha glutathione 5-transferase). In certain cases a biomarker is detected by a monoclonal or polyclonal antibody.

In certain cases, the source of cells is the same as the intended target organ or tissue. In certain cases BRCs or SRCs may be sourced from the kidney to be used in a formulation to be administered to the kidney. In certain cases, the cell population is derived from a kidney biopsy. In certain cases, a cell populations is derived from whole kidney tissue. In certain cases, a cell population is derived from in vitro cultures of mammalian kidney cells, established from kidney biopsies or whole kidney tissue.

In certain cases, the BRCs or SRCs comprise heterogeneous mixtures or fractions of bioactive renal cells. In certain cases, the BRCs or SRCs may be derived from or are themselves renal cell fractions from healthy individuals. In certain cases, included herein is a renal cell population or fraction obtained from an unhealthy individual that may lack certain cell types when compared to the renal cell population of a healthy individual (*e.g*., in a kidney or biopsy thereof). In certain cases, provided herein is a therapeutically-active cell population lacking cell types compared to a healthy individual. In certain cases, a cell population is isolated and expanded from an autologous cell population.

In certain cases, SRCs are obtained from isolation and expansion of renal cells from a patient's renal cortical tissue via a kidney biopsy. In certain cases, renal cells are isolated from the kidney tissue by enzymatic digestion, expanded using standard cell culture techniques, and selected by centrifugation across a density boundary, barrier, or interface from the expanded renal cells. In certain cases, renal cells are isolated from the kidney tissue by enzymatic digestion, expanded using standard cell culture techniques, and selected by continuous or discontinuous single or multistep density gradient centrifugation from the expanded renal cells. In certain cases, SRCs are composed primarily of renal epithelial cells which are known for their regenerative potential. In certain cases, other parenchymal (vascular) and stromal cells may be present in the autologous SRC population.

As described above, BRC are an isolated population of regenerative renal cells naturally involved in renal repair and regeneration. In certain cases, BRC are obtained from renal cells isolated from kidney tissue by enzymatic digestion and expanded using standard cell culture techniques. The cell culture medium may be designed to expand bioactive renal cells with regenerative capacity. In certain cases, BRC are obtained from renal cells isolated from kidney tissue by enzymatic digestion, have been genetically modified (*e.g*., genomically modified and/or modified via RNAi) to render them immunoprivileged or incapable of rejection, and expanded using standard cell culture techniques. The cell culture medium may be designed to expand immunoprivileged bioactive renal cells with regenerative capacity. In certain cases, the cell culture medium does not contain any differentiation factors. In certain cases, the expanded heterogeneous mixtures of renal cells are cultured in hypoxic conditions to further enrich the composition of cells with regenerative capacity. Without wishing to be bound by theory, this may be due to one or more of the following phenomena: 1) selective survival, death, or proliferation of specific cellular components during the hypoxic culture period; 2) alterations in cell granularity and/or size in response to the hypoxic culture, thereby effecting alterations in buoyant density and subsequent localization during density gradient separation; and 3) alterations in cell gene / protein expression in response to the hypoxic culture period, thereby resulting in differential characteristics of the cells within the isolated and expanded population.

In certain cases, the bioactive renal cell population is obtained from isolation and expansion of renal cells from kidney tissue (such as tissue obtained from a biopsy) under culturing conditions that enrich for cells capable of kidney regeneration.

In certain cases, renal cells from kidney tissue (such as tissue obtained from a biopsy) are passaged 1, 2, 3, 4, 5, or more times to produce expanded bioactive renal cells (such as a cell population enriched for cells capable of kidney regeneration). In certain cases, renal cells from kidney tissue (such as tissue obtained from a biopsy) are passaged 1 time to produce expanded bioactive renal cells. In certain cases, renal cells from kidney tissue (such as tissue obtained from a biopsy) are passaged 2 times to produce expanded bioactive renal cells. In certain cases, renal cells from kidney tissue (such as tissue obtained from a biopsy) are passaged 3 times to produce expanded bioactive renal cells. In certain cases, renal cells from kidney tissue (such as tissue obtained from a biopsy) are passaged 4 times to produce expanded bioactive renal cells. In certain cases, renal cells from kidney tissue (such as tissue obtained from a biopsy) are passaged 5 times to produce expanded bioactive renal cells. In certain cases, passaging the cells depletes the cell population of non-bioactive renal cells. In certain cases, passaging the cells depletes the cell population of at least one cell type. In certain cases, passaging the cells depletes the cell population of cells having a density greater than 1.095 g/ml. In certain cases, passaging the cells depletes the cell population of small cells of low granularity. In certain cases, passaging the cells depletes the cell population of cells that are smaller than erythrocytes. In certain cases, passaging the cells depletes the cell population of cells with a diameter of less than 6 µm. In certain cases, passaging cells depletes cell population of cells with a diameter less than 2 µm. In certain cases, passaging the cells depletes the cell population of cells with lower granularity than erythrocytes. In certain cases, the viability of the cell population increases after 1 or more passages. In certain cases, descriptions of small cells and low granularity are used when analyzing cells by fluorescence activated cell sorting (FACs), *e.g.,* using the X-Y axis of a scatter-plot of where the cells show up.

In certain cases, the expanded bioactive renal cells are grown under hypoxic conditions for at least about 6, 9, 10, 12, or 24 hours but less than 48 hours, or from 6 to 9 hours, or from 6 to 48 hours, or from about 12 to about 15 hours, or about 8 hours, or about 12 hours, or about 24 hours, or about 36 hours, or about 48 hours. In certain cases, cells grown under hypoxic conditions are selected based on density. In certain cases, the bioactive renal cell population is a selected renal cell (SRC) population obtained after continuous or discontinuous (single step or multistep) density gradient separation of the expanded renal cells (*e.g.,* after passaging and/or culture under hypoxic conditions). In certain cases, the bioactive renal cell population is a selected renal cell (SRC) population obtained after separation of the expanded renal cells by centrifugation across a density boundary, barrier, or interface (*e.g*., after passaging and/or culture under hypoxic condutions). In certain cases a hypoxic culture condition is a culture condition in which cells are subjected to a reduction in available oxygen levels in the culture system relative to standard culture conditions in which cells are cultured at atmospheric oxygen levels (about 21%). In certain cases, cells cultured under hypoxic culture conditions are cultured at an oxygen level of about 5% to about 15%, or about 5% to about 10%, or about 2% to about 5%, or about 2% to about 7%, or about 2% or about 3%, or about 4%, or about 5%. In certain cases, the SRCs exhibit a buoyant density greater than about 1.0419 g/mL. In certain cases, the SRCs exhibit a buoyant density greater than about 1.04 g/mL. In certain cases, the SRCs exhibit a buoyant density greater than about 1.045 g/mL. In certain cases, the BRCs or SRCs contain a greater percentage of one or more cell populations and lacks or is deficient in one or more other cell populations, as compared to a starting kidney cell population.

In certain cases, expanded bioactive renal cells may be subjected to density gradient separation to obtain SRCs. In certain cases, the SRCs are then genetically modified. In certain cases, genetically modified (*e.g*., genomically modified and/or modified via RNAi) BRCs are subjected to density gradient separation to obtain genetically modified SRCs. In certain cases, genetically modified (*e.g*., genomically modified and/or modified via RNAi) BRCs are subjected to both hypoxic culture conditions and density gradient separation to obtain genetically modified SRCs. In certain cases, continuous or discontinuous single step or multistep density gradient centrifugation is used to separate harvested renal cell populations based on cell buoyant density. In certain cases, expanded bioactive renal cells may be separated by centrifugation across a density boundary, barrier or interface to obtain SRCs. In certain cases, centrifugation across a density boundary or interface is used to separate harvested renal cell populations based on cell buoyant density. In certain cases, the SRCs are generated by using, in part, OPTIPREP (Axis-Shield) medium, comprising a solution of 60% (w/v) of the nonionic iodinated compound iodixanol in water. One of skill in the art, however, will recognize that other media, density gradients (continuous or discontinuous), density boundaries, barriers, interfaces or other means, *e.g*., immunological separation using cell surface markers known in the art, comprising necessary features for isolating cell populations described herein may be used to obtain bioactive renal cells. In certain cases, a cellular fraction exhibiting buoyant density greater than about 1.04 g/mL is collected after centrifugation as a distinct pellet. In certain cases, cells maintaining a buoyant density of less than 1.04 g/mL are excluded and discarded. In certain cases, a cellular fraction exhibiting buoyant density greater than about 1.0419 g/mL is collected after centrifugation as a distinct pellet. In certain cases, cells maintaining a buoyant density of less than 1.0419 g/mL are excluded and discarded. In certain cases, a cellular fraction exhibiting buoyant density greater than about 1.045 g/mL is collected after centrifugation as a distinct pellet. In certain cases, cells maintaining a buoyant density of less than 1.045 g/mL are excluded and discarded.

In certain cases, cell buoyant density is used to obtain an SRC population and/or to determine whether a renal cell population is a bioactive renal cell population. In certain cases, cell buoyant density is used to isolate bioactive renal cells. In certain cases, cell buoyant density is determined by centrifugation across a single-step OptiPrep (7% iodixanol; 60% (w/v) in OptiMEM) density interface (single step discontinuous density gradient). Optiprep is a 60% w/v solution of iodixanol in water. When used in an exemplary density interface or single step discontinuous density gradient, the Optiprep is diluted with OptiMEM (a cell culturing basal medium) to form a final solution of 7% iodixanol (in water and OptiMEM). The formulation of OptiMEM is a modification of Eagle's Minimal Essential Medium, buffered with HEPES and sodium bicarbonate, and supplemented with hypoxanthine, thymidine, sodium pyruvate, L-glutamine or GLUTAMAX, trace elements and growth factors. The protein level is minimal (15 µg/mL), with insulin and transferrin being the only protein supplements. Phenol red is included at a reduced concentration as a pH indicator. In certain cases OptiMEM may be supplemented with 2-mercaptoethanol prior to use.

In certain cases, the OptiPrep solution is prepared and refractive index indicative of desired density is measured (R.I. 1.3456 +/- 0.0004) prior to use. In certain cases, renal cells are layered on top of the solution. In certain cases, the density interface or single step discontinuous density gradient is centrifuged at 800 g for 20 min at room temperature (without brake) in either a centrifuge tube (*e.g.,* a 50ml conical tube) or a cell processor (*e.g.* COBE 2991). In certain cases, the cellular fraction exhibiting buoyant density greater than about 1.04 g/mL is collected after centrifugation as a distinct pellet. In certain cases, cells maintaining a buoyant density of less than 1.04 g/mL are excluded and discarded. In certain cases, the cellular fraction exhibiting buoyant density greater than about 1.0419 g/mL is collected after centrifugation as a distinct pellet. In certain cases, cells maintaining a buoyant density of less than 1.0419 g/mL are excluded and discarded. In certain cases, the cellular fraction exhibiting buoyant density greater than about 1.045 g/mL is collected after centrifugation as a distinct pellet. In certain cases, cells maintaining a buoyant density of less than 1.045 g/mL are excluded and discarded. In certain cases, prior to the assessment of cell density or selection based on density, cells are cultured until they are at least 50% confluent and incubated overnight (*e.g.,* at least about 8 or 12 hours) in a hypoxic incubator set for 2% oxygen in a 5% CO₂ environment at 37°C.

In certain cases, cells obtained from a kidney sample are expanded and then processed (*e.g.* by hypoxia and centrifugation separation) to provide a SRC population. In certain cases, an SRC population is produced using reagents and procedures described herein. In certain cases, a sample of cells from an SRC population is tested for viability before cells of the population are administration to a subject. In certain cases, a sample of cells from an SRC population is tested for the expression of one or more of the markers disclosed herein before cells of the population administration to a subject. Examples

of compositions and methods for preparing SRCs are disclosed in U.S. Patent Application Publication No. 2017/0281684 A1.

In certain cases, the BRCs or SRCs are derived from a native autologous or allogeneic kidney sample. In certain cases, the BRCs or SRCs are derived from a non-autologous kidney sample. In certain cases, the sample may be obtained by kidney biopsy.

In certain cases, renal cell isolation and expansion provides a mixture of renal cell types including renal epithelial cells and stromal cells. In certain SRC are obtained by continuous or discontinuous density gradient separation of the expanded renal cells. In certain cases, the primary cell type in the density gradient separated SRC population is of tubular epithelial phenotype. In certain cases, SRC are obtained by separation of the expanded renal cells by centrifugation across a density boundary, barrier, or interface. In certain cases, the primary cell type in the SRC population separated across a density boundary/barrier/interface is of tubular epithelial phenotype. In certain cases, the characteristics of SRC obtained from expanded renal cells are evaluated using a multi-pronged approach. In certain cases, cell morphology, growth kinetics and cell viability are monitored during the renal cell expansion process. In certain cases cases, SRC buoyant density and viability is characterized by centrifugation on or through a density gradient medium and Trypan Blue exclusion. In certain cases, SRC phenotype is characterized by flow cytometry and SRC function is demonstrated by expression of VEGF and KIM-1. In certain cases, cell function of SRC, pre-formulation, can also be evaluated by measuring the activity of two specific enzymes; GGT (γ-glutamyl transpeptidase) and LAP (leucine aminopeptidase), found in kidney proximal tubules.

In certain cases, cellular features that contribute to separation of cellular subpopulations via a density medium (size and granularity) can be exploited to separate cellular subpopulations via flow cytometry (forward scatter=a reflection of size via flow cytometry, and side scatter=a reflection of granularity). In certain cases, a density gradient or separation medium should have low toxicity towards the specific cells of interest. In certain cases while the density medium should have low toxicity toward the specific cells of interest, the instant disclosure contemplates the use of mediums which play a role in the selection process of the cells of interest. In certain cases, and without wishing to be bound by theory, it appears that the cell populations disclosed herein recovered by the medium comprising iodixanol are iodixanol-resistant, as there is an appreciable loss of cells between the loading and recovery steps, suggesting that exposure to iodixanol under the conditions of the density gradient or density boundary, density, barrier, or density interface leads to elimination of certain cells. In certain cases, cells appearing after an iodixanol density gradient or density interface separation are resistant to any untoward effects of iodixanol and/or density gradient or interface exposure. In certain cases, a contrast medium comprising a mild to moderate nephrotoxin is used in the isolation and/or selection of a cell population, *e.g.* a SRC population. In certain cases, SRCs are iodixanol-resistant. In certain cases, the density medium should not bind to proteins in human plasma or adversely affect key functions of the cells of interest.

In certain cases, a cell population has been enriched and/or depleted of one or more kidney cell types using fluorescent activated cell sorting (FACS). In certain cases, kidney cell types may be enriched and/or depleted using BD FACSAria^{™} or equivalent. In certain embodiments, kidney cell types may be enriched and/or depleted using FACSAria III^{™} or equivalent.

In certain cases, a cell population has been enriched and/or depleted of one or more kidney cell types using magnetic cell sorting. In certain cases, one or more kidney cell types may be enriched and/or depleted using the Miltenyi autoMACS^{®} system or equivalent.

In certain cases, a renal cell population has been subject to three-dimensional culturing. In certain cases, the methods of culturing the cell populations are via continuous perfusion. In certain cases, the cell populations cultured via three-dimensional culturing and continuous perfusion demonstrate greater cellularity and interconnectivity when compared to cell populations cultured statically. In certain cases, the cell populations cultured via three dimensional culturing and continuous perfusion demonstrate greater expression of EPO, as well as enhanced expression of renal tubule-associate genes such as E-cadherin when compared to static cultures of such cell populations. In certain cases, a cell population cultured via continuous perfusion demonstrates a greater level of glucose and glutamine consumption when compared to a cell population cultured statically.

In certain cases, low or hypoxic oxygen conditions may be used in the methods to prepare a cell population provided for herein. In certain cases, a method of preparing a cell population may be used without the step of low oxygen conditioning. In certain cases, normoxic conditions may be used.

In certain embodiments, a renal cell population has been isolated and/or cultured from kidney tissue. Non-limiting examples of methods are disclosed herein for separating and isolating the renal cellular components, e.g., enriched cell populations that will be used in the formulations for therapeutic use, including the treatment of kidney disease, anemia, EPO deficiency, tubular transport deficiency, and glomerular filtration deficiency. In certain cases, a cell population is isolated from freshly digested, *i.e*., mechanically or enzymatically digested, kidney tissue or from a heterogeneous *in vitro* culture of mammalian kidney cells.

In certain cases, the renal cell population comprises EPO-producing kidney cells. In certain cases, a subject has anemia and/or EPO deficiency. In certain cases, EPO-producing kidney cell populations that are characterized by EPO expression and bioresponsiveness to oxygen, such that a reduction in the oxygen tension of the culture system results in an induction in the expression of EPO. In certain cases, the EPO-producing cell populations are enriched for EPO-producing cells. In certain cases, the EPO expression is induced when the cell population is cultured under conditions where the cells are subjected to a reduction in available oxygen levels in the culture system as compared to a cell population cultured at normal atmospheric (about 21%) levels of available oxygen. In certain cases, EPO-producing cells cultured in lower oxygen conditions express greater levels of EPO relative to EPO-producing cells cultured at normal oxygen conditions. In general, the culturing of cells at reduced levels of available oxygen (also referred to as hypoxic culture conditions) means that the level of reduced oxygen is reduced relative to the culturing of cells at normal atmospheric levels of available oxygen (also referred to as normal or normoxic culture conditions). In certain cases, hypoxic cell culture conditions include culturing cells at about less than 1% oxygen, about less than 2% oxygen, about less than 3% oxygen, about less than 4% oxygen, or about less than 5% oxygen. In certain cases, normal or normoxic culture conditions include culturing cells at about 10% oxygen, about 12% oxygen, about 13% oxygen, about 14% oxygen, about 15% oxygen, about 16% oxygen, about 17% oxygen, about 18% oxygen, about 19% oxygen, about 20% oxygen, or about 21% oxygen.

In certain cases, induction or increased expression of EPO is obtained and can be observed by culturing cells at about less than 5% available oxygen and comparing EPO expression levels to cells cultured at atmospheric (about 21%) oxygen. In certain cases, the induction of EPO is obtained in a culture of cells capable of expressing EPO by a method that includes a first culture phase in which the culture of cells is cultivated at atmospheric oxygen (about 21%) for some period of time and a second culture phase in which the available oxygen levels are reduced and the same cells are cultured at about less than 5% available oxygen. In certain cases, the EPO expression that is responsive to hypoxic conditions is regulated by HIF1α. In certain , other oxygen manipulation culture conditions known in the art may be used for the cells described herein.

In certain embodiments, the formulation contains enriched populations of EPO-producing mammalian cells characterized by bio-responsiveness (*e.g*., EPO expression) to perfusion conditions. In certain cases, the perfusion conditions include transient, intermittent, or continuous fluid flow (perfusion). In certain cases, the EPO expression is mechanically-induced when the media in which the cells are cultured is intermittently or continuously circulated or agitated in such a manner that dynamic forces are transferred to the cells via the flow. In certain cases the cells subjected to the transient, intermittent, or continuous fluid flow are cultured in such a manner that they are present as three-dimensional structures in or on a material that provides framework and/or space for such three-dimensional structures to form. In certain cases, the cells are cultured on porous beads and subjected to intermittent or continuous fluid flow by means of a rocking platform, orbiting platform, or spinner flask. In certain cases, the cells are cultured on three-dimensional scaffolding and placed into a device whereby the scaffold is stationary and fluid flows directionally through or across the scaffolding. Those of ordinary skill in the art will appreciate that other perfusion culture conditions known in the art may be used for the cells described herein.

In certain cases, a cell population is derived from a kidney biopsy. In certain embodiments, a cell population is derived from whole kidney tissue. In certain cases, a cell population is derived from an *in vitro* culture of mammalian kidney cells, established from kidney biopsies or whole kidney tissue. In certain cases, the renal cell population is a SRC population. In certain cases, a cell population is an unfractionated cell populations, also referred to herein as a non-enriched cell population.

Compositions containing a variety of active agents (*e.g.,* other than renal cells) are included herein. Non-limiting examples of suitable active agents include, without limitation, cellular aggregates, acellular biomaterials, secreted products from bioactive cells, large and small molecule therapeutics, as well as combinations thereof. For example, one type of bioactive cells may be combined with biomaterial-based microcarriers with or without therapeutic molecules or another type of bioactive cells. In certain embodiments, unattached cells may be combined with acellular particles.

In certain cases, cells of the renal cell population are within spheroids. In certain cases, the renal cell population is in the form of spheroids. In certain cases, spheroids comprising bioactive renal cells are administered to a subject. In certain cases, the spheroids comprise at least one non-renal cell type or population of cells. In certain cases, the a spheroids are produced in a method comprising (i) combining a bioactive renal cell population and a non-renal cell population, and (ii) culturing the bioactive renal cell population and the non-renal cell population in a 3-dimensional culture system comprising a spinner flask until the spheroids form.

In certain cases, the non-renal cell population comprises an endothelial cell population or an endothelial progenitor cell population. In certain cases, the bioactive cell population is an endothelial cell population. In certain cases, the endothelial cell population is a cell line. In certain cases, the endothelial cell population comprises human umbilical vein endothelial cells (HUVECs). In certain cases, the non-renal cell population is a mesenchymal stem cell population. In certain cases, the non-renal cell population is a stem cell population of hematopoietic, mammary, intestinal, placental, lung, bone marrow, blood, umbilical cord, endothelial, dental pulp, adipose, neural, olfactory, neural crest, or testicular origin. In certain cases, the non-renal cell population is an adipose-derived progenitor cell population. In certain cases, the cell populations are xenogeneic, syngeneic, allogeneic, autologous or combinations thereof. In certain cases, the bioactive renal cell population and non-renal cell population are cultured at a ratio of from 0.1:9.9 to 9.9:0.1. In certain cases, the bioactive renal cell population and non-renal cell population are cultured at a ratio of about 1:1. In certain cases, the renal cell population and bioactive cell population are suspended in growth medium.

The expanded bioactive renal cells may be further subjected to continuous or discontinuous density medium separation to obtain the SRC. Specifically, continuous or discontinuous single step or multistep density gradient centrifugation is used to separate harvested renal cell populations based on cell buoyant density. In certain cases, the expanded bioactive renal cells may be further subjected to separation by centrifugation across a density boundary, barrier, or interface to obtain the SRC. Specifically, centrifugation across a density boundary, barrier, or interface is used to separate harvested renal cell populations based on cell buoyant density. In certain cases, the SRC are generated by using, in part, the OPTIPREP (Axis-Shield) medium, comprising a 60% solution of the nonionic iodinated compound iodixanol in water. One of skill in the art, however, will recognize that any density gradient medium without limitation of specific medium or other means, e.g., immunological separation using cell surface markers known in the art, comprising necessary features for isolating the cell populations of the instant disclosure may be used in accordance with the disclosure. For example, Percoll or sucrose may be used to form a density gradient or density boundary. In certain cases, the cellular fraction exhibiting buoyant density greater than about 1.04 g/mL is collected after centrifugation as a distinct pellet. In certain cases, cells maintaining a buoyant density of less than 1.04 g/mL are excluded and discarded. In certain cases, the cellular fraction exhibiting buoyant density greater than about 1.0419 g/mL is collected after centrifugation as a distinct pellet. In certain cases, cells maintaining a buoyant density of less than 1.0419 g/mL are excluded and discarded. In certain cases, the cellular fraction exhibiting buoyant density greater than about 1.045 g/mL is collected after centrifugation as a distinct pellet. In certain cases, cells maintaining a buoyant density of less than 1.045 g/mL are excluded and discarded.

The therapeutic compositions, and formulations thereof, of the present disclosure may contain isolated, heterogeneous populations of kidney cells, and/or admixtures thereof, enriched for specific bioactive components or cell types and/or depleted of specific inactive or undesired components or cell types for use in the treatment of kidney disease that have been genetically modified by removing genetic components encoding for cell surface antigens that are responsible for immune rejection in the recipient, i.e., providing stabilization and/or improvement and/or regeneration of kidney function and/or structure. Non-limiting examples of cells for providing such stabilization and/or improvement were previously described in Presnell et al. U.S. 8,318,484 and Ilagan et al. PCT/US2011/036347 and Jain et al. PCT/US2016/044866

The compositions may contain isolated renal cell fractions that lack cellular components as compared to a healthy individual yet retain therapeutic properties, i.e., provide stabilization and/or improvement and/or regeneration of kidney function. The cell populations, cell fractions, and/or admixtures of cells described herein may be derived from healthy individuals, individuals with a kidney disease, or subjects as described herein.

The present disclosure contemplates therapeutic compositions of genetically modified (*e.g.,* genomically modified and/or modified via RNAi) immunoprivileged selected renal cell populations that are to be administered to target organs or tissue in a subject in need. A bioactive selected renal cell population generally refers to a cell population potentially having therapeutic properties upon administration to a subject. For example, upon administration to a subject in need, a bioactive renal cell population can provide stabilization and/or improvement and/or repair and/or regeneration of kidney function in the subject. The therapeutic properties may include a repair or regenerative effect.

In certain cases, the source of cells is the same as the intended target organ or tissue. For example, BRCs and/or SRCs may be sourced from the kidney to be used in a formulation to be administered to the kidney. In certain cases, the cell populations are derived from a kidney biopsy. In certain cases, the cell populations are derived from whole kidney tissue. In certain cases, the cell populations are derived from in vitro cultures of mammalian kidney cells, established from kidney biopsies or whole kidney tissue. In certain cases, the BRCs and/or SRCs comprise heterogeneous mixtures or fractions of bioactive renal cells. The BRCs and/or SRCs may be derived from or are themselves renal cell fractions from healthy individuals. In addition, the present disclosure provides renal cell fractions obtained from an unhealthy individual that may lack certain cellular components when compared to the corresponding renal cell fractions of a healthy individual, yet still retain therapeutic properties. The present disclosure also provides therapeutically-active cell populations lacking cellular components compared to a healthy individual, which cell populations can be, in certain cases, isolated and expanded from autologous sources in various disease states.

In certain cases, the SRCs are obtained from isolation and expansion of renal cells from a patient's renal cortical tissue via a kidney biopsy. Renal cells are isolated from the kidney tissue by enzymatic digestion, expanded using standard cell culture techniques, and selected by centrifugation of the expanded renal cells across a density boundary, barrier, or interface. In this cases, SRC are composed primarily of renal tubular epithelial cells which are known for their regenerative potential (Bonventre JV. Dedifferentiation and proliferation of surviving epithelial cells in acute renal failure. J Am Soc Nephrol. 2003;14(Suppl. 1):S55-61; Humphreys BD, Czerniak S, DiRocco DP, et al. Repair of injured proximal tubule does not involve specialized progenitors. PNAS. 2011;108:9226-31; Humphreys BD, Valerius MT, Kobayashi A, et al. Intrinsic epithelial cells repair the kidney after injury. Cell Stem Cell. 2008;2:284-91). Other parenchymal (vascular) and stromal cells may be present in the autologous SRC population. In certain cases, renal cells are selected by centrifugation through a continuous or discontinuous single step or multistep gradient.

Included herein are therapeutic compositions of genetically modified (*e.g*., genomically modified and/or modified via RNAi) immunoprivileged selected renal cell populations that are to be administered to target organs or tissue in a subject in need. A bioactive selected renal cell population generally refers to a cell population potentially having therapeutic properties upon administration to a subject. For example, upon administration to a subject in need, a bioactive renal cell population can provide stabilization and/or improvement and/or repair and/or regeneration of kidney function in the subject. The therapeutic properties may include a repair or regenerative effect.

In certain cases the source of cells is the same as the intended target organ or tissue from the same or different sources. For example, BRC and/or SRC may be sourced from the kidney to be used in a formulation to be administered to the kidney. In certain cases, the cell populations are derived from a kidney biopsy. In certain cases, the cell populations are derived from whole kidney tissue. In certain cases, the cell populations are derived from in vitro cultures of mammalian kidney cells, established from kidney biopsies or whole kidney tissue. In certain cases, the BRC and/or SRC comprise heterogeneous mixtures or fractions of genetically modified (*e.g.,* genomically modified and/or modified via RNAi) immunoprivileged bioactive renal cells. The BRC and/or SRC may be derived from or are themselves renal cell fractions from healthy individuals. In addition, the present disclosure provides renal cell fractions obtained from an unhealthy individual that may lack certain cellular components when compared to the corresponding renal cell fractions of a healthy individual, yet still retain therapeutic properties. The present disclosure also provides therapeutically-active cell populations lacking cellular components compared to a healthy individual, which cell populations can be, in one embodiment, isolated and expanded from kidneys sourced from various mammals.

In certain cases, the SRC are obtained from isolation and expansion of renal cells from a different patient's renal cortical tissue via a kidney biopsy. In certain cases, renal cells are isolated from the kidney tissue by enzymatic digestion, genetically modified (*e.g*., genomically modified and/or modified via RNAi) to render them immunoprivileged or incapable of rejection, expanded using standard cell culture techniques, and selected by density gradient centrifugation from the expanded renal cells. In certain cases, SRC are composed primarily of renal epithelial cells which are known for their immunoprivileged and regenerative potential. Other parenchymal (vascular) and stromal cells may be sparsely present in the SRC population.

As described herein, the present invention is based, in part, on the surprising finding that certain subfractions of a heterogeneous population of renal cells, enriched for bioactive components and depleted of inactive or undesired components, provide superior therapeutic and regenerative outcomes than the starting population.

Renal cell isolation and expansion provides a mixture of renal cell types including renal epithelial cells and stromal cells. As noted above, SRC are obtained by density gradient separation of the expanded renal cells. The primary cell type in the density gradient separated SRC population is of tubular epithelial phenotype. The characteristics of SRC obtained from expanded renal cells is evaluated using multi-pronged approach. Cell morphology, growth kinetics and cell viability are monitored during the renal cell expansion process. SRC buoyant density and viability is characterized by density gradient and Trypan Blue exclusion. SRC phenotype is characterized by flow cytometry and SRC function is demonstrated by expression of VEGF and KIM-1.

Those of ordinary skill in the art will appreciate that other methods of isolation and culturing known in the art may be used for the cells described herein. Those of ordinary skill in the art will also appreciate that bioactive cell populations may be derived from sources other than those specifically listed above, including, without limitation, tissues and organs other than the kidney, body fluids and adipose.

### SRC Phenotype

In certain cases, cell phenotype is monitored by expression analysis of renal cell markers using flow cytometry. Phenotypic analysis of cells is based on the use of antigenic markers specific for the cell type being analyzed. Flow cytometric analysis provides a quantitative measure of cells in the sample population which express the antigenic marker being analyzed.

A variety of markers have been reported in the literature as being useful for phenotypic characterization of renal tubular epithelial cells: (i) cytokeratins; (ii) transport membrane proteins (aquaporins and cubilin); (iii) cell binding molecules (adherins and cluster of differentiation and lectins); and (iv) metabolic enzymes (glutathione and gamma-glutamyl transpeptidase (GGT)). (Table 1) Since the majority of cells found in cultures derived from whole kidney digests are epithelial and endothelial cells, the markers examined focus on the expression of proteins specific for these two groups.

**Table 1. Phenotypic Markers for SRC Characterization**

| Antigenic marker | Reactivity |
|---|---|
| CK8/18/19 | Epithelial cells, proximal and distal tubules |
| CK8 | Epithelial cells, proximal tubules |
| CK18 | Epithelial cells, proximal tubules |
| CK19 | Epithelial cells, collecting ducts, distal tubules |
| CK7 | Epithelial cells, collecting ducts, distal tubules |
| CXCR4 | Epithelial cells, distal and proximal tubules |
| E-cadherin | Epithelial cells, distal tubules |
| Cubilin | Epithelial cells, proximal tubules |
| Aquaporin1 | Epithelial cells, proximal tubules, descending thin limb |
| GGT1 | Fetal and adult kidney cells, proximal tubules |
| Aquaporin2 | Renal collecting duct cells, distal tubules |
| DBA | Renal collecting duct cells, distal tubules |
| CD31 | Endothelial cells of the kidney (rat) |
| CD146 | Endothelial cells of the kidney (canine, human) |
| MHC//minor HC Antigen | Reduced to render cells immunoprivileged |

Table 2 provides selected markers, range and mean percentage values of phenotypic in the SRC population and the rationale for their selection.

**Table 2. Marker Selected for Phenotypic Analysis of SRC**

| **Phenotypic Marker** | **Expression Range** | **Average** | **Rationale** | **Expression Level** |
|---|---|---|---|---|
| CK18 | 81.1 to 99.7% (n=87) | 96.7% | Epithelial marker | High |
| GGT1 | 4.5 to 81.2% (n=63) | 50.7% | Functional Tubular marker | Moderate |
| MHC//minor HC Antigen | Reduced to render cells immunoprivileged | >90% | Immunoprivilege | Low |

### Cell Function

SRC actively secrete proteins which can be detected through analysis of conditioned medium. Cell function is assessed by the ability of cells to metabolize PrestoBlue and to secrete VEGF (Vascular Endothelial Growth Factor) and KIM-1 (Kidney Injury Molecule-1).

Table 3 presents VEGF and KIM-1 quantities present in conditioned medium from renal cells and SRC cultures. Renal cells were cultured to near confluence. Conditioned medium from overnight exposure to the renal cell cultures was tested for VEGF and KIM-1.

**Table 3. Production of VEGF and KIM-1 by Human Renal Cells and SRC**

| Conditioned Medium | VEGF | | KIM-1 | |
|---|---|---|---|---|
| | ng/mL | ng/million cells | ng/mL | ng/million cells |
| Renal Cell Culture (n=15) | 0.50 to 2.42 | 2.98 to 14.6 | 0.20 to 3.41 | 1.14 to 15.2 |
| SRC (n=14) | 0.80 to 3.85 | 4.83 to 23.07 | 0.32 to 2.10 | 1.93 to 12.59 |

### SRC Enzymatic Activity

Cell function of SRC, pre-formulation, can also be evaluated by measuring the activity of two specific enzymes; GGT (γ-glutamyl transpeptidase) and LAP (leucine aminopeptidase), found in kidney proximal tubules.

Also described are compositions containing a variety of other active agents. which agents include, without limitation, cellular aggregates, acellular biomaterials, secreted products from bioactive cells, large and small molecule therapeutics, as well as combinations thereof. For example, one type of bioactive cells may be combined with biomaterial-based microcarriers with or without therapeutic molecules or another type of bioactive cells, unattached cells may be combined with acellular particles.

### Exemplary Genetic Modification Methods

In certain embodiments, genetically modifying a cell, such as a BRC (*e.g.,* an SRC) comprises introducing (*e.g*., via expression or by delivery across a cell membrane) a gene editing protein and/or nucleic acid into the BRC.

In certain embodiments, the gene editing protein is a zinc finger nuclease (ZFN), a transcription activator-like effector nuclease (TALEN), a megaTAL, or a Cas protein. In certain embodiments, the protein is a a single-chain rarecleaving nuclease architecture (a "megaTAL"). In certain embodiments, a gene editing protein such as a ZFN, megaTAL, or a Cas protein (such as Cas9) may be delivered as a protein or alternatively, a polynucleotide (such as an mRNA or a vector) encoding the protein is delivered to the cells.

In certain embodiments, a Cas9 protein is delivered as a protein. In certain embodiments, a polynucleotide (such as an mRNA or a vector) encoding the Cas9 protein is delivered to the cells. In certain embodiments, a gene editing polynucleotide such as a gRNA is delibered into a cell. In the context of a CRISPR/Cas9 system, a single-guide RNA (a "gRNA" or "guide RNA") contains both a targeting sequence (crRNA sequence) and a Cas9 nuclease-recruiting sequence (tracrRNA). In certain embodiments, a crRNA and/or a tracrRNA is delivered into a cell. In certain embodiments, the gene editing polynucleotide is expressed in the cell from, *e.g.,* an mRNA or a vector. In certain embodiments, a gene editing complex comprising a Cas9 protein and a gRNA or a crRNA and a tracrRNA, *i.e.,* a CRISPR ribonucleoprotein complex (RNP), is delivered into or expressed within a cell.

In certain embodiments, a Cas protein (*e.g*., expressed or delivered as part fo an RNP) is used to genetically modify a cell. In certain embodiments, the Cas protein is a Cas9 protein or a mutant thereof. Non-limiting examples of Cas proteins (including Cas9 and non-limiting examples of Cas9 mutants) are described herein.

In certain embodiments, first and second gRNA or first and second crRNA molecules together comprise nucleic acid sequences complementary to target sequences flanking a gene or a portion thereof (*e.g.,* a promoter and/or one or more an exon and/or introns thereof), wherein the gene or portion thereof is about 1, 2, 3, 4, 5, 6, 10, 20, 30, 40, 50, 60, 70, 80, 90, 100, 1-100, kilobases in length or is at least about 1, 2, 3, 4, 5, 6, 10, 20, 30, 40, 50, 60, 70, 80, 90, 100, 1-100, kilobases in length. In certain embodiments, pairs of RNPs comprising the first and second gRNA or first and second crRNA molecules are expressed or delivered into a cell. In certain embodiments, one component of an RNP (such as a Cas protein or a gRNA) is expressed in a cell, and the other component (such as a gRNA or a Cas protein) is delivered across the cell membrane of the cell.

In certain embodiments, the target sequence of a gRNA or crRNA is about 12 to about 25, or about 12, 13, 14, 15, 16, 17, 18, 19, 20, 21, 22, 23, 24, 25, 17-23, or 18-22, nucleotides long. In certain embodiments, the target sequence is 20 nucleotides long or about 20 nucleotides long.

In certain embodiments, epigenomic modification at the targeted genomic locus may modify accessibility of the targeted DNA site to the cas protein. In certain embodiments, a gRNA targets a gene near the transcription start site thereof and/or in a region of open chromatin characterized by extensive methylation of the H3K4 (or other) residue of the nucleosome. In certain embodiments, the target sequence is within about 5000, 2500, 2000, 1500, 1250, 1000, 900, 800, 700, 600, 500, 400, 300, 200, 100, 50, or 25 bases of the transcriptional start site (*e.g.,* either at the 5' end or the 3' end) of a the targeted gene. In certain embodiments, the target sequence overlaps with or is complementary to at least a portion of the gene's transcription start site. In certain embodiments, publicly available software (CHOPCHOP, WU-CRISPR) may be used to design CRISPR targeting constructs (gRNAs binding defined DNA sequences). Non-limiting examples of potential gRNA target sites for the B2M and HLA-A genes are shown in Tables 4 and 5.

**Table 4. CHOPCHOP output showing potential genomic target sites for guide RNAs specifically desgined to knockdown the b2-microglobulin gene NM_004048**

| **Rankin g** | **Target sequence** | **Genomic location** | **Exo n** | **Stran d** | **GC (% )** | **Self-complementarity** | **Off-target s** | **Effici -ency** | | | |
|---|---|---|---|---|---|---|---|---|---|---|---|
| 0 | 1 | 2 | 3 | | | | | | | | |
| 1 | CGCGAGCACAGCTAAGGCCACG G (SEQ ID NO: 17) | chr15:4471155 7 | 1 | - | 65 | 2 | 1 | 0 | 0 | 0 | 0.68 |
| 2 | TCACGTCATCCAGCAGAGAATGG (SEQ ID NO: 18) | chr15:4471544 6 | 2 | + | 50 | 0 | 1 | 0 | 0 | 0 | 0.55 |
| 3 | ACAAAGTCACATGGTTCACACGG (SEQ ID NO: 19) | chr15:4471565 5 | 2 | - | 40 | 1 | 1 | 0 | 0 | 0 | 0.52 |
| 4 | GAGTAGCGCGAGCACAGCTAAG G (SEQ ID NO: 20) | chr15:4471156 3 | 1 | - | 60 | 1 | 1 | 0 | 0 | 1 | 0.62 |
| 5 | AGTCACATGGTTCACACGGCAGG (SED ID NO: 21) | chr15:4471565 1 | 2 | - | 55 | 0 | 1 | 0 | 0 | 1 | 0.57 |
| 6 | AAGTCAACTTCAATGTCGGATGG (SEQ ID NO: 22) | chr15:4471550 9 | 2 | - | 40 | 0 | 1 | 0 | 0 | 1 | 0.56 |
| 7 | ACTCACGCTGGATAGCCTCCAGG (SEQ ID NO: 23) | chr15:4471159 7 | 1 | - | 60 | 2 | 1 | 0 | 0 | 1 | 0.53 |
| 8 | TCCTGAATTGCTATGTGTCTGGG (SEQ ID NO: 24) | chr15:4471548 0 | 2 | + | 40 | 0 | 1 | 0 | 0 | 1 | 0.44 |
| 9 | GGCCACGGAGCGAGACATCTCG G (SEQ ID NO: 25) | chr15:4471154 2 | 1 | - | 65 | 2 | 1 | 0 | 0 | 1 | 0.45 |
| 10 | CGTGAGTAAACCTGAATCTTTGG (SEQ ID NO: 26) | chr15:4471542 8 | 2 | - | 40 | 0 | 1 | 0 | 0 | 1 | 0.4 |
| 11 | TTGGAGTACCTGAGGAATATCGG (SEQ ID NO: 27) | chr15:4471540 9 | 2 | - | 40 | 0 | 1 | 0 | 0 | 1 | 0.38 |
| 12 | CAGTAAGTCAACTTCAATGTCGG (SEQ ID NO: 28) | chr15:4471551 3 | 2 | - | 35 | 1 | 1 | 0 | 0 | 0 | 0.58 |
| 13 | GCATACTCATCTTTTTCAGTGGG (SEQ ID NO: 29) | chr15:4471562 9 | 2 | - | 35 | 0 | 1 | 0 | 0 | 0 | 0.52 |
| 14 | AGGGTAGGAGAGACTCACGCTG G (SEQ ID NO: 30) | chr15:4471160 9 | 1 | - | 60 | 0 | 1 | 0 | 0 | 2 | 0.6 |
| 15 | GGCCGAGATGTCTCGCTCCGTGG (SEQ ID NO: 31) | chr15:4471154 0 | 1 | + | 70 | 2 | 1 | 0 | 0 | 2 | 0.5 |
| 16 | GAGACATGTAAGCAGCATCATG G (SEQ ID NO: 32) | chr15:4471633 2 | 3 | + | 45 | 0 | 1 | 0 | 0 | 2 | 0.42 |
| 17 | GTCTTTTCCCGATATTCCTCAGG (SEQ ID NO: 33) | chr15:4471540 1 | 2 | + | 45 | 0 | 1 | 0 | 0 | 2 | 0.4 |
| 18 | CATACTCATCTTTTTCAGTGGGG (SEQ ID NO: 34) | chr15:4471562 8 | 2 | - | 35 | 0 | 1 | 0 | 0 | 1 | 0.49 |
| 19 | ACAGCCCAAGATAGTTAAGTGG G (SEQ ID NO: 35) | chr15:4471567 9 | 2 | + | 40 | 1 | 1 | 0 | 1 | 1 | 0.48 |
| 20 | ACATGTAAGCAGCATCATGGAG G (SEQ ID NO: 36) | chr15:4471633 5 | 3 | + | 45 | 1 | 1 | 0 | 0 | 3 | 0.63 |
| 21 | GGCATACTCATCTTTTTCAGTGG (SEQ ID NO: 37) | chr15:4471563 0 | 2 | - | 40 | 0 | 1 | 0 | 0 | 3 | 0.47 |
| 22 | TGGAGTACCTGAGGAATATCGG G (SEQ ID NO: 38) | chr15:4471540 8 | 2 | - | 45 | 0 | 1 | 0 | 0 | 3 | 0.38 |
| 23 | TTCCTGAATTGCTATGTGTCTGG (SEQ ID NO: 39) | chr15:4471547 9 | 2 | + | 40 | 0 | 1 | 0 | 0 | 3 | 0.26 |
| 24 | TCGATCTATGAAAAAGACAGTGG (SEQ ID NO: 40) | chr15:4471631 1 | 3 | - | 35 | 0 | 1 | 0 | 0 | 2 | 0.67 |
| 25 | CACAGCCCAAGATAGTTAAGTGG (SEQ ID NO: 41) | chr15:4471567 8 | 2 | + | 45 | 1 | 1 | 0 | 0 | 4 | 0.51 |
| 26 | TTCAGACTTGTCTTTCAGCAAGG (SEQ ID NO: 42) | chr15:4471556 8 | 2 | + | 40 | 2 | 1 | 0 | 0 | 4 | 0.47 |
| 27 | ATACTCATCTTTTTCAGTGGGGG (SEQ ID NO: 43) | chr15:4471562 7 | 2 | - | 35 | 0 | 1 | 0 | 0 | 3 | 0.64 |
| 28 | TTACCCCACTTAACTATCTTGGG (SEQ ID NO: 44) | chr15:4471568 3 | 2 | - | 35 | 1 | 1 | 0 | 0 | 3 | 0.37 |
| 29 | ACTTGTCTTTCAGCAAGGACTGG (SEQ ID NO: 45) | chr15:4471557 3 | 2 | + | 45 | 1 | 1 | 0 | 1 | 3 | 0.55 |
| 30 | CAGCCCAAGATAGTTAAGTGGG G (SEQ ID NO: 46) | chr15:4471568 0 | 2 | + | 45 | 1 | 1 | 0 | 0 | 5 | 0.58 |
| 31 | ACCCAGACACATAGCAATTCAGG (SEQ ID NO: 47) | chr15:4471548 1 | 2 | - | 45 | 0 | 1 | 0 | 0 | 5 | 0.33 |
| 32 | TGGGCTGTGACAAAGTCACATG G (SEQ ID NO: 48) | chr15:4471566 4 | 2 | - | 50 | 4 | 1 | 0 | 1 | 4 | 0.56 |
| 33 | GCTACTCTCTCTTTCTGGCCTGG (SEQ ID NO: 49) | chr15:4471157 9 | 1 | + | 55 | 0 | 1 | 0 | 0 | 6 | 0.47 |
| 34 | CTCGCGCTACTCTCTCTTTCTGG (SEQ ID NO: 50) | chr15:4471157 4 | 1 | + | 55 | 0 | 1 | 0 | 0 | 6 | 0.27 |
| 35 | CTCAGGTACTCCAAAGATTCAGG (SEQ ID NO: 51) | chr15:4471541 8 | 2 | + | 45 | 0 | 1 | 0 | 1 | 8 | 0.41 |
| 36 | CTGAATCTTTGGAGTACCTGAGG (SEQ ID NO: 52) | chr15:4471541 7 | 2 | - | 45 | 0 | 1 | 0 | 1 | 1 1 | 0.56 |
| 37 | ACTCTCTCTTTCTGGCCTGGAGG (SEQ ID NO: 53) | chr15:4471158 2 | 1 | + | 55 | 1 | 1 | 0 | 0 | 1 4 | 0.51 |
| 38 | CTTACCCCACTTAACTATCTTGG (SEQ ID NO: 54) | chr15:4471568 4 | 2 | - | 40 | 0 | 1 | 0 | 1 | 1 3 | 0.34 |
| 39 | TTTGACTTTCCATTCTCTGCTGG (SEQ ID NO: 55) | chr15:4471545 5 | 2 | - | 40 | 0 | 1 | 0 | 0 | 1 6 | 0.5 |
| 40 | GAAGTTGACTTACTGAAGAATGG (SEQ ID NO: 56) | chr15:4471552 1 | 2 | + | 35 | 1 | 1 | 0 | 0 | 1 7 | 0.53 |
| 41 | TGGAGAGAGAATTGAAAAAGTG G (SEQ ID NO: 57) | chr15:4471554 1 | 2 | + | 35 | 0 | 1 | 0 | 0 | 1 9 | 0.65 |

**Table 5. CHOPCHOP output showing potential genomic target sites for guide RNAs spewcifically desgined to knockdown the HLA-A gene NM_002116**

| Ranking | Target sequence | Genomic location | Exon | Strand | GC (%) | Self-complementarity | Off-targets | Efficiency | | | |
|---|---|---|---|---|---|---|---|---|---|---|---|
| 0 | 1 | 2 | 3 | | | | | | | | |
| 1 | CAGTTGAGAGCCTACCTGGATGG (SEQ ID NO: 58) | chr6:29943459 | 3 | + | 55 | 2 | 0 | 0 | 0 | 2 | 0.51 |
| 2 | TGCCGTCGTAGGCGTCCTGCCGG (SEQ ID NO: 59) | chr6:29943335 | 3 | - | 70 | 0 | 3 | 0 | 0 | 0 | 0.46 |
| 3 | GGCGCTTCCTCCGCGGGTACCGG (SEQ ID NO: 60) | chr6:29943316 | 3 | + | 75 | 0 | 3 | 0 | 0 | 0 | 0.48 |
| 4 | AGACTGACCGAGTGGACCTGGGG (SEQ ID NO: 61) | chr6:29942970 | 2 | + | 60 | 3 | 0 | 2 | 4 | 1 | 0.58 |
| 5 | CAGACTGACCGAGTGGACCTGGG (SEQ ID NO: 62) | chr6:29942969 | 2 | + | 60 | 3 | 0 | 2 | 4 | 1 | 0.57 |
| 6 | ACAGACTGACCGAGTGGACCTGG (SEQ ID NO: 63) | chr6:29942968 | 2 | + | 60 | 2 | 0 | 2 | 4 | 2 | 0.42 |
| 7 | CTTCCTCCGCGGGTACCGGCAGG (SEQ ID NO: 64) | chr6:29943320 | 3 | + | 75 | 0 | 3 | 1 | 1 | 0 | 0.6 |
| 8 | TACCGGCAGGACGCCTACGACGG (SEQ ID NO: 65) | chr6:29943333 | 3 | + | 65 | 2 | 3 | 1 | 1 | 2 | 0.68 |
| 9 | AGGCGTCCTGCCGGTACCCGCGG (SEQ ID NO: 66) | chr6:29943326 | 3 | - | 75 | 0 | 3 | 1 | 1 | 2 | 0.64 |
| 10 | CGTCCTGCCGGTACCCGCGGAGG (SEQ ID NO: 67) | chr6:29943323 | 3 | - | 80 | 0 | 3 | 2 | 0 | 2 | 0.73 |
| 11 | CCAGTCACAGACTGACCGAGTGG (SEQ ID NO: 68) | chr6:29942962 | 2 | + | 60 | 3 | 0 | 0 | 2 | 12 | 0.55 |
| 12 | GCAGCCATACATTATCTGGATGG (SEQ ID NO: 69) | chr6:29943277 | 3 | - | 45 | 1 | 2 | 2 | 1 | 10 | 0.47 |
| 13 | CCAGGAGACACGGAATGTGAAGG (SEQ ID NO: 70) | chr6:29942938 | 2 | + | 55 | 2 | 1 | 2 | 1 | 13 | 0.72 |
| 14 | CTCTCAACTGCTCCGCCTCATGG (SEQ ID NO: 71) | chr6:29943446 | 3 | - | 60 | 1 | 0 | 3 | 6 | 6 | 0.4 |
| 15 | TTGTAAAGGTGAGAGCTTGGAGG (SEQ ID NO: 72) | chr6:29945274 | 7 | + | 45 | 0 | 2 | 5 | 0 | 7 | 0.52 |
| 16 | TCGCTGCCGTGATGTGGAGGAGG (SEQ ID NO: 73) | chr6:29944584 | 5 | + | 65 | 0 | 2 | 3 | 6 | 4 | 0.66 |
| 17 | GGCGCCATGACGGCCATCCTCGG (SEQ ID NO: 74) | chr6:29942548 | 1 | - | 70 | 1 | 6 | 0 | 0 | 9 | 0.52 |
| 18 | TGTGTCTTGGGGGGGTCTGACGG (SEQ ID NO: 75) | chr6:29944115 | 4 | - | 60 | 0 | 6 | 0 | 3 | 5 | 0.47 |
| 19 | GCGCCCGCGGCTCCATCCTCTGG (SEQ ID NO: 76) | chr6:29942881 | 2 | - | 80 | 0 | 7 | 3 | 0 | 3 | 0.53 |
| 20 | CTCAGGTGGAGAAGGGGTGAAGG (SEQ ID NO: 77) | chr6:29944612 | 5 | + | 60 | 0 | 2 | 3 | 2 | 15 | 0.55 |
| 21 | ATTTCTTCACATCCGTGTCCCGG (SEQ ID NO: 78) | chr6:29942775 | 2 | + | 45 | 0 | 5 | 0 | 3 | 12 | 0.5 |
| 22 | GTGGACCTGGGGACCCTGCGCGG (SEQ ID NO: 79) | chr6:29942981 | 2 | + | 75 | 2 | 2 | 0 | 4 | 17 | 0.57 |
| 23 | TTCACATCCGTGTCCCGGCCCGG (SEQ ID NO: 80) | chr6:29942780 | 2 | + | 65 | 0 | 5 | 0 | 4 | 11 | 0.45 |
| 24 | AGCTTGTAAAGGTGAGAGCTTGG (SEQ ID NO: 81) | chr6:29945271 | 7 | + | 45 | 0 | 2 | 5 | 1 | 14 | 0.42 |
| 25 | ACGGCCATCCTCGGCGTCTGGGG (SEQ ID NO: 82) | chr6:29942539 | 1 | - | 70 | 0 | 6 | 0 | 0 | 16 | 0.41 |
| 26 | GACGGCCATCCTCGGCGTCTGGG (SEQ ID NO: 83) | chr6:29942540 | 1 | - | 70 | 0 | 6 | 0 | 0 | 16 | 0.39 |
| 27 | TCAGGTGGAGAAGGGGTGAAGGG (SEQ ID NO: 84) | chr6:29944613 | 5 | + | 55 | 0 | 2 | 2 | 1 | 21 | 0.45 |
| 28 | GACGCCGAGGATGGCCGTCATGG (SEQ ID NO: 85) | chr6:29942544 | 1 | + | 70 | 0 | 6 | 0 | 0 | 17 | 0.46 |
| 29 | TGACGGCCATCCTCGGCGTCTGG (SEQ ID NO: 86) | chr6:29942541 | 1 | - | 70 | 3 | 6 | 0 | 0 | 17 | 0.4 |
| 30 | TCTCAACTGCTCCGCCTCATGGG (SEQ ID NO: 87) | chr6:29943445 | 3 | - | 55 | 1 | 0 | 3 | 12 | 12 | 0.37 |
| 31 | GTATGGCTGCGACGTGGGGTCGG (SEQ ID NO: 88) | chr6:29943290 | 3 | + | 65 | 0 | 3 | 4 | 14 | 0 | 0.54 |
| 32 | CACTCGGTCAGTCTGTGACTGGG (SEQ ID NO: 89) | chr6:29942961 | 2 | - | 55 | 2 | 0 | 9 | 8 | 7 | 0.56 |
| 33 | CGCACGGGTACCAGGGGCCACGG (SEQ ID NO: 90) | chr6:29943537 | 3 | + | 75 | 1 | 6 | 7 | 5 | 0 | 0.58 |
| 34 | GGATGTGAAGAAATACCTCATGG (SEQ ID NO: 91) | chr6:29942766 | 2 | - | 40 | 0 | 5 | 0 | 3 | 21 | 0.57 |
| 35 | CACACCATCCAGATAATGTATGG (SEQ ID NO: 92) | chr6:29943273 | 3 | + | 40 | 0 | 2 | 2 | 7 | 19 | 0.42 |
| 36 | CGTCGCAGCCATACATTATCTGG (SEQ ID NO: 93) | chr6:29943281 | 3 | - | 50 | 1 | 2 | 9 | 4 | 10 | 0.35 |
| 37 | CGGCTCCATCCTCTGGCTCGCGG (SEQ ID NO: 94) | chr6:29942874 | 2 | - | 70 | 0 | 8 | 2 | 0 | 16 | 0.61 |
| 38 | GTGTCTTGGGGGGGTCTGACGGG (SEQ ID NO: 95) | chr6:29944114 | 4 | - | 65 | 0 | 6 | 0 | 9 | 13 | 0.32 |
| 39 | ACAGCGACGCCGCGAGCCAGAGG (SEQ ID NO: 96) | chr6:29942865 | 2 | + | 75 | 1 | 8 | 0 | 2 | 18 | 0.68 |
| 40 | CGACGCCGCGAGCCAGAGGATGG (SEQ ID NO: 97) | chr6:29942869 | 2 | + | 75 | 2 | 8 | 2 | 8 | 8 | 0.6 |
| 41 | GATAATGTATGGCTGCGACGTGG (SEQ ID NO: 98) | chr6:29943284 | 3 | + | 50 | 0 | 2 | 8 | 12 | 10 | 0.62 |
| 42 | ATAATGTATGGCTGCGACGTGGG (SEQ ID NO: 99) | chr6:29943285 | 3 | + | 45 | 0 | 2 | 8 | 13 | 9 | 0.52 |
| 43 | CACCAAGCGCAAGTGGGAGGCGG (SEQ ID NO: 100) | chr6:29943422 | 3 | + | 65 | 0 | 50 | 50 | 50 | 50 | 0.74 |
| 44 | GCTGCAAGTAAGTATGAAGGAGG (SEQ ID NO: 101) | chr6:29945085 | 6 | + | 45 | 0 | 50 | 50 | 50 | 50 | 0.73 |
| 45 | CGTGGAGACCAGGCCTGCAGGGG (SEQ ID NO: 102) | chr6:29944264 | 4 | + | 70 | 1 | 50 | 50 | 50 | 50 | 0.72 |
| 46 | GGTGGAGAAGGGGTGAAGGGTGG (SEQ ID NO: 103) | chr6:29944616 | 5 | + | 65 | 0 | 50 | 50 | 50 | 50 | 0.71 |
| 47 | CTGCAGCGCACGGGTACCAGGGG (SEQ ID NO: 104) | chr6:29943531 | 3 | + | 70 | 1 | 50 | 50 | 50 | 50 | 0.72 |
| 48 | CGGCTACTACAACCAGAGCGAGG (SEQ ID NO: 105) | chr6:29943001 | 2 | + | 60 | 0 | 50 | 50 | 50 | 50 | 0.71 |
| 49 | AGATCACACTGACCTGGCAGCGG (SEQ ID NO: 106) | chr6:29944209 | 4 | + | 55 | 0 | 50 | 50 | 50 | 50 | 0.69 |
| 50 | TGTGGTGGTGCCTTCTGGAGAGG (SEQ ID NO: 107) | chr6:29944312 | 4 | + | 60 | 1 | 50 | 50 | 50 | 50 | 0.68 |
| 51 | AGACAGCTCTGGGAAAAGAGGGG (SEQ ID NO: 108) | chr6:29944485 | 5 | - | 50 | 2 | 50 | 50 | 50 | 50 | 0.69 |
| 52 | GAAGAGCTCAGGTGGAGAAGGGG (SEQ ID NO: 109) | chr6:29944606 | 5 | + | 55 | 0 | 50 | 50 | 50 | 50 | 0.67 |
| 53 | TGAAGAAATACCTCATGGAGTGG (SEQ ID NO: 110) | chr6:29942761 | 2 | - | 40 | 0 | 50 | 50 | 50 | 50 | 0.67 |
| 54 | TAGCCCACGGCGATGAAGCGGGG (SEQ ID NO: 111) | chr6:29942813 | 2 | - | 65 | 1 | 50 | 50 | 50 | 50 | 0.68 |
| 55 | CGTGTCGTCCACGTAGCCCACGG (SEQ ID NO: 112) | chr6:29942826 | 2 | - | 65 | 2 | 50 | 50 | 50 | 50 | 0.68 |
| 56 | GATCACCAAGCGCAAGTGGGAGG (SEQ ID NO: 113) | chr6:29943419 | 3 | + | 60 | 0 | 50 | 50 | 50 | 50 | 0.66 |
| 57 | GGACCTGCGCTCTTGGACCGCGG (SEQ ID NO: 114) | chr6:29943380 | 3 | + | 70 | 2 | 50 | 50 | 50 | 50 | 0.68 |
| 58 | GGCGCCGTGGATAGAGCAGGAGG (SEQ ID NO: 115) | chr6:29942899 | 2 | + | 70 | 0 | 50 | 50 | 50 | 50 | 0.66 |
| 59 | CGCCGTGGATAGAGCAGGAGGGG (SEQ ID NO: 116) | chr6:29942901 | 2 | + | 65 | 0 | 50 | 50 | 50 | 50 | 0.66 |
| 60 | TGGATGGCACGTGCGTGGAGTGG (SEQ ID NO: 117) | chr6:29943475 | 3 | + | 65 | 1 | 50 | 50 | 50 | 50 | 0.67 |
| 61 | CTTCTCACAGATAGAAAAGGAGG (SEQ ID NO: 118) | chr6:29945049 | 6 | + | 40 | 0 | 50 | 50 | 50 | 50 | 0.66 |
| 62 | TGGTCGCTGCCGTGATGTGGAGG (SEQ ID NO: 119) | chr6:29944581 | 5 | + | 65 | 0 | 50 | 50 | 50 | 50 | 0.66 |
| 63 | CAGATTCTCCCCAGACGCCGAGG (SEQ ID NO: 120) | chr6:29942531 | 1 | + | 65 | 1 | 50 | 50 | 50 | 50 | 0.66 |
| 64 | GGATGGGGAGGACCAGACCCAGG (SEQ ID NO: 121) | chr6:29944231 | 4 | + | 70 | 1 | 50 | 50 | 50 | 50 | 0.66 |
| 65 | TGGGGTAAGGAGGGAGATGGGGG (SEQ ID NO: 122) | chr6:29944394 | 4 | + | 60 | 0 | 50 | 50 | 50 | 50 | 0.65 |
| 66 | CTCATGGAGTGGGAGCCTGGGGG (SEQ ID NO: 123) | chr6:29942750 | 2 | - | 65 | 2 | 50 | 50 | 50 | 50 | 0.67 |
| 67 | CAGATACCTGGAGAACGGGAAGG (SEQ ID NO: 124) | chr6:29943503 | 3 | + | 55 | 1 | 50 | 50 | 50 | 50 | 0.66 |
| 68 | GAGCCAGAGGATGGAGCCGCGGG (SEQ ID NO: 125) | chr6:29942878 | 2 | + | 70 | 1 | 50 | 50 | 50 | 50 | 0.65 |
| 69 | CCTGGCCCTGACCCAGACCTGGG (SEQ ID NO: 126) | chr6:29942601 | 1 | + | 70 | 0 | 50 | 50 | 50 | 50 | 0.64 |
| 70 | CATGGTCAGAGATGGGGTGGTGG (SEQ ID NO: 127) | chr6:29944144 | 4 | - | 60 | 0 | 50 | 50 | 50 | 50 | 0.64 |
| 71 | TGGAGGAGGAAGAGCTCAGGTGG (SEQ ID NO: 128) | chr6:29944598 | 5 | + | 60 | 0 | 50 | 50 | 50 | 50 | 0.64 |
| 72 | TTGGACCGCGGCGGACATGGCGG (SEQ ID NO: 129) | chr6:29943392 | 3 | + | 70 | 4 | 50 | 50 | 50 | 50 | 0.67 |
| 73 | TGGAACCTTCCAGAAGTGGGCGG (SEQ ID NO: 130) | chr6:29944288 | 4 | + | 55 | 2 | 50 | 50 | 50 | 50 | 0.65 |
| 74 | CCTCCTCCTGCTACTCTCGGGGG (SEQ ID NO: 131) | chr6:29942577 | 1 | + | 65 | 0 | 50 | 50 | 50 | 50 | 0.63 |
| 75 | AGCTCTGGGAAAAGAGGGGAAGG (SEQ ID NO: 132) | chr6:29944481 | 5 | - | 55 | 1 | 50 | 50 | 50 | 50 | 0.64 |
| 76 | GGGATGGAACCTTCCAGAAGTGG (SEQ ID NO: 133) | chr6:29944284 | 4 | + | 55 | 3 | 50 | 50 | 50 | 50 | 0.65 |
| 77 | AGATGGGGTAAGGAGGGAGATGG (SEQ ID NO: 134) | chr6:29944391 | 4 | + | 55 | 0 | 50 | 50 | 50 | 50 | 0.62 |
| 78 | GGTGGCCTCATGGTCAGAGATGG (SEQ ID NO: 135) | chr6:29944152 | 4 | - | 60 | 1 | 50 | 50 | 50 | 50 | 0.62 |
| 79 | CTGACCATGAGGCCACCCTGAGG (SEQ ID NO: 136) | chr6:29944158 | 4 | + | 65 | 1 | 50 | 50 | 50 | 50 | 0.62 |
| 80 | GGGTCATATGTGTCTTGGGGGGG (SEQ ID NO: 137) | chr6:29944123 | 4 | - | 55 | 0 | 50 | 50 | 50 | 50 | 0.61 |
| 81 | CTCCGCAGGGTAGAAGCCCAGGG (SEQ ID NO: 138) | chr6:29944188 | 4 | - | 65 | 1 | 50 | 50 | 50 | 50 | 0.62 |
| 82 | AAGCCCCTCACCCTGAGATGGGG (SEQ ID NO: 139) | chr6:29944376 | 4 | + | 60 | 1 | 50 | 50 | 50 | 50 | 0.62 |
| 83 | GTGGAGAAGGGGTGAAGGGTGGG (SEQ ID NO: 140) | chr6:29944617 | 5 | + | 60 | 0 | 50 | 50 | 50 | 50 | 0.61 |
| 84 | GTGGATAGAGCAGGAGGGGCCGG (SEQ ID NO: 141) | chr6:29942905 | 2 | + | 65 | 0 | 50 | 50 | 50 | 50 | 0.61 |
| 85 | TGCTCTATCCACGGCGCCCGCGG (SEQ ID NO: 142) | chr6:29942894 | 2 | - | 70 | 1 | 50 | 50 | 50 | 50 | 0.62 |
| 86 | AAAAGGAGGGAGTTACACTCAGG (SEQ ID NO: 143) | chr6:29945063 | 6 | + | 45 | 1 | 50 | 50 | 50 | 50 | 0.61 |
| 87 | TGAGGGACACATCAGAGCCCTGG (SEQ ID NO: 144) | chr6:29945247 | 7 | - | 60 | 2 | 50 | 50 | 50 | 50 | 0.62 |
| 88 | GAAGGAGACGCTGCAGCGCACGG (SEQ ID NO: 145) | chr6:29943521 | 3 | + | 65 | 1 | 50 | 50 | 50 | 50 | 0.61 |
| 89 | TCTCCGCAGGGTAGAAGCCCAGG (SEQ ID NO: 146) | chr6:29944189 | 4 | - | 65 | 1 | 50 | 50 | 50 | 50 | 0.61 |
| 90 | GAAGACAGCTCTGGGAAAAGAGG (SEQ ID NO: 147) | chr6:29944487 | 5 | - | 50 | 0 | 50 | 50 | 50 | 50 | 0.59 |
| 91 | CCAGAAGTGGGCGGCTGTGGTGG (SEQ ID NO: 148) | chr6:29944297 | 4 | + | 70 | 0 | 50 | 50 | 50 | 50 | 0.59 |
| 92 | TCTGGTTGTAGTAGCCGCGCAGG (SEQ ID NO: 149) | chr6:29942995 | 2 | - | 60 | 2 | 50 | 50 | 50 | 50 | 0.61 |
| 93 | AATGATGCCCACGATGGGGATGG (SEQ ID NO: 150) | chr6:29944518 | 5 | - | 55 | 1 | 50 | 50 | 50 | 50 | 0.6 |
| 94 | TGGAAGGTTCCATCCCCTGCAGG (SEQ ID NO: 151) | chr6:29944277 | 4 | - | 60 | 2 | 50 | 50 | 50 | 50 | 0.61 |
| 95 | ACTGACCTGGCAGCGGGATGGGG (SEQ ID NO: 152) | chr6:29944216 | 4 | + | 65 | 0 | 50 | 50 | 50 | 50 | 0.59 |
| 96 | AGGAAGAGCTCAGGTGGAGAAGG (SEQ ID NO: 153) | chr6:29944604 | 5 | + | 55 | 0 | 50 | 50 | 50 | 50 | 0.59 |
| 97 | ATGTGGAGGAGGAAGAGCTCAGG (SEQ ID NO: 154) | chr6:29944595 | 5 | + | 55 | 0 | 50 | 50 | 50 | 50 | 0.59 |
| 98 | GAGCCCCGCTTCATCGCCGTGGG (SEQ ID NO: 155) | chr6:29942810 | 2 | + | 70 | 0 | 50 | 50 | 50 | 50 | 0.58 |
| 99 | CAGGTCAGTGTGATCTCCGCAGG (SEQ ID NO: 156) | chr6:29944202 | 4 | - | 60 | 0 | 50 | 50 | 50 | 50 | 0.58 |
| 100 | GCGCCGTGGATAGAGCAGGAGGG (SEQ ID NO: 157) | chr6:29942900 | 2 | + | 65 | 0 | 50 | 50 | 50 | 50 | 0.58 |
| 101 | AGACCTGGGCGGGTGAGTGCGGG (SEQ ID NO: 158) | chr6:29942615 | 1 | + | 70 | 1 | 50 | 50 | 50 | 50 | 0.59 |
| 102 | GGAGGACCAGACCCAGGACACGG (SEQ ID NO: 159) | chr6:29944237 | 4 | + | 65 | 2 | 50 | 50 | 50 | 50 | 0.6 |
| 103 | GAGACCAGGCCTGCAGGGGATGG (SEQ ID NO: 160) | chr6:29944268 | 4 | + | 70 | 2 | 50 | 50 | 50 | 50 | 0.6 |
| 104 | TATCTCTGCTCCTCTCCAGAAGG (SEQ ID NO: 161) | chr6:29944322 | 4 | - | 50 | 1 | 50 | 50 | 50 | 50 | 0.59 |
| 105 | CACCCTGAGATGGGGTAAGGAGG (SEQ ID NO: 162) | chr6:29944384 | 4 | + | 60 | 0 | 50 | 50 | 50 | 50 | 0.58 |
| 106 | CGTAGCCCACGGCGATGAAGCGG (SEQ ID NO: 163) | chr6:29942815 | 2 | - | 65 | 3 | 50 | 50 | 50 | 50 | 0.61 |
| 107 | TGGGTCATATGTGTCTTGGGGGG (SEQ ID NO: 164) | chr6:29944124 | 4 | - | 50 | 0 | 50 | 50 | 50 | 50 | 0.57 |
| 108 | CCCAGGCAGTGACAGTGCCCAGG (SEQ ID NO: 165) | chr6:29945229 | 7 | + | 70 | 2 | 50 | 50 | 50 | 50 | 0.59 |
| 109 | GGATGGAACCTTCCAGAAGTGGG (SEQ ID NO: 166) | chr6:29944285 | 4 | + | 50 | 3 | 50 | 50 | 50 | 50 | 0.6 |
| 110 | CCAAGCCCCTCACCCTGAGATGG (SEQ ID NO: 167) | chr6:29944374 | 4 | + | 65 | 2 | 50 | 50 | 50 | 50 | 0.59 |
| 111 | CAAGTGGGAGGCGGCCCATGAGG (SEQ ID NO: 168) | chr6:29943431 | 3 | + | 70 | 1 | 50 | 50 | 50 | 50 | 0.58 |
| 112 | GGAAGAGCTCAGGTGGAGAAGGG (SEQ ID NO: 169) | chr6:29944605 | 5 | + | 55 | 0 | 50 | 50 | 50 | 50 | 0.56 |
| 113 | CAGCAATGATGCCCACGATGGGG (SEQ ID NO: 170) | chr6:29944522 | 5 | - | 55 | 2 | 50 | 50 | 50 | 50 | 0.58 |
| 114 | ATGGGGTAAGGAGGGAGATGGGG (SEQ ID NO: 171) | chr6:29944393 | 4 | + | 55 | 0 | 50 | 50 | 50 | 50 | 0.56 |
| 115 | CTGCGGAGATCACACTGACCTGG (SEQ ID NO: 172) | chr6:29944203 | 4 | + | 60 | 1 | 50 | 50 | 50 | 50 | 0.57 |
| 116 | GAAGAAATACCTCATGGAGTGGG (SEQ ID NO: 173) | chr6:29942760 | 2 | - | 40 | 0 | 50 | 50 | 50 | 50 | 0.56 |
| 117 | TCAGATCACCAAGCGCAAGTGGG (SEQ ID NO: 174) | chr6:29943416 | 3 | + | 50 | 0 | 50 | 50 | 50 | 50 | 0.56 |
| 118 | GCTCTTCCTCCTCCACATCACGG (SEQ ID NO: 175) | chr6:29944590 | 5 | - | 55 | 0 | 50 | 50 | 50 | 50 | 0.56 |
| 119 | GGATTACATCGCCCTGAACGAGG (SEQ ID NO: 176) | chr6:29943359 | 3 | + | 55 | 0 | 50 | 50 | 50 | 50 | 0.56 |
| 120 | AAGACAGCTCTGGGAAAAGAGGG (SEQ ID NO: 177) | chr6:29944486 | 5 | | 45 | 1 | 50 | 50 | 50 | 50 | 0.57 |
| 121 | CCAGGCAGTGACAGTGCCCAGGG (SEQ ID NO: 178) | chr6:29945230 | 7 | + | 65 | 1 | 50 | 50 | 50 | 50 | 0.57 |
| 122 | AGCTGTGATCACTGGAGCTGTGG (SEQ ID NO: 179) | chr6:29944561 | 5 | + | 55 | 0 | 50 | 50 | 50 | 50 | 0.56 |
| 123 | AAGGAGACGCTGCAGCGCACGGG (SEQ ID NO: 180) | chr6:29943522 | 3 | + | 65 | 1 | 50 | 50 | 50 | 50 | 0.56 |
| 124 | TCACAGCTCCAAGGAGAACCAGG (SEQ ID NO: 181) | chr6:29944546 | 5 | - | 55 | 2 | 50 | 50 | 50 | 50 | 0.57 |
| 125 | ACACTGACCTGGCAGCGGGATGG (SEQ ID NO: 182) | chr6:29944214 | 4 | + | 65 | 2 | 50 | 50 | 50 | 50 | 0.57 |
| 126 | GGCCCTGGGCTTCTACCCTGCGG (SEQ ID NO: 183) | chr6:29944186 | 4 | + | 70 | 1 | 50 | 50 | 50 | 50 | 0.56 |
| 127 | TTCTGTTTTTGTTCTACCCCAGG (SEQ ID NO: 184) | chr6:29945212 | 7 | + | 40 | 0 | 50 | 50 | 50 | 50 | 0.55 |
| 128 | CTGGGCACTGTCACTGCCTGGGG (SEQ ID NO: 185) | chr6:29945228 | 7 | - | 65 | 2 | 50 | 50 | 50 | 50 | 0.57 |
| 129 | GTGTCCCTCACAGCTTGTAAAGG (SEQ ID NO: 186) | chr6:29945260 | 7 | + | 50 | 0 | 50 | 50 | 50 | 50 | 0.55 |
| 130 | AGGTCAGTGTGATCTCCGCAGGG (SEQ ID NO: 187) | chr6:29944201 | 4 | - | 55 | 0 | 50 | 50 | 50 | 50 | 0.54 |
| 131 | CAGCCCACCATCCCCATCGTGGG (SEQ ID NO: 188) | chr6:29944511 | 5 | + | 65 | 0 | 50 | 50 | 50 | 50 | 0.54 |
| 132 | GATCACACTGACCTGGCAGCGGG (SEQ ID NO: 189) | chr6:29944210 | 4 | + | 60 | 2 | 50 | 50 | 50 | 50 | 0.56 |
| 133 | GCAGACCCTCATGCTGCACATGG (SEQ ID NO: 190) | chr6:29944351 | 4 | - | 60 | 0 | 50 | 50 | 50 | 50 | 0.54 |
| 134 | GCCAGCAATGATGCCCACGATGG (SEQ ID NO: 191) | chr6:29944524 | 5 | - | 60 | 1 | 50 | 50 | 50 | 50 | 0.55 |
| 135 | GATGCCCACGATGGGGATGGTGG (SEQ ID NO: 192) | chr6:29944515 | 5 | - | 65 | 1 | 50 | 50 | 50 | 50 | 0.55 |
| 136 | GCTGCAGCGCACGGGTACCAGGG (SEQ ID NO: 193) | chr6:29943530 | 3 | + | 70 | 2 | 50 | 50 | 50 | 50 | 0.55 |
| 137 | TGGCCTCATGGTCAGAGATGGGG (SEQ ID NO: 194) | chr6:29944150 | 4 | - | 55 | 1 | 50 | 50 | 50 | 50 | 0.54 |
| 138 | ACACGGAGCTCGTGGAGACCAGG (SEQ ID NO: 195) | chr6:29944254 | 4 | + | 65 | 3 | 50 | 50 | 50 | 50 | 0.56 |
| 139 | CACGCACGTGCCATCCAGGTAGG (SEQ ID NO: 196) | chr6:29943469 | 3 | - | 65 | 0 | 50 | 50 | 50 | 50 | 0.53 |
| 140 | TGGGCTGGGAAGACAGCTCTGGG (SEQ ID NO: 197) | chr6:29944495 | 5 | - | 60 | 1 | 50 | 50 | 50 | 50 | 0.54 |
| 141 | ACCCGCCCAGGTCTGGGTCAGGG (SEQ ID NO: 198) | chr6:29942606 | 1 | - | 70 | 2 | 50 | 50 | 50 | 50 | 0.55 |
| 142 | GTAGCCCACGGCGATGAAGCGGG (SEQ ID NO: 199) | chr6:29942814 | 2 | - | 65 | 2 | 50 | 50 | 50 | 50 | 0.55 |
| 143 | GGTGGGTCATATGTGTCTTGGGG (SEQ ID NO: 200) | chr6:29944126 | 4 | - | 50 | 0 | 50 | 50 | 50 | 50 | 0.52 |
| 144 | GAGGGACACATCAGAGCCCTGGG (SEQ ID NO: 201) | chr6:29945246 | 7 | - | 60 | 2 | 50 | 50 | 50 | 50 | 0.54 |
| 145 | CTCCGCAGATACCTGGAGAACGG (SEQ ID NO: 202) | chr6:29943498 | 3 | + | 55 | 0 | 50 | 50 | 50 | 50 | 0.52 |
| 146 | TTCTCCCCAGACGCCGAGGATGG (SEQ ID NO: 203) | chr6:29942535 | 1 | + | 65 | 0 | 50 | 50 | 50 | 50 | 0.52 |
| 147 | GGGGCCGGAGTATTGGGACCAGG (SEQ ID NO: 204) | chr6:29942920 | 2 | + | 70 | 2 | 50 | 50 | 50 | 50 | 0.54 |
| 148 | TCCGCAGATACCTGGAGAACGGG (SEQ ID NO: 205) | chr6:29943499 | 3 | + | 55 | 0 | 50 | 50 | 50 | 50 | 0.52 |
| 149 | CTTACCCCATCTCAGGGTGAGGG (SEQ ID NO: 206) | chr6:29944380 | 4 | - | 55 | 1 | 50 | 50 | 50 | 50 | 0.53 |
| 150 | ATGAGGCCACCCTGAGGTGCTGG (SEQ ID NO: 207) | chr6:29944164 | 4 | + | 65 | 1 | 50 | 50 | 50 | 50 | 0.53 |
| 151 | GTGGGCTGGGAAGACAGCTCTGG (SEQ ID NO: 208) | chr6:29944496 | 5 | - | 65 | 1 | 50 | 50 | 50 | 50 | 0.52 |
| 152 | GCAGGACGCCTACGACGGCAAGG (SEQ ID NO: 209) | chr6:29943338 | 3 | + | 70 | 3 | 50 | 50 | 50 | 50 | 0.54 |
| 153 | GATGGGGTAAGGAGGGAGATGGG (SEQ ID NO: 210) | chr6:29944392 | 4 | + | 55 | 0 | 50 | 50 | 50 | 50 | 0.51 |
| 154 | TCCCTCCTTACCCCATCTCAGGG (SEQ ID NO: 211) | chr6:29944386 | 4 | - | 55 | 1 | 50 | 50 | 50 | 50 | 0.52 |
| 155 | CCCCAGGCTCCCACTCCATGAGG (SEQ ID NO: 212) | chr6:29942751 | 2 | + | 70 | 0 | 50 | 50 | 50 | 50 | 0.51 |
| 156 | CTGGTTGTAGTAGCCGCGCAGGG (SEQ ID NO: 213) | chr6:29942994 | 2 | - | 60 | 2 | 50 | 50 | 50 | 50 | 0.53 |
| 157 | CACCTGCCATGTGCAGCATGAGG (SEQ ID NO: 214) | chr6:29944345 | 4 | + | 60 | 3 | 50 | 50 | 50 | 50 | 0.54 |
| 158 | CTCACCTTTACAAGCTGTGAGGG (SEQ ID NO: 215) | chr6:29945264 | 7 | - | 45 | 0 | 50 | 50 | 50 | 50 | 0.51 |
| 159 | GAGGGTTCGGGGCGCCATGACGG (SEQ ID NO: 216) | chr6:29942558 | 1 | - | 70 | 0 | 50 | 50 | 50 | 50 | 0.51 |
| 160 | CCCTCCTCCTGCTACTCTCGGGG (SEQ ID NO: 217) | chr6:29942576 | 1 | + | 65 | 0 | 50 | 50 | 50 | 50 | 0.51 |
| 161 | CCACCCCATCTCTGACCATGAGG (SEQ ID NO: 218) | chr6:29944147 | 4 | + | 60 | 1 | 50 | 50 | 50 | 50 | 0.51 |
| 162 | GGCCCCTCCTGCTCTATCCACGG (SEQ ID NO: 219) | chr6:29942903 | 2 | - | 65 | 0 | 50 | 50 | 50 | 50 | 0.5 |
| 163 | GTCTCCTGGTCCCAATACTCCGG (SEQ ID NO: 220) | chr6:29942924 | 2 | - | 55 | 0 | 50 | 50 | 50 | 50 | 0.5 |
| 164 | CCAGCCCACCATCCCCATCGTGG (SEQ ID NO: 221) | chr6:29944510 | 5 | + | 70 | 1 | 50 | 50 | 50 | 50 | 0.51 |
| 165 | ACCCTGAGATGGGGTAAGGAGGG (SEQ ID NO: 222) | chr6:29944385 | 4 | + | 55 | 1 | 50 | 50 | 50 | 50 | 0.51 |
| 166 | GTGGGTCATATGTGTCTTGGGGG (SEQ ID NO: 223) | chr6:29944125 | 4 | - | 50 | 0 | 50 | 50 | 50 | 50 | 0.5 |
| 167 | GATGTAATCCTTGCCGTCGTAGG (SEQ ID NO: 224) | chr6:29943346 | 3 | - | 50 | 0 | 50 | 50 | 50 | 50 | 0.5 |
| 168 | CTCAGATCACCAAGCGCAAGTGG (SEQ ID NO: 225) | chr6:29943415 | 3 | + | 55 | 0 | 50 | 50 | 50 | 50 | 0.5 |
| 169 | AGTAGCAGGAGGAGGGTTCGGGG (SEQ ID NO: 226) | chr6:29942569 | 1 | - | 60 | 1 | 50 | 50 | 50 | 50 | 0.51 |
| 170 | CACAGCCGCCCACTTCTGGAAGG (SEQ ID NO: 227) | chr6:29944293 | 4 | - | 65 | 1 | 50 | 50 | 50 | 50 | 0.5 |
| 171 | GTGGCCTCATGGTCAGAGATGGG (SEQ ID NO: 228) | chr6:29944151 | 4 | - | 55 | 1 | 50 | 50 | 50 | 50 | 0.5 |
| 172 | GTGGTGGGTCATATGTGTCTTGG (SEQ ID NO: 229) | chr6:29944128 | 4 | - | 50 | 0 | 50 | 50 | 50 | 50 | 0.49 |
| 173 | CTCCAGTGATCACAGCTCCAAGG (SEQ ID NO: 230) | chr6:29944555 | 5 | - | 55 | 0 | 50 | 50 | 50 | 50 | 0.49 |
| 174 | GACCCAGGACACGGAGCTCGTGG (SEQ ID NO: 231) | chr6:29944246 | 4 | + | 70 | 3 | 50 | 50 | 50 | 50 | 0.52 |
| 175 | CTGTGGTCGCTGCCGTGATGTGG (SEQ ID NO: 232) | chr6:29944578 | 5 | + | 65 | 0 | 50 | 50 | 50 | 50 | 0.48 |
| 176 | CTACCTGGATGGCACGTGCGTGG (SEQ ID NO: 233) | chr6:29943470 | 3 | + | 65 | 1 | 50 | 50 | 50 | 50 | 0.49 |
| 177 | CCTCACCCTGAGATGGGGTAAGG (SEQ ID NO: 234) | chr6:29944381 | 4 | + | 60 | 1 | 50 | 50 | 50 | 50 | 0.48 |
| 178 | ACCCTGAGGTGCTGGGCCCTGGG (SEQ ID NO: 235) | chr6:29944172 | 4 | + | 70 | 2 | 50 | 50 | 50 | 50 | 0.49 |
| 179 | CTCGTGGAGACCAGGCCTGCAGG (SEQ ID NO: 236) | chr6:29944262 | 4 | + | 70 | 1 | 50 | 50 | 50 | 50 | 0.48 |
| 180 | GCACTCACCCGCCCAGGTCTGGG (SEQ ID NO: 237) | chr6:29942612 | 1 | - | 70 | 2 | 50 | 50 | 50 | 50 | 0.49 |
| 181 | ACCTCATGGAGTGGGAGCCTGGG (SEQ ID NO: 238) | chr6:29942752 | 2 | - | 60 | 3 | 50 | 50 | 50 | 50 | 0.5 |
| 182 | CTTCCAGAAGTGGGCGGCTGTGG (SEQ ID NO: 239) | chr6:29944294 | 4 | + | 65 | 0 | 50 | 50 | 50 | 50 | 0.46 |
| 183 | GTGAGACAGCTGCCTTGTGTGGG (SEQ ID NO: 240) | chr6:29945452 | 8 | + | 55 | 1 | 50 | 50 | 50 | 50 | 0.47 |
| 184 | TACTACAACCAGAGCGAGGCCGG (SEQ ID NO: 241) | chr6:29943005 | 2 | + | 55 | 0 | 50 | 50 | 50 | 50 | 0.46 |
| 185 | TGGACGACACGCAGTTCGTGCGG (SEQ ID NO: 242) | chr6:29942838 | 2 | + | 60 | 3 | 50 | 50 | 50 | 50 | 0.49 |
| 186 | TCCCGTTCTCCAGGTATCTGCGG (SEQ ID NO: 243) | chr6:29943500 | 3 | - | 55 | 0 | 50 | 50 | 50 | 50 | 0.46 |
| 187 | CTCTTGGACCGCGGCGGACATGG (SEQ ID NO: 244) | chr6:29943389 | 3 | + | 70 | 4 | 50 | 50 | 50 | 50 | 0.5 |
| 188 | CCCATCGTGGGCATCATTGCTGG (SEQ ID NO: 245) | chr6:29944523 | 5 | + | 60 | 0 | 50 | 50 | 50 | 50 | 0.45 |
| 189 | AGAGTAGCAGGAGGAGGGTTCGG (SEQ ID NO: 246) | chr6:29942571 | 1 | - | 55 | 1 | 50 | 50 | 50 | 50 | 0.46 |
| 190 | CAAGCCCCTCACCCTGAGATGGG (SEQ ID NO: 247) | chr6:29944375 | 4 | + | 60 | 2 | 50 | 50 | 50 | 50 | 0.47 |
| 191 | CGTGGGCATCATTGCTGGCCTGG (SEQ ID NO: 248) | chr6:29944528 | 5 | + | 65 | 0 | 50 | 50 | 50 | 50 | 0.44 |
| 192 | CACGATGGGGATGGTGGGCTGGG (SEQ ID NO: 249) | chr6:29944509 | 5 | - | 65 | 0 | 50 | 50 | 50 | 50 | 0.44 |
| 193 | ATCTGGATGGTGTGAGAACCTGG (SEQ ID NO: 250) | chr6:29943264 | 3 | - | 50 | 0 | 50 | 50 | 50 | 50 | 0.44 |
| 194 | ACCTGCCATGTGCAGCATGAGGG (SEQ ID NO: 251) | chr6:29944346 | 4 | + | 55 | 3 | 50 | 50 | 50 | 50 | 0.47 |
| 195 | TCTCACCTTTACAAGCTGTGAGG (SEQ ID NO: 252) | chr6:29945265 | 7 | - | 45 | 0 | 50 | 50 | 50 | 50 | 0.44 |
| 196 | GGTTCCATCCCCTGCAGGCCTGG (SEQ ID NO: 253) | chr6:29944272 | 4 | - | 70 | 0 | 50 | 50 | 50 | 50 | 0.44 |
| 197 | CAGACCTGGGCGGGTGAGTGCGG (SEQ ID NO: 254) | chr6:29942614 | 1 | + | 70 | 1 | 50 | 50 | 50 | 50 | 0.44 |
| 198 | GGAGTGGCTCCGCAGATACCTGG (SEQ ID NO: 255) | chr6:29943491 | 3 | + | 65 | 1 | 50 | 50 | 50 | 50 | 0.44 |
| 199 | AAGAGCGCAGGTCCTCGTTCAGG (SEQ ID NO: 256) | chr6:29943371 | 3 | - | 60 | 0 | 50 | 50 | 50 | 50 | 0.43 |
| 200 | TCGTGGAGACCAGGCCTGCAGGG (SEQ ID NO: 257) | chr6:29944263 | 4 | + | 65 | 1 | 50 | 50 | 50 | 50 | 0.43 |
| 201 | ATGCCCACGATGGGGATGGTGGG (SEQ ID NO: 258) | chr6:29944514 | 5 | - | 60 | 1 | 50 | 50 | 50 | 50 | 0.43 |
| 202 | TTACCCCATCTCAGGGTGAGGGG (SEQ ID NO: 259) | chr6:29944379 | 4 | - | 55 | 3 | 50 | 50 | 50 | 50 | 0.45 |
| 203 | CTTCATCGCCGTGGGCTACGTGG (SEQ ID NO: 260) | chr6:29942818 | 2 | + | 65 | 0 | 50 | 50 | 50 | 50 | 0.42 |
| 204 | AGAGCGCAGGTCCTCGTTCAGGG (SEQ ID NO: 261) | chr6:29943370 | 3 | - | 60 | 0 | 50 | 50 | 50 | 50 | 0.41 |
| 205 | GAGTAGCAGGAGGAGGGTTCGGG (SEQ ID NO: 262) | chr6:29942570 | 1 | - | 60 | 1 | 50 | 50 | 50 | 50 | 0.42 |
| 206 | AACCCTCCTCCTGCTACTCTCGG (SEQ ID NO: 263) | chr6:29942574 | 1 | + | 55 | 0 | 50 | 50 | 50 | 50 | 0.41 |
| 207 | CTCCTTGGAGCTGTGATCACTGG (SEQ ID NO: 264) | chr6:29944553 | 5 | + | 55 | 1 | 50 | 50 | 50 | 50 | 0.42 |
| 208 | TGTGAGACAGCTGCCTTGTGTGG (SEQ ID NO: 265) | chr6:29945451 | 8 | + | 55 | 1 | 50 | 50 | 50 | 50 | 0.42 |
| 209 | TGAGGCCACCCTGAGGTGCTGGG (SEQ ID NO: 266) | chr6:29944165 | 4 | + | 65 | 1 | 50 | 50 | 50 | 50 | 0.41 |
| 210 | ACTCCACGCACGTGCCATCCAGG (SEQ ID NO: 267) | chr6:29943473 | 3 | - | 65 | 1 | 50 | 50 | 50 | 50 | 0.41 |
| 211 | CACTGACCTGGCAGCGGGATGGG (SEQ ID NO: 268) | chr6:29944215 | 4 | + | 65 | 1 | 50 | 50 | 50 | 50 | 0.41 |
| 212 | CATCTCAGGGTGAGGGGCTTGGG (SEQ ID NO: 269) | chr6:29944373 | 4 | - | 60 | 1 | 50 | 50 | 50 | 50 | 0.4 |
| 213 | TCTCCACGAGCTCCGTGTCCTGG (SEQ ID NO: 270) | chr6:29944249 | 4 | - | 65 | 1 | 50 | 50 | 50 | 50 | 0.4 |
| 214 | ACCCTCATGCTGCACATGGCAGG (SEQ ID NO: 271) | chr6:29944347 | 4 | - | 60 | 2 | 50 | 50 | 50 | 50 | 0.4 |
| 215 | AGCAGGAGGGGCCGGAGTATTGG (SEQ ID NO: 272) | chr6:29942913 | 2 | + | 65 | 1 | 50 | 50 | 50 | 50 | 0.39 |
| 216 | GCGTCTCCTTCCCGTTCTCCAGG (SEQ ID NO: 273) | chr6:29943509 | 3 | - | 65 | 0 | 50 | 50 | 50 | 50 | 0.38 |
| 217 | CTCCCTCCTTACCCCATCTCAGG (SEQ ID NO: 274) | chr6:29944387 | 4 | - | 60 | 0 | 50 | 50 | 50 | 50 | 0.38 |
| 218 | CGAGCTCCGTGTCCTGGGTCTGG (SEQ ID NO: 275) | chr6:29944243 | 4 | - | 70 | 0 | 50 | 50 | 50 | 50 | 0.37 |
| 219 | GTCACTCACCGGCCTCGCTCTGG (SEQ ID NO: 276) | chr6:29943013 | 2 | - | 70 | 1 | 50 | 50 | 50 | 50 | 0.38 |
| 220 | ATGGTCAGAGATGGGGTGGTGGG (SEQ ID NO: 277) | chr6:29944143 | 4 | - | 55 | 0 | 50 | 50 | 50 | 50 | 0.37 |
| 221 | TGGTGGGTCATATGTGTCTTGGG (SEQ ID NO: 278) | chr6:29944127 | 4 | - | 45 | 0 | 50 | 50 | 50 | 50 | 0.37 |
| 222 | TGAACGAGGACCTGCGCTCTTGG (SEQ ID NO: 279) | chr6:29943373 | 3 | + | 60 | 1 | 50 | 50 | 50 | 50 | 0.38 |
| 223 | ATTGCTGGCCTGGTTCTCCTTGG (SEQ ID NO: 280) | chr6:29944538 | 5 | + | 55 | 1 | 50 | 50 | 50 | 50 | 0.37 |
| 224 | ACCCTCCTCCTGCTACTCTCGGG (SEQ ID NO: 281) | chr6:29942575 | 1 | + | 60 | 1 | 50 | 50 | 50 | 50 | 0.37 |
| 225 | TACCTCATGGAGTGGGAGCCTGG (SEQ ID NO: 282) | chr6:29942753 | 2 | - | 60 | 1 | 50 | 50 | 50 | 50 | 0.36 |
| 226 | GCAGGAGGGGCCGGAGTATTGGG (SEQ ID NO: 283) | chr6:29942914 | 2 | + | 65 | 1 | 50 | 50 | 50 | 50 | 0.34 |
| 227 | GCGGCTGTGGTGGTGCCTTCTGG (SEQ ID NO: 284) | chr6:29944307 | 4 | + | 70 | 1 | 50 | 50 | 50 | 50 | 0.34 |
| 228 | GGAGCAGTTGAGAGCCTACCTGG (SEQ ID NO: 285) | chr6:29943455 | 3 | + | 60 | 2 | 50 | 50 | 50 | 50 | 0.34 |
| 229 | AGCACCTCAGGGTGGCCTCATGG (SEQ ID NO: 286) | chr6:29944162 | 4 | - | 65 | 2 | 50 | 50 | 50 | 50 | 0.34 |
| 230 | CTCCACGAGCTCCGTGTCCTGGG (SEQ ID NO: 287) | chr6:29944248 | 4 | - | 65 | 1 | 50 | 50 | 50 | 50 | 0.33 |
| 231 | CGGTGAGTGACCCCGGCCGGGGG (SEQ ID NO: 288) | chr6:29943025 | 2 | + | 80 | 1 | 50 | 50 | 50 | 50 | 0.78 |
| 232 | GGGCCCCCGAGAGTAGCAGGAGG (SEQ ID NO: 289) | chr6:29942580 | 1 | - | 75 | 1 | 50 | 50 | 50 | 50 | 0.75 |
| 233 | CAGGGCCCAGCACCTCAGGGTGG (SEQ ID NO: 290) | chr6:29944170 | 4 | - | 75 | 0 | 50 | 50 | 50 | 50 | 0.71 |
| 234 | GGGAGCCTGGGGGCGAGCAGTGG (SEQ ID NO: 291) | chr6:29942740 | 2 | - | 80 | 2 | 50 | 50 | 50 | 50 | 0.72 |
| 235 | CTGAGCCGCCATGTCCGCCGCGG (SEQ ID NO: 292) | chr6:29943397 | 3 | - | 75 | 1 | 50 | 50 | 50 | 50 | 0.71 |
| 236 | CCGGTGAGTGACCCCGGCCGGGG (SEQ ID NO: 293) | chr6:29943024 | 2 | + | 80 | 2 | 50 | 50 | 50 | 50 | 0.71 |
| 237 | CCTGCGCTCTTGGACCGCGGCGG (SEQ ID NO: 294) | chr6:29943383 | 3 | + | 75 | 1 | 50 | 50 | 50 | 50 | 0.7 |
| 238 | CGATGAAGCGGGGCTCCCCGCGG (SEQ ID NO: 295) | chr6:29942803 | 2 | - | 75 | 2 | 50 | 50 | 50 | 50 | 0.7 |
| 239 | GACCTGGCAGCGGGATGGGGAGG (SEQ ID NO: 296) | chr6:29944219 | 4 | + | 75 | 1 | 50 | 50 | 50 | 50 | 0.69 |
| 240 | TCCGTGTCCCGGCCCGGCCGCGG (SEQ ID NO: 297) | chr6:29942786 | 2 | + | 85 | 3 | 50 | 50 | 50 | 50 | 0.67 |
| 241 | CGGGTGAGTGCGGGGTCGGGAGG (SEQ ID NO: 298) | chr6:29942624 | 1 | + | 80 | 0 | 50 | 50 | 50 | 50 | 0.64 |
| 242 | TTTCTTCTCACAGATAGAAAAGG (SEQ ID NO: 299) | chr6:29945046 | 6 | + | 30 | 0 | 50 | 50 | 50 | 50 | 0.61 |
| 243 | GGATGGAGCCGCGGGCGCCGTGG (SEQ ID NO: 300) | chr6:29942886 | 2 | + | 85 | 1 | 50 | 50 | 50 | 50 | 0.62 |
| 244 | CGGACGGGCGCTTCCTCCGCGGG (SEQ ID NO: 301) | chr6:29943310 | 3 | + | 80 | 2 | 50 | 50 | 50 | 50 | 0.63 |
| 245 | TCGGACGGGCGCTTCCTCCGCGG (SEQ ID NO: 302) | chr6:29943309 | 3 | + | 75 | 2 | 50 | 50 | 50 | 50 | 0.62 |
| 246 | GCGGGCGCCGTGGATAGAGCAGG (SEQ ID NO: 303) | chr6:29942896 | 2 | + | 75 | 0 | 50 | 50 | 50 | 50 | 0.6 |
| 247 | GGAGCCCCGCTTCATCGCCGTGG (SEQ ID NO: 304) | chr6:29942809 | 2 | + | 75 | 1 | 50 | 50 | 50 | 50 | 0.61 |
| 248 | TTCTCACAGATAGAAAAGGAGGG (SEQ ID NO: 305) | chr6:29945050 | 6 | + | 35 | 0 | 50 | 50 | 50 | 50 | 0.6 |
| 249 | CCGTGTCCCGGCCCGGCCGCGGG (SEQ ID NO: 306) | chr6:29942787 | 2 | + | 90 | 3 | 50 | 50 | 50 | 50 | 0.6 |
| 250 | AAGCGGGGCTCCCCGCGGCCGGG (SEQ ID NO: 307) | chr6:29942798 | 2 | - | 85 | 3 | 50 | 50 | 50 | 50 | 0.6 |
| 251 | GGGTGAGTGCGGGGTCGGGAGGG (SEQ ID NO: 308) | chr6:29942625 | 1 | + | 75 | 0 | 50 | 50 | 50 | 50 | 0.57 |
| 252 | GGCCCTGACCCAGACCTGGGCGG (SEQ ID NO: 309) | chr6:29942604 | 1 | + | 75 | 2 | 50 | 50 | 50 | 50 | 0.58 |
| 253 | GCCCAGGGCCCAGCACCTCAGGG (SEQ ID NO: 310) | chr6:29944173 | 4 | - | 75 | 1 | 50 | 50 | 50 | 50 | 0.56 |
| 254 | TGGGCGGGTGAGTGCGGGGTCGG (SEQ ID NO: 311) | chr6:29942620 | 1 | + | 75 | 0 | 50 | 50 | 50 | 50 | 0.55 |
| 255 | GCCGGTGAGTGACCCCGGCCGGG (SEQ ID NO: 312) | chr6:29943023 | 2 | + | 80 | 0 | 50 | 50 | 50 | 50 | 0.55 |
| 256 | GCACGGGTACCAGGGGCCACGGG (SEQ ID NO: 313) | chr6:29943538 | 3 | + | 75 | 2 | 50 | 50 | 50 | 50 | 0.57 |
| 257 | CTCAGCCACTGCTCGCCCCCAGG (SEQ ID NO: 314) | chr6:29942735 | 2 | + | 75 | 2 | 50 | 50 | 50 | 50 | 0.56 |
| 258 | GCCCTGACCCAGACCTGGGCGGG (SEQ ID NO: 315) | chr6:29942605 | 1 | + | 75 | 3 | 50 | 50 | 50 | 50 | 0.56 |
| 259 | CGTGTCCCGGCCCGGCCGCGGGG (SEQ ID NO: 316) | chr6:29942788 | 2 | + | 90 | 3 | 50 | 50 | 50 | 50 | 0.55 |
| 260 | GCGAGGCCGGTGAGTGACCCCGG (SEQ ID NO: 317) | chr6:29943018 | 2 | + | 75 | 0 | 50 | 50 | 50 | 50 | 0.52 |
| 261 | CCCTGGCCCTGACCCAGACCTGG (SEQ ID NO: 318) | chr6:29942600 | 1 | + | 75 | 1 | 50 | 50 | 50 | 50 | 0.53 |
| 262 | GGGCGGGTGAGTGCGGGGTCGGG (SEQ ID NO: 319) | chr6:29942621 | 1 | + | 80 | 0 | 50 | 50 | 50 | 50 | 0.52 |
| 263 | GACCCCGCACTCACCCGCCCAGG (SEQ ID NO: 320) | chr6:29942618 | 1 | - | 80 | 0 | 50 | 50 | 50 | 50 | 0.51 |
| 264 | GACCTGGGCGGGTGAGTGCGGGG (SEQ ID NO: 321) | chr6:29942616 | 1 | + | 75 | 1 | 50 | 50 | 50 | 50 | 0.52 |
| 265 | GGGGCTCCCCGCGGCCGGGCCGG (SEQ ID NO: 322) | chr6:29942794 | 2 | - | 95 | 2 | 50 | 50 | 50 | 50 | 0.52 |
| 266 | GAAGCGGGGCTCCCCGCGGCCGG (SEQ ID NO: 323) | chr6:29942799 | 2 | - | 85 | 5 | 50 | 50 | 50 | 50 | 0.55 |
| 267 | AGTAGCCGCGCAGGGTCCCCAGG (SEQ ID NO: 324) | chr6:29942986 | 2 | - | 75 | 2 | 50 | 50 | 50 | 50 | 0.52 |
| 268 | GCTGCGACGTGGGGTCGGACGGG (SEQ ID NO: 325) | chr6:29943295 | 3 | + | 75 | 2 | 50 | 50 | 50 | 50 | 0.5 |
| 269 | GGGCTCCCCGCGGCCGGGCCGGG (SEQ ID NO: 326) | chr6:29942793 | 2 | - | 95 | 2 | 50 | 50 | 50 | 50 | 0.5 |
| 270 | GGCCGGTGAGTGACCCCGGCCGG (SEQ ID NO: 327) | chr6:29943022 | 2 | + | 80 | 2 | 50 | 50 | 50 | 50 | 0.49 |
| 271 | CTGACCGCGGGGTCGGGGCCAGG (SEQ ID NO: 328) | chr6:29943246 | 3 | + | 85 | 2 | 50 | 50 | 50 | 50 | 0.49 |
| 272 | GGCTGCGACGTGGGGTCGGACGG (SEQ ID NO: 329) | chr6:29943294 | 3 | + | 75 | 1 | 50 | 50 | 50 | 50 | 0.47 |
| 273 | CCTGCTACTCTCGGGGGCCCTGG (SEQ ID NO: 330) | chr6:29942583 | 1 | + | 75 | 0 | 50 | 50 | 50 | 50 | 0.46 |
| 274 | CACCCTGAGGTGCTGGGCCCTGG (SEQ ID NO: 331) | chr6:29944171 | 4 | + | 75 | 2 | 50 | 50 | 50 | 50 | 0.45 |
| 275 | CACCCGCCCAGGTCTGGGTCAGG (SEQ ID NO: 332) | chr6:29942607 | 1 | - | 75 | 2 | 50 | 50 | 50 | 50 | 0.45 |
| 276 | AGCCCAGGGCCCAGCACCTCAGG (SEQ ID NO: 333) | chr6:29944174 | 4 | - | 75 | 2 | 50 | 50 | 50 | 50 | 0.42 |
| 277 | GGCCGCCTCCCACTTGCGCTTGG (SEQ ID NO: 334) | chr6:29943424 | 3 | - | 75 | 1 | 50 | 50 | 50 | 50 | 0.41 |
| 278 | CGCACTCACCCGCCCAGGTCTGG (SEQ ID NO: 335) | chr6:29942613 | 1 | - | 75 | 0 | 50 | 50 | 50 | 50 | 0.39 |
| 279 | GTCCTCCCCATCCCGCTGCCAGG (SEQ ID NO: 336) | chr6:29944221 | 4 | - | 75 | 1 | 50 | 50 | 50 | 50 | 0.35 |
| 280 | CGCTGCAGCGCACGGGTACCAGG (SEQ ID NO: 337) | chr6:29943529 | 3 | + | 75 | 1 | 50 | 50 | 50 | 50 | 0.35 |
| 281 | GCAGGGTCCCCAGGTCCACTCGG (SEQ ID NO: 338) | chr6:29942977 | 2 | - | 70 | 0 | 2 | 0 | 18 | 20 | 0.46 |
| 282 | CGAGCCAGAGGATGGAGCCGCGG (SEQ ID NO: 339) | chr6:29942877 | 2 | + | 70 | 2 | 8 | 2 | 0 | 30 | 0.69 |
| 283 | AGTATTGGGACCAGGAGACACGG (SEQ ID NO: 340) | chr6:29942928 | 2 | + | 50 | 1 | 9 | 7 | 11 | 2 | 0.56 |
| 284 | AGAACCTGGCCCCGACCCCGCGG (SEQ ID NO: 341) | chr6:29943250 | 3 | - | 75 | 0 | 2 | 11 | 19 | 5 | 0.62 |
| 285 | TAATGTATGGCTGCGACGTGGGG (SEQ ID NO: 342) | chr6:29943286 | 3 | + | 50 | 0 | 2 | 16 | 5 | 26 | 0.56 |
| 286 | GTGGGAGGCGGCCCATGAGGCGG (SEQ ID NO: 343) | chr6:29943434 | 3 | + | 75 | 0 | 7 | 9 | 12 | 18 | 0.76 |
| 287 | CACGGGTACCAGGGGCCACGGGG (SEQ ID NO: 344) | chr6:29943539 | 3 | + | 75 | 0 | 6 | 12 | 16 | 14 | 0.6 |
| 288 | CAGGCTGCAAGTAAGTATGAAGG (SEQ ID NO: 345) | chr6:29945082 | 6 | + | 45 | 0 | 31 | 1 | 1 | 5 | 0.6 |

In certain cases, at least about 10%, 20%, 30%, 40%, 50%, 60%, 70%, 80%, 90%, 95%, 99%, 1-99%, or more of the population of cells (sich as BRCs, *e.g.,* SRCs), are viable after the cells are subjected to a procedure for delivering a Cas protein, a gRNA molecule, a RNP, and or one or more vectors expressing a Cas protein and/or a gRNA molecule across the cell membranes thereof (*e.g.,* by electroporation or some other procedure).

Various aspects of the CRISPR-Cas system are known in the art. Non-limiting aspects of this system are described, *e.g.,* in U.S. Patent No. 9,023,649, issued May 5, 2015; U.S.

Patent No. 9,074,199, issued July 7, 2015; U.S. Patent No. 8,697,359, issued April 15, 2014; U.S. Patent No. 8,932,814, issued January 13, 2015; U.S. Application Publication No. 2016/0298096, published October 13, 2016; Cho et al., (2013) Nature Biotechnology Vol 31 No 3 pp 230-232 (including supplementary information); and Jinek et al., (2012) Science Vol 337 No 6096 pp 816-821.

In the CRISPR/Cas nuclease system, the CRISPR locus, encodes RNA components of the system, and the Cas (CRISPR-associated) locus, encodes proteins. CRISPR loci in microbial hosts contain a combination of CRISPR-associated (Cas) genes as well as non-coding RNA elements capable of programming the specificity of the CRISPR-mediated polynucleotide cleavage.

The Type II CRISPR is one of the most well characterized systems and carries out targeted double-stranded breaks in four sequential steps. First, two non-coding RNAs, the pre-crRNA array and tracrRNA, are transcribed from the CRISPR locus. Second, tracrRNA hybridizes to the repeat regions of the pre-crRNA and mediates the processing of pre-crRNA into mature crRNAs containing individual spacer sequences. Third, the mature crRNA:tracrRNA complex directs Cas9 to the target DNA via Watson-Crick base-pairing between the spacer on the crRNA and the protospacer on the target DNA next to the protospacer adjacent motif (PAM), an additional requirement for target recognition. In engineered CRISPR/Cas9 systems, gRNA also called single-guide RNA ("gRNA") may replace crRNA and tracrRNA with a single RNA construct that includes the protospacer element and a linker loop sequence. Use of gRNA may simplify the components needed to use CRISPR/Cas9 for genome editing. The Cas9 species of different organisms have different PAM sequences. For example, *Streptococcus pyogenes* (Sp) has a PAM sequence of 5'-NGG-*3', Staphylococcus aureus* (Sa) has a PAM sequence of 5'-NGRRT-3' or 5'-NGRRN-3', *Neisseria meningitidis* (NM) has a PAM sequence of 5'-NNNNGATT-3', *Streptococcus thermophilus* (St) has a PAM sequence of 5'-NNAGAAW-3', *Treponema denticola* (Td) has a PAM sequence of 5'-NAAAAC-3'. Cas9 mediates cleavage of target DNA to create a DSB within the protospacer. Activity of the CRISPR/Cas system in nature comprises three steps: (i) insertion of alien DNA sequences into the CRISPR array to prevent future attacks, in a process called 'adaptation,' (ii) expression of the relevant proteins, as well as expression and processing of the array, followed by (iii) RNA-mediated interference with the alien polynucleotide. The alien polynucleotides come from viruses attaching the bacterial cell. Thus, in the bacterial cell, several of the so-called 'Cas' proteins are involved with the natural function of the CRISPR/Cas system and serve roles in functions such as insertion of the alien DNA, etc. CRISPR may also function with nucleases other than Cas9. Two genes from the Cpf1 family contain a RuvC-like endonuclease domain, but they lack Cas9's second HNH endonuclease domain. Cpf1 cleaves DNA in a staggered pattern and requires only one RNA rather than the two (tracrRNA and crRNA) needed by Cas9 for cleavage. Cpfl's preferred PAM is 5'-TTN, differing from that of Cas9 (3'-NGG) in both genomic location and GC-content. Mature crRNAs for Cpfl-mediated cleavage are 42-44 nucleotides in length, about the same size as Cas9's, but with the direct repeat preceding the spacer rather than following it. The Cpf1 crRNA is also much simpler in structure than Cas9's; only a short stem-loop structure in the direct repeat region is necessary for cleavage of a target. Cpf1 also does not require an additional tracrRNA. Whereas Cas9 generates blunt ends 3 nt upstream of the PAM site, Cpf1 cleaves in a staggered fashion, creating a five nucleotide 5' overhang 18-23 nt away from the PAM. Other CRISPR-associated proteins besides Cas9 may be used instead of Cas9. For example, CRISPR-associated protein 1 (Casl) is one of the two universally conserved proteins found in the CRISPR prokaryotic immune defense system. Cas1 is a metal-dependent DNA-specific endonuclease that produces double-stranded DNA fragments. Cas1 forms a stable complex with the other universally conserved CRISPR-associated protein, Cas2, which is part of spacer acquisition for CRISPR systems.

There are also CRISPR/Cas9 variants that do not use a PAM sequence such as NgAgo. NgAgo functions with a 24-nucleotide ssDNA guide and is believed to cut 8-11 nucleotides from the start of this sequence. The ssDNA is loaded as the protein folds and cannot be swapped to a different guide unless the temperature is increased to non-physiological 55° C. A few nucleotides in the target DNA are removed near the cut site. Techniques for using NgAgo are described in Gao, F. et al., DNA-guided Genome Editing Using the Natronobacterium Gregoryi Argonaute, 34 Nature Biotechnology 768 (2016). DSBs may be formed by making two single-stranded breaks at different locations creating a cut DNA molecule with sticky ends. Single-strand breaks or "nicks" may be formed by modified versions of the Cas9 enzyme containing only one active catalytic domain (called "Cas9 nickase"). Cas9 nickases still bind DNA based on gRNA specificity, but nickases are only capable of cutting one of the DNA strands. Two nickases targeting opposite strands are required to generate a DSB within the target DNA (often referred to as a "double nick" or "dual nickase" CRISPR system). This requirement dramatically increases target specificity, since it is unlikely that two off-target nicks will be generated within close enough proximity to cause a DSB. Techniques for using a dual nickase CRISPR system to create a DSB are described in Ran, et al., Double Nicking by RNA-Guided CRISPR Cas9 for Enhanced Genome Editing Specificity, 154 Cell 6:1380 (2013),.

Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Csel, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, as well as homologs and modified versions thereof. These enzymes are known; for example, the amino acid sequence of *S. pyogenes* Cas9 protein may be found in the SwissProt database under accession number Q99ZW2 (SEQ ID NO:1) and in the NCBI database as under accession number Q99ZW2.1. UniProt database accession numbers A0A0G4DEU5 and CDJ55032 provide another example of a Cas9 protein amino acid sequence (SEQ ID NO: 2). Another non-limiting example is a *Streptococcus thermophilus* Cas9 protein, the amino acid sequence of which may be found in the UniProt database under accession number Q03JI6.1 (SEQ ID NO:3). In certain embodiments, the unmodified CRISPR enzyme has DNA cleavage activity, such as Cas9. In certain embodiments, the CRISPR enzyme is Cas9, and may be Cas9 from *S. pyogenes* or *S. pneumoniae.* In certain embodiments, the CRISPR enzyme directs cleavage of one or both strands at the location of a target sequence, such as within the target sequence and/or within the complement of the target sequence. In certain embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In certain embodiments, a vector encodes a CRISPR enzyme that is mutated with respect to a corresponding wild-type enzyme such that the mutated CRISPR enzyme lacks the ability to cleave one or both strands of a target polynucleotide containing a target sequence. For example, an aspartate-to-alanine substitution (D10A, where the amino acid numbering is as shown in SEQ ID NO: 1) in the RuvC I catalytic domain of Cas9 from *S. pyogenes* converts Cas9 from a nuclease that cleaves both strands to a nickase (cleaves a single strand). Other examples of mutations that render Cas9 a nickase include, without limitation, H840A, N854A, and N863A (where the amino acid numbering is as shown in SEQ ID NO: 1). In certain embodiments, nickases may be used for genome editing via homologous recombination.

In certain embodiments, a Cas9 nickase may be used in combination with guide sequence(s), *e*.*g*., two guide sequences, which target respectively sense and antisense strands of the DNA target. This combination allows both strands to be nicked and used to induce NHEJ.

In certain embodiments, genetic manipulation is achieved using a base-editing protein. In certain embodiments, a base-editing protein is a modified protein (such as a Cas protein or another protein) that catalyzes transitions and transversions of one base into another (e.g., A to T, C to G, etc.) without the introduction (initial or otherwise) of a double stranded DNA break. CRISPR/Cas systems comprising a base-editor Cas protein represent a next-generation CRISPR system. Non-limiting descriptions of such systems are provided in Gaudelli et al. (2017) Programmable base editing of A•T to G•C in genomic DNA without DNA cleavage, Nature volume 551, pages 464-471; and Gehrke et al. (2018) High-precision CRISPR-Cas9 base editors with minimized bystander and off-target mutations, bioRxiv 273938; doi: https://doi.org/10.1101/273938, In certain embodiments, the base-editing protein is an adenine base editor (ABE) that mediates the conversion of A•T to G•C in genomic DNA. In certain embodiments, the base-editing protein comprises an adenosine deaminase *(e.g.,* a RNA adenosine deaminase modified to operate on DNA) fused to a catalytically impaired CRISPR-Cas mutant (such as a CRISPR-Cas9 mutant). In certain embodiments, the base-editing protein is an citidine base editor (ABE) that mediates the conversion of C•G to T•A in genomic DNA. In certain embodiments, the base-editing protein comprises a Cas9 nuclease bearing a mutation that converts it into a nickase (nCas9) fused to an Apolipoprotein B Editing Complex (APOBEC) (*e.g.,* rat APOBEC1 or a human APOBEC3A (A3A) cytidine deaminase) and a uracil glycosylase inhibitor (UGI). In certain embodiments, an RNA or DNA base editor comprises a Cas variant other than a Cas9 variant, such as a Cas13 variant. Various gene editing proteins and and Cas variants may be used for base-editing.

In certain embodiments, a gene editing protein being delivered (such as a a ZFN, megaTAL, or Cas protein) may comprise or be fused to a subcellular localization signal to comprise a gene editing fusion protein. Depending on context, a fusion protein comprising, *e.g.,* Cas9 and a nuclear localization signal may be referred to as "Cas9" herein without specifying the inclusion of the nuclear localization signal. In certain embodiments, the fusion-protein may contain a nuclear localization signal. In certain embodiments, a Cas protein may comprise more than one localization signals, such as 2, 3, 4, 5, or more nuclear localization signals. In certain embodiments, the localization signal is at the N-terminal end of the Cas protein and in other embodiments the localization signal is at the C-terminal end of the Cas protein.

Non-limiting examples of nuclear localization signals include GGSGPPKKKRKV (SEQ ID NO: 4), PKKKRKV (SEQ ID NO: 5), KR[PAATKKAGQA]KKKK (SEQ ID NO: 6), KR[XXXXXXXXXX]KKKK (SEQ ID NO: 7), KKXK (SEQ ID NO: 8), KRXK (SEQ ID NO: 9), KKXR (SEQ ID NO: 10), KRXR (SEQ ID NO: 11), AVKRPAATKKAGQAKKKKLD (SEQ ID NO: 12), MSRRRKANPTKLSENAKKLAKEVEN (SEQ ID NO: 13), PAAKRVKLD (SEQ ID NO: 14), PPKKKRKV (SEQ ID NO: 15), and KLKIKRPVK (SEQ ID NO: 16).

In certain embodiments, an enzyme coding sequence encoding a CRISPR enzyme is codon optimized for expression in particular cells, such as mammalian cells, *e.g.,* human cells.

In general, a guide sequence is any polynucleotide sequence having sufficient complementarity with a target polynucleotide sequence to hybridize with the target sequence and direct sequence-specific binding of a CRISPR complex to the target sequence. In certain embodiments, the degree of complementarity between a guide sequence and its corresponding target sequence, when optimally aligned using a suitable alignment algorithm, is about or more than about 90%, 95%, 97.5%, 98%, 99%, or more. In certain embodiments, the degree of complementarity is 100%. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies, ELAND (Illumina, San Diego, Calif.), SOAP (available at soap.genomics.org.cn), and Maq (available at maq.sourceforge.net). In certain embodiments, a guide sequence is about or more than about 15, 16, 17, 18, 19, or 20, or more nucleotides in length. In certain embodiments, a guide sequence is less than about 30, 25, 20, 15, or fewer nucleotides in length. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions.

A guide sequence may be selected to target any target sequence. In certain embodiments, the target sequence is a sequence within a genome of a cell. In certain embodiments, a target sequence is unique in the target genome.

In certain embodiments, a ZFN is used to genetically modify a cell. ZFNs are artificial restriction enzymes generated by fusing a zinc finger DNA-binding domain to a DNA-cleavage domain. Zinc finger domains can be engineered to target specific desired DNA sequences and this enables zinc-finger nucleases to target unique sequences within complex genomes. By taking advantage of endogenous DNA repair machinery, these reagents can be used to precisely alter the genomes of higher organisms. Alongside Cas9 and TALEN proteins, ZFN is becoming a prominent tool in the field of genome editing. A zinc finger nuclease is a site-specific endonuclease designed to bind and cleave DNA at specific positions. There are two protein domains. The first domain is the DNA binding domain, which contains eukaryotic transcription factors and the zinc finger. The second domain is the nuclease domain, which includes a restriction enzyme (such as the FokI restriction enzyme) and is responsible for the catalytic cleavage of DNA. The DNA-binding domains of individual ZFNs typically contain between three and six individual zinc finger repeats and can each recognize between 9 and 18 basepairs. In certain embodiments, if the zinc finger domains are perfectly specific for their intended target site then even a pair of 3-finger ZFNs that recognize a total of 18 basepairs can target a single locus in a mammalian (*e*.*g*., human) genome. In certain embodiments, smaller zinc-finger "modules" of known specificity are combined. In certain embodiments, a modular assembly process involves combining three separate zinc fingers that can each recognize a 3 basepair DNA sequence to generate a 3-finger array that can recognize a 9 basepair target site. In certain embodiments, a nonspecific cleavage domain from the type IIs restriction endonuclease FokI is used as the cleavage domain in a ZFN. This cleavage domain must dimerize in order to cleave DNA. In certain embodiments, a pair of ZFNs is used to target a non-palindromic DNA site. In certain embodiments, the cleavage domain is fused to the C-terminus of each zinc finger domain. In certain embodiments, two individual ZFNs bind opposite strands of DNA with their C-termini a certain distance apart.

TALENs are restriction enzymes that can be engineered to cut specific sequences of DNA. They are made by fusing a TAL effector DNA-binding domain to a DNA cleavage domain (*i.e.,* a nuclease that cuts DNA strands). Transcription activator-like effectors (TALEs) can be engineered to bind practically any desired DNA sequence, so when combined with a nuclease, DNA can be cut at specific locations. The restriction enzymes can be introduced into cells, for use in gene editing or for genome editing *in situ.* The DNA binding domain contains a repeated highly conserved 33-34 amino acid sequence with divergent 12^{th} and 13^{th} amino acids. These two positions, referred to as the Repeat Variable Diresidue (RVD), are highly variable and show a strong correlation with specific nucleotide recognition. This straightforward relationship between amino acid sequence and DNA recognition has allowed for the engineering of specific DNA-binding domains by selecting a combination of repeat segments containing the appropriate RVDs. Notably, slight changes in the RVD and the incorporation of "nonconventional" RVD sequences can improve targeting specificity. One of ordinary skill in the art will know how to design and use TALENs to create DSBs in dsDNA at a desired target site. Some suitable protocols are available in Hermann, M. et al., Mouse Genome Engineering Using Designer Nucleases, 86 J. Vis. Exp. 50930 (2014) and Sakuma, T. et al., Efficient TALEN Construction and Evaluation Methods for Human Cell and Animal Applications, 18(4) Genes Cells 315 (2013).

MegaTALs are derived from the combination of two distinct classes of DNA targeting enzymes. Meganucleases (also referred to as homing endonucleases) are single peptide chains that have the advantage of both DNA recognition and nuclease functions in the same domain. However, meganuclease target recognition is difficult to modify, and they often have reduced specificity and lower on-target cleavage efficiency than other genome targeting endonucleases. Transcription activator-like (TAL) effectors are DNA recognizing proteins that have been linked to separate DNA endonuclease domains in order to achieve a targeted DNA double strand break. In contrast to meganucleases, TALs are easily engineered to target specific DNA sequences. A megaTAL is the unification of a TAL effector with a meganuclease, in which the DNA binding region of a TAL effector is used to "address" a site-specific meganuclease adjacent to a single desired genomic target site. Non-limiting descriptions regarding megaTALs are described in Boissel et al. (2014) Nucleic Acids Research 42(4):2591-2601.

Aspects of the present disclosure relate to the modification of a genomic immunogenicity gene, *e.g.,* by mutation (*e.g.,* an insertion, a deletion, or a point mutation). In certain embodiments, the gene encodes a component of MHC-I. In certain embodiments, the gene encodes a component of MHC-II. Non-limiting examples of genomic immunogenicity genes include B2M, HLA-A, HLA-B, HLA-C, HLA-DRA, HLA-DRB1, HLA-DRB3, HLA-DRB4, HLA-DRB5, HLA-DPA1, HLA-DPA2, HLA-DQA1, and/or HLA-DQB1. Many variants of such genes naturally exist, *e.g.,* in humans.

In certain embodiments, the genetic modification is not genomic. In certain embodiments, short interfering and short hairpin RNAs are used to mediate gene silencing. In certain embodiments, the native genome is not modified, rather, RNAi acts at the RNA level to reduce or shut down the expression of a targeted gene such as B2M or HLA-A, thereby potentially achieving a similar final outcome as gene-editing.

### Genetically Engineered Renal Cell Populations

One major objective of the present invention is the use of gene editing techniques (*e.g.* CRISPR/Cas9) as previously described in a method for engineering non-alloreactive renal cells, in particular BRC *(e.g.,* SRC populations), for use in the treatment of chronic kidney disease. The methods and compositions described herein increase the immunocompatibility (immunoprivileged) of donor cells *(e.g.,* BRC such as SRC) for transplantation to a recipient subject, thereby establishing the ability to use a 'universal donor' renal cell population and allowing an 'allogeneic' renal cell population to be administered to patients without immunosuppression. This may be accomplished by the target-specific inactivation or modification of one or more immunogenicity genes *(e.g.,* an HLA gene) of a cell, resulting in donor cells that are suitable for transplantation into a recipient subject.

The discovery and application of the CRISPR/Cas9 system in mammalian cells has proved to be an effective and precise means for editing of target genes, *e.g.,* through the non-homologous end joining pathway (NHEJ), homology directed repair (HDR), or other DNA repair pathways. Co- delivery of a Cas9 molecule and a target-specific guide RNA (gRNA) molecule, optionally along with a donor DNA repair template molecule, facilitates gene-editing of a target sequence in the genome. Thus, the use of the CRISPR/Cas9 system to modify genes in cells is a promising strategy for treating multiple genetic disorders. For further details see, e.g. Sanders and Joung, Nature Biotechnology 32, 347-355 (2014).

In certain embodiments, a specific HLA allele is altered by contacting the cells described herein with a Cas9 molecule and at least one target-specific guide RNA (gRNA) that targets an endogenous immunogenicity gene to generate an immune compatible cell population (*e.g.,* an immune compatible renal cell population). Cells generated using the methods and compositions described herein are less likely to induce an immune response when transplanted in the recipient subject and/or are less likely to be rejected by the recipient's subject immune system. The ability to improve the immunocompatibility of donor cells that can be customized to be transplanted into any donor subject, regardless of immunogenicity gene haplotype of the donor, is particularly advantageous as it results in a dramatic increase in the pool of donor cells that can be used in the field of cell therapy for a multitude of clinical applications.

By inactivating a gene it is intended that the gene of interest is not expressed in a functional protein form. In certain embodiments, the genetic modification of the method relies on the expression, in provided cells to engineer, of the RNA guided endonuclease such that same catalyzes cleavage in one targeted gene thereby inactivating the targeted gene. The nucleic acid strand breaks caused by the endonuclease are commonly repaired through the distinct mechanisms of homologous recombination or non-homologous end joining (NHEJ). However, NHEJ is an imperfect repair process that often results in changes to the DNA sequence at the site of the cleavage. Mechanisms involve rejoining of what remains of the two DNA ends through direct re-ligation (Critchlow and Jackson 1998) or via the so- called microhomology-mediated end joining (Betts, Brenchley et al. 2003; Ma, Kim et al. 2003). Repair via non-homologous end joining (NHEJ) often results in small insertions or deletions and can be used for the creation of specific gene knockouts. Said modification may be a substitution, deletion, or addition of at least one nucleotide. Cells in which a cleavage-induced mutagenesis event, i.e. a mutagenesis event consecutive to an NHEJ event, has occurred can be identified and/or selected by well-known methods in the art. In certain embodiments, a base-editing CRISPR protein that does not introduce DNA breaks (*e.g.,* double stranded DNA breaks) is used. In certain embodiments, a base-editing protein is a modified Cas protein that catalyzes transitions and transversions of one base into another (*e.g.,* A to T, C to G, etc.) without the introduction (initial or otherwise) of a double stranded DNA break. In certain embodiments, the base-editing protein comprises an adenosine deaminase fused to a catalytically impaired CRISPR-Cas mutant (such as a CRISPR-Cas9 mutant). In certain embodiments, the base-editing protein comprises a Cas9 nuclease bearing a mutation that converts it into a nickase (nCas9) fused to an Apolipoprotein B Editing Complex (APOBEC) (*e.g.,* rat APOBEC1 or a human APOBEC3A (A3A) cytidine deaminase) and a uracil glycosylase inhibitor (UGI). In certain embodiments, the base-editing protein is a Cas13 mutant that acts specifically on RNA.

In certain embodiments, a method is provided for reducing the cell surface expression of a first allele of an endogenous immunogenicity gene(s) in a renal cell, comprising contacting the renal cell with a first allele-specific gRNA molecule and a Cas9 molecule, wherein the allele-specific gRNA molecule and the Cas9 molecule associate with the first allele of the endogenous immunogenicity gene, thereby reducing the cell surface expression of the first allele of the endogenous immunogenicity gene. In certain embodiments, a method is provided for producing a modified immune-compatible renal cell population by contacting a renal cell population with a first allele-specific modified gRNA molecule and a Cas9 molecule, wherein the first allele-specific modified gRNA molecule and the Cas9 molecule associate with a first allele of an endogenous immunogenicity gene, thereby modifying the first allele of the endogenous immunogenicity gene and producing the immune-compatible renal cell population. In certain embodiments, the genetically-modified renal cell has a decreased likelihood of rejection by the recipient subject based on increased matching between donor and recipient cells and reduced immunogenicity as determined by mixed lymphocyte or leukocyte reaction assays. In certain embodiments, the methods described herein may be used to alter a first, second, third, fourth, fifth sixth, second, eighth, ninth, tenth, or more alleles using one or more allele-specific gRNA molecule(s) and a Cas9 molecule. In certain embodiments, the alleles altered using the methods described herein may lead to the inactivation of the altered allele. In certain embodiments, the method may further comprise contacting the cell, or population of cells, with a second Cas9 molecule.

In certain embodiments, an *ex vivo* method is provided for making a composition comprising a population of cells enriched for a specific gene modification by contacting a population of cells with at least one target-specific gRNA molecule and a Cas9 molecule, wherein the target-specific gRNA molecule and the Cas9 molecule associate with a gene encoding an identifiable gene product; and enriching for cells that lack expression of the identifiable gene product. In certain embodiments, the step of enriching for cells that express the gene product in the methods described herein may comprise sorting the cells using, *e.g.,* FACS or MACS. In certain embodiments, the *ex vivo* method comprises enriching for a population of cells having an allele- specific gene modification, wherein the allele-specific gRNA molecule and the Cas9 molecule associate with a single allele of a gene encoding an identifiable gene product; and enriching for cells that express the identifiable gene product but do not express the first allele. In certain embodiments, the step of enriching for cells that express the gene but do not express the first allele may comprise contacting each of the plurality of cells with a first antibody that specifically binds to a first variant of the identifiable gene product encoded by the first allele of the gene and a second antibody that binds to a second variant of the identifiable gene product. In certain embodiments, the step of enriching for cells that express the gene but do not express the first allele may comprise detecting, in each cell of the plurality of cells, a substance or signal associated with a functional variant of the identifiable gene product. The population of cells may be a population of bioactive renal cells.

The methods described herein may optionally further comprise selecting a cell expressing a specific allele of a gene by sorting the population of cells using an allele-specific antibody. In certain embodiments, the population of cells may be sorted by fluorescence activated cell sorting (FACS) or immunomagnetic microbead mediated cell sorting. In certain embodiments, the method further comprises acquiring a sequence of the cell to confirm modification.

In certain embodiments, the gene may be an immunogenicity gene. In certaine embodiments, the identifiable gene product may be a cell surface marker. In certain embodiments, the identifiable gene product may be a human leukocyte antigen (HLA). In certain embodiments, the identifiable gene product may be a major histocompatibility antigen complex protein or a minor histocompatibility antigen (MiHA or mHA) *(e.g.,* a chemokine receptor). Non-limiting examples of mHAs include:

| **Examples of mHA** | **Chromosome** |
|---|---|
| **HA-1** | 19 |
| **HA-2** | 6 |
| **HA-8** | 9 |
| **HB-1** | 5 |
| **HY-A1** | Y |
| **HY-A2** | Y |
| **HY-B7** | Y |
| **HY-B8** | Y |
| **HY-B60** | Y |
| **HY-DQ5** | Y |

Although any genetic polymorphism could give rise to minor histoincompatibility, the number of genes encoding for minor antigens is limited. Around 10 minor antigens located on autosomal chromosomes have been identified among one hundred or so existing. The Y chro9mosme also bears some genes encoding mHAs. The first minor histocompatibility peptide identified was HA-2, a graft versus host disease-associated mHA. The most studied of all mHAs are HY-antigens. In certain embodiments, the gRNA molecule may comprise a targeting domain which is complementary to a target domain in a mHA gene. In certain embodiments, the gRNA molecule may comprise a targeting domain which is complementary to a target domain in a HA-1, HA-2, HA-8, HB-1, HY-A1, HY-A2, HY-B7, HY-B8, HY-B60, or HY-DQ5 gene.

In certain embodiments, the gRNA molecule may comprise a targeting domain which is complementary to a target domain in a HLA gene. The HLA gene may be selected from the group consisting of HLA- A, HLA-B, HLA-C, HLA-DRB1, HLA-DRB3/4/5, a HLA-DQ family gene *(e.g.,* HLA-DQA1 and/or HLA-DQB1), and a HLA-DP family gene *(e.g.,* HLA-DPA1 and/or HLA-DPA2).

In certain embodiments, the cell or population of cells may be a primary renal cell or population of selected renal cells. In certain embodiments, the population of cells may be a heterogeneous population of cells or a homogeneous population of cells.

In certain embodiments, to validate that one or more targeted HLA alleles have been inactivated by CRISPR/Cas9 activity, donor cells before and after targeting can be assayed for alteration of the allele sequence(s) or expression of the allele(s) using conventional methods (e.g., one or more of allele-specific PCR, qRT- PCR, or flow cytometry). In certain embodiments, donor cells with or without genome editing can be co- cultured with NK cells and the cytolytic activity directed against the donor cells is quantified to determine the down-regulation of HLA expression. After validation, cells having one or more mismatched donor HLA alleles inactivated and/or one or more matched recipient HLA alleles introduced can be enriched, isolated, or purified from the unmodified cells by conventional sorting methods.

### Cellular Aggregates

In an aspect, the formulations of the present disclosure contain cellular aggregates or spheroids. In certain cases, the cellular aggregate comprises a bioactive cell population described herein. In certain cases, the cellular aggregate comprises bioactive renal cells such as, for example, renal cell admixtures, enriched renal cell populations, and combinations of renal cell fractions and admixtures of renal cells with mesenchymal stem cells, endothelial progenitor cells, cells derived from the stromal vascular fraction of adipose, or any other non-renal cell population without limitation.

In certain cases, the bioactive renal cells of the disclosure may be cultured in 3D formats as described further herein. In certain cases, the term "organoid" refers to an accumulation of cells, with a phenotype and/or function, that recapitulates aspects of native kidney. In certain cases, organoids comprise mixed populations of cells, of a variety of lineages, which are typically found *in vivo* in a given tissue. In certain cases, the organoids of this disclosure are formed *in vitro,* via any means, whereby the cells of the disclosure form aggregates, which in turn may form spheroids, organoids, or a combination thereof. Such aggregates, spheroids or organoids, may, assume a structure consistent with a particular organ. In certain cases such aggregates, spheroids or organoids, express surface markers, which are typically expressed by cells of the particular organ. In certain cases, such aggregates, spheroids or organoids, produce compounds or materials, which are typically expressed by cells of the particular organ. In certain cases, the cells of the disclosure may be cultured on natural substrates, *e*.*g*., gelatin. In certain cases, the cells of the disclosure may be cultured on synthetic substrates, *e.g*., PLGA.

### Biomaterials

A variety of biomaterials may be combined with an active agent to provide the therapeutic formulations of the present disclosure. In certain cases, the biomaterials may be in any suitable shape *(e.g.,* beads) or form *(e.g.,* liquid, gel, etc.). Suitable biomaterials in the form of polymeric matrices are described in Bertram et al. U.S. Published Application 20070276507. In certain, polymeric matrices or scaffolds may be shaped into any number of desirable configurations to satisfy any number of overall system, geometry or space restrictions. In certain cases, a biomaterial is in the form of a liquid suspension. In certain cases, the matrices or scaffolds of the present disclosure may be three-dimensional and shaped to conform to the dimensions and shapes of an organ or tissue structure. For example, in the use of the polymeric scaffold for treating kidney disease, tubular transport deficiency, or glomerular filtration deficiency, a three-dimensional (3-D) matrix may be used that recapitulates aspects or the entirety of native kidney tissue structure and organization as well as that of renal parenchyma.

A variety of differently shaped 3-D scaffolds may be used. Naturally, the polymeric matrix may be shaped in different sizes and shapes to conform to differently sized patients. The polymeric matrix may also be shaped in other ways to accommodate the special needs of the patient. In certain cases, the polymeric matrix or scaffold may be a biocompatible, material (such as a porous polymeric scaffold). The scaffolds may be formed from a variety of synthetic or naturally-occurring materials including, but not limited to, open-cell polylactic acid (OPLA^{®}), cellulose ether, cellulose, cellulosic ester, fluorinated polyethylene, phenolic, poly-4-methylpentene, polyacrylonitrile, polyamide, polyamideimide, polyacrylate, polybenzoxazole, polycarbonate, polycyanoarylether, polyester, polyestercarbonate, polyether, polyetheretherketone, polyetherimide, polyetherketone, polyethersulfone, polyethylene, polyfluoroolefin, polyimide, polyolefin, polyoxadiazole, polyphenylene oxide, polyphenylene sulfide, polypropylene, polystyrene, polysulfide, polysulfone, polytetrafluoroethylene, polythioether, polytriazole, polyurethane, polyvinyl, polyvinylidene fluoride, regenerated cellulose, silicone, urea-formaldehyde, collagens, gelatin, alginate, laminins, fibronectin, silk, elastin, alginate, hyaluronic acid, agarose, or copolymers or physical blends thereof. Scaffolding configurations may range from soft porous scaffolds to rigid, shape-holding porous scaffolds. In certain cases, a scaffold is configured as a liquid solution that is capable of becoming a hydrogel, *e.g.,* hydrogel that is above a melting temperature.

In certain cases, the scaffold is derived from an existing kidney or other organ of human or animal origin, where the native cell population has been eliminated through application of detergent and/or other chemical agents and/or other enzymatic and/or physical methodologies known to those of ordinary skill in the art. In this case, the native three dimensional structure of the source organ is retained together with all associated extracellular matrix components in their native, biologically active context. In certain cases the scaffold is extracellular matrix derived from human or animal kidney or other organ. In certain cases, the configuration is assembled into a tissue-like structure through application of three dimensional bioprinting methodologies. In certain cases, the configuration is the liquid form of a solution that is capable of becoming a hydrogel.

In certain embodiments, the biomaterial is a hydrogel. Hydrogels may be formed from a variety of polymeric materials and are useful in a variety of biomedical applications. Hydrogels can be described physically as three-dimensional networks of hydrophilic polymers. Depending on the type of hydrogel, they contain varying percentages of water, but altogether do not dissolve in water. Despite their high water content, hydrogels are capable of additionally binding great volumes of liquid due to the presence of hydrophilic residues. Hydrogels swell extensively without changing their gelatinous structure. The basic physical features of a hydrogel can be specifically modified, according to the properties of the polymers used and a device used to administer the hydrogel.

The hydrogel material preferably does not induce an inflammatory response. Examples of other materials which can be used to form a hydrogel include (a) modified alginates, (b) polysaccharides (e.g. gellan gum and carrageenans) which gel by exposure to monovalent cations, (c) polysaccharides (e.g., hyaluronic acid) that are very viscous liquids or are thixotropic and form a gel over time by the slow evolution of structure, (d) gelatin or collagen, and (e) polymeric hydrogel precursors (e.g., polyethylene oxide-polypropylene glycol block copolymers and proteins). U.S. Pat. No. 6,224,893 B1 provides a detailed description of the various polymers, and the chemical properties of such polymers, that are suitable for making hydrogels in accordance with the present disclosure.

In a particular embodiment, the hydrogel used to formulate the biomaterials of the present disclosure is gelatin-based. Gelatin is a non-toxic, biodegradable and water-soluble protein derived from collagen, which is a major component of mesenchymal tissue extracellular matrix (ECM). Collagen is the main structural protein in the extracellular space in the various connective tissues in animal bodies. As the main component of connective tissue, it is the most abundant protein in mammals, making up from 25% to 35% of the whole-body protein content. Depending upon the degree of mineralization, collagen tissues may be rigid (bone), compliant (tendon), or have a gradient from rigid to compliant (cartilage). Collagen, in the form of elongated fibrils, is mostly found in fibrous tissues such as tendons, ligaments and skin. It is also abundant in corneas, cartilage, bones, blood vessels, the gut, intervertebral discs and the dentin in teeth. In muscle tissue, it serves as a major component of the endomysium. Collagen constitutes one to two percent of muscle tissue, and accounts for 6% of the weight of strong, tendinous muscles. Collagen occurs in many places throughout the body. Over 90% of the collagen in the human body, however, is type I.

To date, 28 types of collagen have been identified and described. They can be divided into several groups according to the structure they form: Fibrillar (Type I, II, III, V, XI). Non-fibrillar FACIT (Fibril Associated Collagens with Interrupted Triple Helices) (Type IX, XII, XIV, XVI, XIX). Short chain (Type VIII, X). Basement membrane (Type IV). Multiplexin (Multiple Triple Helix domains with Interruptions) (Type XV, XVIII). MACIT (Membrane Associated Collagens with Interrupted Triple Helices) (Type XIII, XVII). Other (Type VI, VII). The five most common types are: Type I: skin, tendon, vascular ligature, organs, bone (main component of the organic part of bone). Type II: cartilage (main collagenous component of cartilage) Type III: reticulate (main component of reticular fibers), commonly found alongside type I.Type IV: forms basal lamina, the epithelium-secreted layer of the basement membrane. Type V: cell surfaces, hair and placenta.

Gelatin retains informational signals including an arginine-glycine-aspartic acid (RGD) sequence, which promotes cell adhesion, proliferation and stem cell differentiation. A characteristic property of gelatin is that it exhibits Upper Critical Solution Temperature behavior (UCST). Above a specific temperature threshold of 40 °C, gelatin can be dissolved in water by the formation of flexible, random single coils. Upon cooling, hydrogen bonding and Van der Waals interactions occur, resulting in the formation of triple helices. These collagen-like triple helices act as junction zones and thus trigger the sol-gel transition. Gelatin is widely used in pharmaceutical and medical applications.

In certain embodiments, the hydrogel used to formulate the injectable cell compositions herein is based on porcine gelatin, which may be sourced from porcine skin and is commercially available, for example from Nitta Gelatin NA Inc (NC, USA) or Gelita USA Inc. (IA, USA). Gelatin may be dissolved, for example, in Dulbecco's phosphate-buffered saline (DPBS) to form a thermally responsive hydrogel, which can gel and liquefy at different temperatures. In certain embodiments, the hydrogel used to formulate the injectable cell compositions herein is based on recombinant human or animal gelatin expressed and purified using methodologies known to those of ordinary skill in the art. In certain embodiments, an expression vector containing all or part of the cDNA for Type I, alpha I human collagen is expressed in the yeast Pichia pastoris. Other expression vector systems and organisms will be known to those of ordinary skill in the art. In a particular embodiment, the gelatin-based hydrogel of the present disclosure is liquid at and above room temperature (22-28°C)and gels when cooled to refrigerated temperatures (2-8°C).

Those of ordinary skill in the art will appreciate that other types of synthetic or naturally-occurring materials known in the art may be used to form scaffolds as described herein.

In certain embodiments, the biomaterial used in accordance with the present disclosure is comprised of hyaluronic acid (HA) in hydrogel form, containing HA molecules ranging in size from 5.1 kDA to >2 x 105 kDa. HA may promote branching morphogenesis and three dimensional self-organization of associated bioactive cell populations. In certain embodiments, the biomaterial used in accordance with the present disclosure is comprised of hyaluronic acid in porous foam form, also containing HA molecules ranging in size from 5.1 kDA to >2 x 105 kDa. In certain embodiments, the hydrogel is derived from, or contains extracellular matrix sourced from kidney or any other tissue or organ without limitation. In yet another embodiment, the biomaterial used in accordance with the present disclosure is comprised of a poly-lactic acid (PLA)-based foam, having an open-cell structure and pore size of about 50 microns to about 300 microns.

### Temperature-Sensitive Biomaterials

The biomaterials described herein may also be designed or adapted to respond to certain external conditions, *e.g., in vitro* or *in vivo.* In the invention, the biomaterials are temperature-sensitive (*e.g.,* either *in vitro* or *in vivo*)*.* In certain cases, the biomaterials are adapted to respond to exposure to enzymatic degradation (*e.g.,* either *in vitro* or *in vivo*)*.* The biomaterials' response to external conditions can be fine-tuned as described herein. Temperature sensitivity of the formulation described can be varied by adjusting the percentage of a biomaterial in the formulation. For example, the percentage of gelatin in a solution can be adjusted to modulate the temperature sensitivity of the gelatin in the final formulation (*e*.*g*., liquid, gel, beads, etc.). Alternatively, biomaterials may be chemically crosslinked to provide greater resistance to enzymatic degradation. For instance, a carbodiimide crosslinker may be used to chemically crosslink gelatin beads thereby providing a reduced susceptibility to endogenous enzymes.

In an aspect, the formulations described herein incorporate biomaterials having properties which create a favorable environment for the active agent, such as bioactive renal cells, to be administered to a subject. In certain cases, the formulation contains a first biomaterial that provides a favorable environment from the time the active agent is formulated with the biomaterial up until the point of administration to the subject. In certain embodiments, the favorable environment concerns the advantages of having bioactive cells suspended in a substantially solid state versus cells in a fluid (as described herein) prior to administration to a subject. In certain embodiments, the first biomaterial is a temperature-sensitive biomaterial. The temperature-sensitive biomaterial may have (i) a substantially solid state at about 8°C or below, and (ii) a substantially liquid state at ambient temperature or above. In certain embodiments, the ambient temperature is about room temperature.

In the invention, the biomaterial is a temperature-sensitive biomaterial that can maintain at least two different phases or states depending on temperature. The biomaterial is capable of maintaining a first state at a first temperature, a second state at a second temperature, and/or a third state at a third temperature. The first, second or third state may be a substantially solid, a substantially liquid, or a substantially semi-solid or semi-liquid state. In certain embodiments, the biomaterial has a first state at a first temperature and a second state at a second temperature, wherein the first temperature is lower than the second temperature.

In the invention, the state of the temperature-sensitive biomaterial is a substantially solid state at a temperature of about 8°C or below. In certain embodiments, the substantially solid state is maintained at about 1°C, about 2°C, about 3°C, about 4°C, about 5°C, about 6°C, about 7°C, or about 8°C. In the invention, the substantially solid state has the form of a gel. In the invention, the state of the temperature-sensitive biomaterial is a substantially liquid state at ambient temperature or above. In certain embodiments, the substantially liquid state is maintained at about 25°C, about 25.5°C, about 26°C, about 26.5°C, about 27°C, about 27.5°C, about 28°C, about 28.5°C, about 29°C, about 29.5°C, about 30°C, about 31°C, about 32°C, about 33°C, about 34°C, about 35°C, about 36°C, or about 37°C. In certain embodiments, the ambient temperature is about room temperature.

In certain embodiments, the substantially solid state has the form of a bead.

The temperature-sensitive biomaterials may be provided in the form of a solution, in the form of a solid, in the form of beads, or in other suitable forms described herein and/or known to those of ordinary skill in the art. The cell populations and preparations described herein may be coated with, deposited on, embedded in, attached to, seeded, suspended in, or entrapped in a temperature-sensitive biomaterial. In certain embodiments, the cell populations described herein may be assembled as three dimensional cellular aggregrates or organoids or three dimensional tubular structures prior to complexing with the temperature-sensitive biomaterial or may be assembled as such upon complexing with the temperature-sensitive biomaterial. Alternatively, the temperature-sensitive biomaterial may be provided without any cells, such as, for example in the form of spacer beads. In this embodiment, the temperature sensitive biomaterial functions in a purely passive role to create space within the target organ for regenerative bioactivity, for example, angiogenesis or infiltration and migration of host cell populations.

In certain embodiments, the temperature-sensitive biomaterial has a transitional state between a first state and a second state In wherein, the transitional state is a solid-to-liquid transitional state between a temperature of about 8°C and about ambient temperature. In certain embodiments, the ambient temperature is about room temperature. In certain embodiments, the solid-to-liquid transitional state occurs at one or more temperatures of about 8°C, about 9°C, about 10°C, about 11°C, about 12°C, about 13°C, about 14°C, about 15°C, about 16°C, about 17°C, and about 18°C.

The temperature-sensitive biomaterials have a certain viscosity at a given temperature measured in centipoise (cP). In certain cases, the biomaterial has a viscosity at 25°C of about 1 cP to about 5 cP, about 1.1 cP to about 4.5 cP, about 1.2 cP to about 4 cP, about 1.3 cP to about 3.5 cP, about 1.4 cP to about 3.5 cP, about 1.5 cP to about 3 cP, about 1.55 cP to about 2.5 cP, or about 1.6 cP to about 2 cP. In certain cases, the biomaterial has a viscosity at 37°C of about 1.0 cP to about 1.15 cP. The viscosity at 37°C may be about 1.0 cP, about 1.01 cP, about 1.02 cP, about 1.03 cP, about 1.04 cP, about 1.05 cP, about 1.06 cP, about 1.07 cP, about 1.08 cP, about 1.09 cP, about 1.10 cP, about 1.11 cP, about 1.12 cP, about 1.13 cP, about 1.14 cP, or about 1.15 cP. In certain cases, the biomaterial is a gelatin solution. The gelatin is present at about 0.5%, about 0.55%, about 0.6%, about 0.65%, about 0.7%, about 0.75%, about 0.8%, about 0.85%, about 0.9%, about 0.95% or about 1%, (w/v) in the solution. In one example, the biomaterial is a 0.75% (w/v) gelatin solution in PBS. In certain cases, the 0.75% (w/v) solution has a viscosity at 25°C of about 1.6 cP to about 2 cP. In certain cases, the 0.75% (w/v) solution has a viscosity at 37°C of about 1.07 cP to about 1.08 cP. The gelatin solution may be provided in PBS, DMEM, or another suitable solvent.

In an aspect, the formulation contains bioactive cells combined with a second biomaterial that provides a favorable environment for the combined cells from the time of formulation up until a point after administration to the subject. In certain cases, the favorable environment provided by the second biomaterial concerns the advantages of administering cells in a biomaterial that retains structural integrity up until the point of administration to a subject and for a period of time after administration. In certain cases, the structural integrity of the second biomaterial following implantation is minutes, hours, days, or weeks. In certain cases, the structural integrity is less than one month, less than one week, less than one day, or less than one hour. The relatively short term structural integrity provides a formulation that can deliver the active agent and biomaterial to a target location in a tissue or organ with controlled handling, placement or dispersion without being a hindrance or barrier to the interaction of the incorporated elements with the tissue or organ into which it was placed.

In certain cases, the second biomaterial is a temperature-sensitive biomaterial that has a different sensitivity than the first biomaterial. The second biomaterial may have (i) a substantially solid state at about ambient temperature or below, and (ii) a substantially liquid state at about 37°C or above. In certain cases, the ambient temperature is about room temperature.

In certain cases, the second biomaterial is crosslinked beads. The crosslinked beads may have finely tunable *in vivo* residence times depending on the degree of crosslinking, as described herein. In certain cases, the crosslinked beads comprise bioactive cells and are resistant to enzymatic degradation as described herein. The formulations of the present disclosure may include the first biomaterial combined with an active agent, e.g., bioactive cells, with or without a second biomaterial combined with an active agent, e.g., bioactive cells. Where a formulation includes a second biomaterial, it may be a temperature sensitive bead and/or a crosslinked bead.

In an aspect, the present disclosure provides formulations that contain biomaterials which degrade over a period of time on the order of minutes, hours, or days. This is in contrast to a large body of work focusing on the implantation of solid materials that then slowly degrade over days, weeks, or months. In certain cases, the biomaterial has one or more of the following characteristics: biocompatibility, biodegradeability/bioresorbablity, a substantially solid state prior to and during implantation into a subject, loss of structural integrity (substantially solid state) after implantation, and cytocompatible environment to support cellular viability and proliferation. The biomaterial's ability to keep implanted particles spaced out during implantation enhances native tissue ingrowth. The biomaterial also facilitates implantation of solid formulations. The biomaterial provides for localization of the formulation described herein since insertion of a solid unit helps prevent the delivered materials from dispersing within the tissue during implantation. For cell-based formulations, a solid biomaterial also improves stability and viability of anchorage dependent cells compared to cells suspended in a fluid. However, the short duration of the structural integrity means that soon after implantation, the biomaterial does not provide a significant barrier to tissue ingrowth or integration of the delivered cells/materials with host tissue.

In an aspect, the present disclosure provides formulations that contain biomaterials which are implanted in a substantially solid form and then liquefy/melt or otherwise lose structural integrity following implantation into the body. This is in contrast to the significant body of work focusing on the use of materials that can be injected as a liquid, which then solidify in the body.

### Biocompatible Beads

As also described the formulation may include a temperature-sensitive biomaterial described herein and a population of biocompatible beads containing a biomaterial. In certain cases, the beads are crosslinked. Crosslinking may be achieved using any suitable crosslinking agent known to those of ordinary skill in the art, such as, for example, carbodiimides; aldehydes (e.g. furfural, acrolein, formaldehyde, glutaraldehyde, glyceryl aldehyde), succinimide-based crosslinkers {Bis(sulfosuccinimidyl) suberate (BS3), Disuccinimidyl glutarate (DSG), Disuccinimidyl suberate (DSS), Dithiobis(succinimidyl propionate), Ethylene glycolbis(sulfosuccinimidylsuccinate), Ethylene glycolbis(succinimidylsuccinate) (EGS), Bis(Sulfosuccinimidyl) glutarate (BS2G), Disuccinimidyl tartrate (DST); epoxides (Ethylene glycol diglycidyl ether , 1,4 Butanediol diglycidyl ether); saccharides (glucose and aldose sugars); sulfonic acids and p-toluene sulfonic acid; carbonyldiimidazole; genipin; imines; ketones; diphenylphosphorylazide (DDPA); terephthaloyl chloride; cerium (III) nitrate hexahydrate; microbial transglutaminase; and hydrogen peroxide. Those of ordinary skill in the art will appreciate other suitable crosslinking agents and crosslinking methods for use in accordance with the present disclosure.

In certain cases, the beads are carbodiimide-crosslinked beads. The carbodiimide-crosslinked beads may be crosslinked with a carbodiimide selected from the group consisting of 1-Ethyl-3-[3-dimethylaminopropyl] carbodiimide hydrochloride (EDC), DCC - N,N'-dicyclohexylcarbodiimide (DCC), and N,N'-Diisopropylcarbodiimide (DIPC). Beads treated with lower concentration of EDC were expected to have a higher number of free primary amines, while samples treated with high concentrations of crosslinker would have most of the primary amines engaged in amide bonds. The intensity of the orange color developed by the covalent bonding between the primary amine and picrylsulfonic acid, detectable spectrophotometrically at 335 nm, is proportional to the number of primary amines present in the sample. When normalized per milligram of protein present in the sample, an inverse correlation between the number of free amines present and the initial concentration of EDC used for crosslinking can be observed. This result is indicative of differential bead crosslinking, dictated by the amount of carbodiimide used in the reaction. In general, crosslinked beads exhibit a reduced number of free primary amines as compared to non-crosslinked beads.

The crosslinked beads have a reduced susceptibility to enzymatic degradation as compared to non-crosslinked biocompatible beads, thereby providing beads with finely tunable *in vivo* residence times. For example, the crosslinked beads are resistant to endogenous enzymes, such as collagenases. The provision of crosslinked beads is part of a delivery system that facilitate one or more of: (a) delivery of attached cells to the desired sites and creation of space for regeneration and ingrowth of native tissue and vascular supply; (b) ability to persist at the site long enough to allow cells to establish, function, remodel their microenvironment and secrete their own extracellular matrix (ECM); (c) promotion of integration of the transplanted cells with the surrounding tissue; (d) ability to implant cells in a substantially solid form; (e) short term structural integrity that does not provide a significant barrier to tissue ingrowth, de novo angiogenesis or integration of delivered cells/materials with the host tissue; (f) localized *in vivo* delivery in a substantially solid form thereby preventing dispersion of cells within the tissue during implantation; (g) improved stability and viability of anchorage dependent cells compared to cells suspended in a fluid; and (h) biphasic release profile when cells are delivered 1) in a substantially solid form *(e.g.,* attached to beads), and 2) in a substantially liquid form (*e*.*g*., suspended in a fluid); i) recapitulation and mimicry of the three dimensional biological niche or renal parenchyma from which these bioactive cell populations were derived.

The present disclosure provides crosslinked beads containing gelatin. Non-crosslinked gelatin beads are not suitable for a bioactive cell formulation because they rapidly lose integrity and cells dissipate from the injection site. In contrast, highly crosslinked gelatin beads may persist too long at the injection site and may hinder the *de-novo* ECM secretion, cell integration, angiogenesis and tissue regeneration. The present disclosure allows for the *in vivo* residence time of the crosslinked beads to be finely tuned. In order to tailor the biodegradability of biomaterials, different crosslinker concentrations of carbodiimide are used while the overall reaction conditions were kept constant for all samples. For example, the enzymatic susceptibility of carbodiimide-crosslinked beads can be finely tuned by varying the concentration of crosslinking agent from about zero to about 1M.

In certain embodiments, the concentration is about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 26 mM, about 27 mM, about 28 mM, about 29 mM, about 30 mM, about 31 mM, about 32 mM, about 33 mM, about 34 mM, about 35 mM, about 36 mM, about 37 mM, about 38 mM, about 39 mM, about 40 mM, about 41 mM, about 42 mM, about 43 mM, about 44 mM, about 45 mM, about 46 mM, about 47 mM, about 48 mM, about 49 mM, about 50 mM, about 55 mM, about 60 mM, about 65 mM, about 70 mM, about 75 mM, about 80 mM, about 85 mM, about 90 mM, about 95 mM, or about 100 mM. The crosslinker concentration may also be about 0.15 M, about 0.2 M, about 0.25 M, about 0.3 M, about 0.35 M, about 0.4 M, about 0.45 M, about 0.5 M, about 0.55 M, about 0.6 M, about 0.65 M, about 0.7 M, about 0.75 M, about 0.8 M, about 0.85 M, about 0.9 M, about 0.95 M, or about 1 M. In certain cases, the crosslinking agent is 1-Ethyl-3-[3-dimethylaminopropyl] carbodiimide hydrochloride (EDC). In certain cases, the EDC-crosslinked beads are gelatin beads. The % degradation of the beads can be finely tuned depending upon the concentration of crosslinking agent. In certain cases, gelatin beads may be mixed with beads or microparticles other than gelatin (for example, without limitation, alginate or HA) to additionally facilitate the potency of the bioactive cell population being delivered.

Crosslinked beads may have certain characteristics that favor the seeding, attachment, or encapsulation of bioactive cell populations. For example, the beads may have a porous surface and/or may be substantially hollow. The presence of pores provides an increased cell attachment surface allowing for a greater number of cells to attach as compared to a nonporous or smooth surface. In addition, the pore structure can support host tissue integration with the porous beads supporting the formation of *de novo* tissue. The beads have a size distribution that can be fitted to a Weibull plot corresponding to the general particle distribution pattern. In certain cases, the crosslinked beads have an average diameter of less than about 120 µm, about 115 µm, about 110 µm, about 109 µm, about 108 µm, about 107 µm, about 106 µm, about 105 µm, about 104 µm, about 103 µm, about 102 µm, about 101 µm, about 100 µm, about 99 µm, about 98 µm, about 97 µm, about 96 µm, about 95 µm, about 94 µm, about 93 µm, about 92 µm, about 91 µm, or about 90 µm. The characteristics of the crosslinked beads vary depending upon the casting process. For instance, a process in which a stream of air is used to aerosolize a liquid gelatin solution and spray it into liquid nitrogen with a thin layer chromatography reagent sprayer (ACE Glassware) is used to provide beads having the afore-mentioned characteristics. Those of skill in the art will appreciate that modulating the parameters of the casting process provides the opportunity to tailor different characteristics of the beads, e.g., different size distributions. In certain cases, the microtopography, surface and internal characteristics of the beads may be further modified to facilitate cell attachment.

The cytocompatibility of the crosslinked beads is assessed *in vitro* prior to formulation using cell culture techniques in which beads are cultured with cells that correspond to the final bioactive cell formulation. For instance, the beads are cultured with primary renal cells prior to preparation of a bioactive renal cell formulation and live/dead cell assays are used to confirm cytocompatibility. In addition to cellular viability, specific functional tests to measure cellular metabolic activity, secretion of certain key cytokines and growth factors and exosomes and the expression of certain key protein and nucleic acid markers including miRNAs associated with functionally bioactive renal cell populations are well known to those of ordinary skill in the art and are additionally used to confirm cell potency upon formulation with crosslinked beads.

In certain formulations, the biocompatible crosslinked beads are combined with a temperature-sensitive biomaterial in solution at about 5% (w/w) to about 15% (w/w) of the volume of the solution. The crosslinked beads may be present at about 5% (w/w), about 5.5% (w/w), about 6% (w/w), about 6.5% (w/w), about 7% (w/w), about 7.5% (w/w), about 8% (w/w), about 8.5% (w/w), about 9% (w/w), about 9.5% (w/w), about 10% (w/w), about 10.5% (w/w), about 11% (w/w), about 11.5% (w/w), about 12% (w/w), about 12.5% (w/w), about 13% (w/w), about 13.5% (w/w), about 14% (w/w), about 14.5% (w/w), or about 15 % (w/w) of the volume of the solution.

In an aspect, the present disclosure provides formulations that contain biomaterials which degrade over a period time on the order of minutes, hours, or days. This is in contrast to a large body of work focusing on the implantation of solid materials that then slowly degrade over days, weeks, or months.

Also described are formulations having biocompatible crosslinked beads seeded with bioactive cells together with a delivery matrix. In certain cases, the delivery matrix has one or more of the following characteristics: biocompatibility, biodegradeability/bioresorbability, a substantially solid state prior to and during implantation into a subject, loss of structural integrity (substantially solid state) after implantation, and a cytocompatible environment to support cellular viability. The delivery matrix's ability to keep implanted particles (e.g., crosslinked beads) spaced out during implantation enhances native tissue ingrowth. If the delivery matrix is absent, then compaction of cellularized beads during implantation can lead to inadequate room for sufficient tissue ingrowth. The delivery matrix facilitates implantation of solid formulations. In addition, the short duration of the structural integrity means that soon after implantation, the matrix does not provide a significant barrier to tissue ingrowth, de novo angiogenesis or integration of the delivered cells/materials with host tissue. The delivery matrix provides for localization of the formulation described herein since insertion of a solid unit helps prevent the delivered materials from dispersing within the tissue during implantation. In certain embodiments, application of a delivery matrix as described herein helps prevent rapid loss of implanted cells through urination upon delivery to the renal parenchyme. For cell-based formulations, a solid delivery matrix improves stability and viability of anchorage dependent cells compared to cells suspended in a fluid.

In certain cases, the delivery matrix is a population of biocompatible beads that is not seeded with cells. In certain cases, the unseeded beads are dispersed throughout and in between the individual cell-seeded beads. The unseeded beads act as "spacer beads" between the cell-seeded beads prior to and immediately after transplantation. The spacer beads contain a temperature-sensitive biomaterial having a substantially solid state at a first temperature and a substantially liquid state at a second temperature, wherein the first temperature is lower than the second temperature. For example, the spacer beads contain a biomaterial having a substantially solid state at about ambient temperature or below and a substantially liquid state at about 37°C, such as that described herein. In certain cases, the ambient temperature is about room temperature. In certain cases, the biomaterial is a gelatin solution. The gelatin solution is present at about 4%, about 4.5%, about 5%, about 5.5%, about 6%, about 6.5%, about 7%, about 7.5%, about 8%, about 8.5%, about 9%, about 9.5%, about 10%, about 10.5%, or about 11%, (w/v). The gelatin solution may be provided in PBS, cell culture media (e.g., DMEM), or another suitable solvent. In certain cases, the biomaterial is hyaluronic acid. In certain cases, the biomaterial is decellularized extracellular matrix sourced from human or animal kidney which may be further reconstituted as a hydrogel.

In an aspect, the present disclosure provides formulations that contain biomaterials which are implanted in a substantially solid form (e.g., spacer beads) and then liquefy/melt or otherwise lose structural integrity following implantation into the body. This is in contrast to the significant body of work focusing on the use of materials that can be injected as a liquid, which then solidify in the body.

The temperature-sensitivity of spacer beads can be assessed *in vitro* prior to formulation. Spacer beads can be labeled and mixed with unlabeled non-temperature-sensitive beads. The mixture is then incubated at 37°C to observe changes in physical transition. The loss of shape of the labeled temperature-sensitive beads at the higher temperature is observed over time. For example, temperature-sensitive gelatin beads may be made with Alcian blue dye to serve as a marker of physical transition. The blue gelatin beads are mixed with crosslinked beads (white), loaded into a catheter, then extruded and incubated in 1X PBS, pH 7.4, at 37°C. The loss of shape of the blue gelatin beads is followed microscopically at different time points. Changes in the physical state of the blue gelatin beads are visible after 30 min becoming more pronounced with prolonged incubation times. The beads do not completely dissipate because of the viscosity of the material.

### Modified Release Formulations

The formulations of the present disclosure may be provided as modified release formulations. In general, the modified release is characterized by an initial release of a first active agent upon administration followed by at least one additional, subsequent release of a second active agent. The first and second active agents may be the same or they may be different. In certain cases, the formulations provide modified release through multiple components in the same formulation. In certain cases, the modified release formulation contains an active agent as part of a first component that allows the active agent to move freely throughout the volume of the formulation, thereby permitting immediate release at the target site upon administration. The first component may be a temperature-sensitive biomaterial having a substantially liquid phase and a substantially solid phase, wherein the first component is in a substantially liquid phase at the time of administration. In certain cases, the active agent is in the substantially liquid phase such that it is substantially free to move throughout the volume of the formulation, and therefore is immediately released to the target site upon administration.

In certain cases, the modified release formulation has an active agent as part of a second component in which the active agent is attached to, deposited on, coated with, embedded in, seeded upon, or entrapped in the second component, which persists before and after administration to the target site. The second component contains structural elements with which the active agent is able to associate with, thereby preventing immediate release of the active agent from the second component at the time of administration. For example, the second component is provided in a substantially solid form, *e*.*g*., biocompatible beads, which may be crosslinked to prevent or delay *in vivo* enzymatic degradation. **In** certain cases, the active agent in the substantially solid phase retains its structural integrity within the formulation before and after administration and therefore it does not immediately release the active agent to the target site upon administration. Suitable carriers for modified release formulations have been described herein but those of ordinary skill in the art will appreciate other carriers that are appropriate for use in the present disclosure.

In certain cases, the formulation provides an initial rapid delivery/release of delivered elements, including cells, nanoparticles, therapeutic molecules, etc. followed by a later delayed release of elements. In certain cases, the formulation provides an initial rapid delivery/release of exosomes, miRNA and other bioactive nucleic acid or protein molecules that are soluble and are secreted, bioactive products sourced from renal or other cell populations. Other molecules or therapeutic agents associated with regenerative bioactivity will be appreciated by those of ordinary skill in the art. The formulations of the present disclosure can be designed for such biphasic release profile where the agent to be delivered is provided in both an unattached form *(e.g.,* cells in a solution) and an attached form *(e.g.,* cells together with beads or another suitable carrier). Upon initial administration, the unencumbered agent is provided immediately to the site of delivery while release of the encumbered agent is delayed until structural integrity of the carrier *(e.g.,* beads) fails at which point the previously attached agent is released. As discussed below, other suitable mechanisms of release will be appreciated by those of ordinary skill in the art.

The time delay for release can be adjusted based upon the nature of the active agent. For example, the time delay for release in a bioactive cell formulation may be on the order of seconds, minutes, hours, or days. In some circumstances, a delay on the order of weeks may be appropriate. For other active agents, such as small or large molecules, the time delay for release in a formulation may be on the order of seconds, minutes, hours, days, weeks, or months. It is also possible for the formulation to contain different biomaterials that provide different time delay release profiles. For example, a first biomaterial with a first active agent may have a first release time and a second biomaterial with a second active agent may have a second release time. The first and second active agent may be the same or different.

As discussed herein, the time period of delayed release may generally correspond to the time period for loss of structural integrity of a biomaterial. However, those of ordinary skill in the art will appreciate other mechanisms of delayed release. For example, an active agent may be continually released over time independent of the degradation time of any particular biomaterial, *e*.*g*., diffusion of a drug from a polymeric matrix. In addition, bioactive cells can migrate away from a formulation containing a biomaterial and the bioactive cells to native tissue. In certain embodiments, bioactive cells migrate off of a biomaterial, *e*.*g*., a bead, to the native tissue. In one case, bioactive cells migrate off a biomaterial to the native tissue and induce secretion of growth factors, cytokines, exosomes, miRNA and other nucleic acids and proteins associated with regenerative bioactivity. In certain cases, exosomes and other extracellular vesicles, as well as miRNA, other bioactive nucleic acids and proteins migrate off of a biomaterial. In yet another embodiment, bioactive cells migrate off a biomaterial to the native tissue and mediate mobilization of host stem and progenitor cells that then migrate or home towards the injury or disease location.

Biodegradable, biocompatible polymers can be used, such as ethylene vinyl acetate, polyanhydrides, polyglycolic acid, collagen, polyorthoesters, and polylactic acid. Prolonged absorption of injectable formulations can be brought about by including in the formulation an agent that delays absorption, for example, monostearate salts and gelatin. Many methods for the preparation of such formulations are patented or generally known to those skilled in the art. See, e.g., Sustained and Controlled Release Drug Delivery Systems, J. R. Robinson, ed., Marcel Dekker, Inc., New York, 1978. Additional methods applicable to the controlled or extended release of polypeptide agents are described, for example, in U.S. Pat. Nos. 6,306,406 and 6,346,274, as well as, for example, in U.S. Patent Application Nos. US20020182254 and US20020051808.

### Bioactive Cell Formulations

The bioactive cell formulations described herein contain implantable constructs made from the above-referenced biomaterials having the genetically modified bioactive renal cells described herein for the treatment of kidney disease in a subject in need. In certain cases, the construct is made up of a biocompatible material or biomaterial, scaffold or matrix composed of one or more synthetic or naturally-occurring biocompatible materials and one or more cell populations or admixtures of cells described herein deposited on or embedded in a surface of the scaffold by attachment and/or entrapment. In certain cases the construct is made up of a biomaterial and one or more cell populations or admixtures of cells described herein coated with, deposited on, deposited in, attached to, entrapped in, embedded in, seeded, or combined with the biomaterial component(s). Any of the cell populations described herein, including enriched cell populations or admixtures thereof, may be used in combination with a matrix to form a construct. In certain cases, the bioactive cell formulation is made up of a biocompatible material or biomaterial and a genetically-modified SRC population described herein.

In certain embodiments, the bioactive cell formulation is an injectable product composed of SRC genetically modified to reduce immunogenicity and formulated in a biomaterial (*e.g*. gelatin-based hydrogel). In an aspect, allogeneic SRC are obtained from isolation and expansion of renal cells from a donor patient's renal cortical tissue via a kidney biopsy, genetic modification of the SRC using gene editing techniques and selection by density gradient centrifugation from the expanded renal cells. SRC are composed primarily of renal epithelial cells which are well known for their regenerative potential (Humphreys et al. (2008) Intrinsic epithelial cells repair the kidney after injury. Cell Stem Cell. 2(3):284-91). Other parenchymal (vascular) and stromal (collecting duct) cells may be sparsely present in the SRC population. Injection of SRC into recipient kidneys has resulted in significant improvement in animal survival, urine concentration and filtration functions in nonclinical studies. However, SRC have limited shelf life and stability. Formulation of SRC in a gelatin-based hydrogel biomaterial provides enhanced stability of the cells thus extending product shelf life, improved stability during transport and delivery into the kidney cortex for clinical utility.

In an aspect, bioactive cell formulations are manufactured by first obtaining renal cortical tissue from the donor using a standard-of-clinical-care kidney biopsy procedure. Renal cells are isolated from the kidney tissue by enzymatic digestion and expanded using standard cell culture techniques. Cell culture medium is designed to expand primary renal cells and does not contain any differentiation factors. Harvested renal cells are subjected to density gradient separation to obtain SRC. The use of gene editing techniques to modify the immunogenicity of the SRC may be carried out either before or after the density gradient separation.

In certain embodiments, the formulated cell population is substantially free to move throughout the volume of the biomaterial at about ambient temperature or above. Having the cell population suspended in the substantially solid phase at a lower temperature provides stability advantages for the cells, such as for anchorage-dependent cells, as compared to cells in a fluid. Moreover, having cells suspended in the substantially solid state provides one or more of the following benefits: i) prevents settling of the cells, ii) allows the cells to remain anchored to the biomaterial in a suspended state; iii) allows the cells to remain more uniformly dispersed throughout the volume of the biomaterial; iv) prevents the formation of cell aggregates; and v) provides better protection for the cells during storage and transportation of the formulation. A formulation that can retain such features leading up to the administration to a subject is advantageous at least because the overall health of the cells in the formulation will be better and a more uniform and consistent dosage of cells will be administered.

In a preferred embodiment, the gelatin-based hydrogel biomaterial used to formulate SRC is a porcine gelatin dissolved in buffer to form a thermally responsive hydrogel. This hydrogel is fluid at room temperature but gels when cooled to refrigerated temperature (2-8°C). SRC are formulated with the hydrogel, gelled by cooling and shipped to the clinic under refrigerated temperature (2-8°C). At the clinical site, the product is warmed to room temperature before injecting into the patient's kidney. The bioactive cell formulation is implanted into the kidney cortex using a needle and syringe suitable for delivery via a percutaneous or laparoscopic procedure.

**Description and Composition of Exemplary Neo-Kidney Augment Composition**

In certain embodiments, the bioactive cell formulation is a Neo-Kidney Augment (NKA), which is an injectable product composed of autologous, selected renal cells (SRC) formulated in a Biomaterial (gelatin-based hydrogel). In an aspect, autologous SRC are obtained from isolation and expansion of renal cells from the patient's renal cortical tissue via a kidney biopsy and selection by centrifugation of the expanded renal cells across a density boundary, barrier, or interface. In certain embodiments, autologous SRC are obtained from isolation and expansion of renal cells from the patient's renal cortical tissue via a kidney biopsy and selection of the expanded renal cells over a continuous or discontinuous single step or multistep density gradient. SRC are composed primarily of renal tubular epithelial cells which are well known for their regenerative potential (Humphreys et al. (2008) Intrinsic epithelial cells repair the kidney after injury. Cell Stem Cell. 2(3):284-91). Other parenchymal (vascular) and stromal (collecting duct) cells may be sparsely present in the autologous SRC population. Injection of SRC into recipient kidneys has resulted in significant improvement in animal survival, urine concentration and filtration functions in preclinical studies. However, SRC have limited shelf life and stability. Formulation of SRC in a gelatin-based hydrogel biomaterial provides enhanced stability of the cells thus extending product shelf life, improved stability of NKA during transport and delivery of NKA into the kidney cortex for clinical utility.

In an aspect, NKA is manufactured by first obtaining renal cortical tissue from the donor/recipient using a standard-of-clinical-care kidney biopsy procedure. In certain embodiments, tenal cells are isolated from the kidney tissue by enzymatic digestion and expanded using standard cell culture techniques. In certain embodiments, a cell culture medium is designed to expand primary renal cells and does not contain any differentiation factors. In certain embodiments, harvested renal cells are subjected to separation across a density boundary or interface or density gradient to obtain SRCs. In certain embodiments, the SRCs are genetically modified in accordance with the present disclosure.

Included herein is a formulation made up of biomaterials designed or adapted to respond to external conditions as described herein. As a result, the nature of the association of the bioactive cell population with the biomaterial in a construct will change depending upon the external conditions. For example, a cell population's association with a temperature-sensitive biomaterial varies with temperature. In certain embodiments, the construct contains a bioactive renal cell population and biomaterial having a substantially solid state at about 8°C or lower and a substantially liquid state at about ambient temperature or above, wherein the cell population is suspended in the biomaterial at about 8°C or lower. However, the cell population is substantially free to move throughout the volume of the biomaterial at about ambient temperature or above. Having the cell population suspended in the substantially solid phase at a lower temperature provides stability advantages for the cells, such as for anchorage-dependent cells, as compared to cells in a fluid. Moreover, having cells suspended in the substantially solid state provides one or more of the following benefits: i) prevents settling of the cells, ii) allows the cells to remain anchored to the biomaterial in a suspended state; iii) allows the cells to remain more uniformly dispersed throughout the volume of the biomaterial; iv) prevents the formation of cell aggregates; and v) provides better protection for the cells during storage and transportation of the formulation. A formulation that can retain such features leading up to the administration to a subject is advantageous at least because the overall health of the cells in the formulation will be better and a more uniform and consistent dosage of cells will be administered.

In certain embodiments, the gelatin-based hydrogel biomaterial used to formulate SRC into NKA is a porcine gelatin dissolved in buffer to form a thermally responsive hydrogel. This hydrogel is fluid at room temperature but gels when cooled to refrigerated temperature (2-8°C). SRC are formulated with the hydrogel to obtain NKA. NKA is gelled by cooling and is shipped to the clinic under refrigerated temperature (2-8°C). NKA has a shelf life of 3 days. At the clinical site, the product is warmed to room temperature before injecting into the patient's kidney. NKA is implanted into the kidney cortex using a needle and syringe suitable for delivery of NKA via a percutaneous or laparoscopic procedure. In certain embodiments, the hydrogel is derived from gelatin or another extracellular matrix protein of recombinant origin. In certain embodiments, the hydrogel is derived from extracellular matrix sourced from kidney or another tissue or organ. In certain embodiments, the hydrogel is derived from a recombinant extracellular matrix protein. In certain embodiments, the hydrogel comprises gelatin derived from recombinant collagen (*i.e.,* recombinant gelatin).

### Cell Viability Agents

In an aspect, the bioactive cell formulation also includes a cell viability agent. In certain cases the cell viability agent is selected from the group consisting of an antioxidant, an oxygen carrier, an immunomodulatory factor, a cell recruitment factor, a cell attachment factor, an anti-inflammatory agent, an angiogenic factor, a matrix metalloprotease, a wound healing factor, and products secreted from bioactive cells.

Antioxidants are characterized by the ability to inhibit oxidation of other molecules. Antioxidants include, without limitation, one or more of 6-hydroxy-2,5,7,8-tetramethylchroman-2-carboxylic acid (Trolox^{®}), carotenoids, flavonoids, isoflavones, ubiquinone, glutathione, lipoic acid, superoxide dismutase, ascorbic acid, vitamin E, vitamin A, mixed carotenoids (e.g., beta carotene, alpha carotene, gamma carotene, lutein, lycopene, phytopene, phytofluene, and astaxanthin), selenium, Coenzyme Q10, indole-3-carbinol, proanthocyanidins, resveratrol, quercetin, catechins, salicylic acid, curcumin, bilirubin, oxalic acid, phytic acid, lipoic acid, vanilic acid, polyphenols, ferulic acid, theaflavins, and derivatives thereof. Those of ordinary skill in the art will appreciate other suitable antioxidants may be used.

Oxygen carriers are agents characterized by the ability to carry and release oxygen. They include, without limitation, perfluorocarbons and pharmaceuticals containing perfluorocarbons. Suitable perfluorocarbon-based oxygen carriers include, without limitation, perfluorooctyl bromide (C8F17Br); perfluorodichorotane (C8F16C12); perfluorodecyl bromide; perfluobron; perfluorodecalin; perfluorotripopylamine; perfluoromethylcyclopiperidine; Fluosol^{®} (perfluorodecalin & perfluorotripopylamine); Perftoran^{®} (perfluorodecalin & perfluoromethylcyclopiperidine); Oxygent^{®} (perfluorodecyl bromide & perfluobron); Ocycyte^{™} (perfluoro (tert-butylcyclohexane)). Those of ordinary skill in the art will appreciate other suitable perfluorocarbon-based oxygen carriers may be used.

Immunomodulatory factors include, without limitation, osteopontin, FAS Ligand factors, interleukins, transforming growth factor beta, platelet derived growth factor, clusterin, transferrin, regulated upon action, normal T-cell expressed, secreted protein (RANTES), plasminogen activator inhibitor - 1 (Pai-1), tumor necrosis factor alpha (TNF-alpha), interleukin 6 (IL-6), alpha-1 microglobulin, and beta-2-microglobulin. Those of ordinary skill in the art will appreciate other suitable immunomodulatory factors may be used.

Anti-inflammatory agents or immunosuppressant agents (described below) may also be part of the formulation. Those of ordinary skill in the art will appreciate other suitable antioxidants may be used.

Cell recruitment factors include, without limitation, monocyte chemotatic protein 1 (MCP-1), and CXCL-1. Those of ordinary skill in the art will appreciate other suitable cell recruitment factors may be used.

Cell attachment factors include, without limitation, fibronectin, procollagen, collagen, ICAM-1, connective tissue growth factor, laminins, proteoglycans, specific cell adhesion peptides such as RGD and YSIGR. Those of ordinary skill in the art will appreciate other suitable cell attachment factors may be used.

Angiogenic factors include, without limitation, vascular endothelial growth factor F (VEGF) and angiopoietin-2 (ANG-2). Those of ordinary skill in the art will appreciate other suitable angiogenic factors may be used.

Matrix metalloproteases include, without limitation, matrix metalloprotease 1 (MMP1), matrix metalloprotease 2 (MMP2), matrix metalloprotease 9 (MMP-9), and tissue inhibitor and matalloproteases - 1 (TIMP-1).

Wound healing factors include, without limitation, keratinocyte growth factor 1 (KGF-1), tissue plasminogen activator (tPA), calbindin, clusterin, cystatin C, trefoil factor 3. Those of ordinary skill in the art will appreciate other suitable wound healing factors may be used.

Secreted products from bioactive cells described herein may also be added to the bioactive cell formulation as a cell viability agent.

Compositions sourced from body fluids, tissue or organs from human or animal sources, including, without limitation, human plasma, human platelet lysate, bovine fetal plasma or bovine pituitary extract, may also be added to the bioactive cell formulations as a cell viability agent.

Those of ordinary skill in the art will appreciate there are several suitable methods for depositing or otherwise combining cell populations with biomaterials to form a construct.

### Methods of Use

In an aspect, the cells obtainable via the methods of the present invention are suitable for use in the methods of use described herein. In certain embodiments, the formulations of the present invention may be administered for the treatment of disease. For example, genetically modified bioactive cells may be administered to a native organ as part of a formulation described herein. In certain embodiments, the genetically modified bioactive cells may be sourced from the native organ that is the subject of the administration or from a source that is not the target native organ.

The present disclosure provides methods for the treatment of a kidney disease, in a subject in need with the formulations containing bioactive renal cell populations as described herein. In certain cases the therapeutic formulation contains a selected renal cell population or admixtures thereof modified by gene editing to reduce immunogenicity. In certain cases, the formulations are suitable for administration to a subject in need of improved kidney function.

In an aspect, the effective treatment of a kidney disease in a subject by use of cells obtainable via the methods of the present disclosure can be observed through various indicators of kidney function. In certain cases, the indicators of kidney function include, without limitation, serum albumin, albumin to globulin ratio (A/G ratio), serum phosphorous, serum sodium, kidney size (measurable by ultrasound), serum calcium, phosphorous:calcium ratio, serum potassium, proteinuria, urine creatinine, serum creatinine, blood nitrogen urea (BUN), cholesterol levels, triglyceride levels and glomerular filtration rate (GFR). Furthermore, several indicators of general health and well-being include, without limitation, weight gain or loss, survival, blood pressure (mean systemic blood pressure, diastolic blood pressure, or systolic blood pressure), and physical endurance performance.

In an aspect, an effective treatment with a bioactive renal cell formulation is evidenced by stabilization of one or more indicators of kidney function. The stabilization of kidney function is demonstrated by the observation of a change in an indicator in a subject treated by a method of the present disclosure as compared to the same indicator in a subject that has not been treated by a method of the present disclosure. Alternatively, the stabilization of kidney function may be demonstrated by the observation of a change in an indicator in a subject treated by a method of the present disclosure as compared to the same indicator in the same subject prior to treatment. The change in the first indicator may be an increase or a decrease in value. In certain cases, the treatment provided by the present disclosure may include stabilization of serum creatinine and/or blood urea nitrogen (BUN) levels in a subject where the BUN levels observed in the subject are lower as compared to a subject with a similar disease state who has not been treated by the methods of the present disclosure. In certain cases, the treatment may include stabilization of serum creatinine levels in a subject where the serum creatinine levels observed in the subject are lower as compared to a subject with a similar disease state who has not been treated by the methods of the present disclosure. In certain cases, the stabilization of one or more of the above indicators of kidney function is the result of treatment with a selected renal cell formulation modified by gene editing to reduce immunogenicity.

Those of ordinary skill in the art will appreciate that one or more additional indicators described herein or known in the art may be measured to determine the effective treatment of a kidney disease in the subject.

In certain cases an effective treatment with a genetically modified bioactive renal cell formulation is evidenced by improvement of one or more indicators of kidney structure and/or function. In certain cases, the genetically modified bioactive renal cell population provides an improved level of serum creatinine and/or blood urea nitrogen (BUN). In certain cases, the genetically modified bioactive renal cell population provides an improved retention of protein in the serum. In certain cases, the genetically modified bioactive renal cell population provides improved levels of serum albumin as compared to the non-enriched cell population. In certain embodiments, the genetically modified bioactive renal cell population provides improved A:G ratio as compared to the non-enriched cell population. In certain cases, the genetically modified bioactive renal cell population provides improved levels of serum cholesterol and/or triglycerides. In certain cases, the genetically modified bioactive renal cell population provides an improved level of Vitamin D. In certain cases, the genetically modified bioactive renal cell population provides an improved phosphorus:calcium ratio as compared to a non-enriched cell population. In certain cases, the genetically modified bioactive renal cell population provides an improved level of hemoglobin as compared to a non-enriched cell population. In certain cases, the genetically modified bioactive renal cell population provides an improved level of serum creatinine as compared to a non-enriched cell population. In certain cases, the genetically modified bioactive renal cell population provides an improved level of hematocrit as compared to a non-enriched cell population. In certain cases, the improvement of one or more of the above indicators of kidney function is the result of treatment with a selected renal cell formulation. In an cases, the improvement of one or more of the above indicators of kidney function is the result of treatment with a selected renal cell formulation modified by gene editing to reduce immunogenicity.

In an aspect, the present disclosure provides formulations for use in methods for the regeneration of a native kidney in a subject in need thereof. In certain cases, the method includes the step of administering or implanting agenetically modified bioactive cell population, admixture, or construct described herein to the subject. A regenerated native kidney may be characterized by a number of indicators including, without limitation, development of function or capacity in the native kidney, improvement of function or capacity in the native kidney, and the expression of certain markers in the native kidney. In certain cases, the developed or improved function or capacity may be observed based on the various indicators of kidney function described above. In certain cases, the regenerated kidney is characterized by differential expression of one or more stem cell markers. The stem cell marker may be one or more of the following: SRY (sex determining region Y)-box 2 (Sox2); Undifferentiated Embryonic Cell Transcription Factor (UTF1); Nodal Homolog from Mouse (NODAL); Prominin 1 (PROM1) or CD133 (CD133); CD24; and any combination thereof (see Ilagan et al. PCT/US2011/036347), see also Genheimer et al., 2012. Molecular characterization of the regenerative response induced by intrarenal transplantation of selected renal cells in a rodent model of chronic kidney disease. Cells Tissue Organs 196: 374-384.

In certain cases, the expression of the stem cell marker(s) is up-regulated compared to a control. Also described is method of treating kidney disease in a subject, the method comprising injecting a formulation, composition, or cell population disclosed herein into the subject. In certain cases, the formulation, composition, for cell population is injected through a 18 to 30 gauge needle. In certain cases, the formulation, composition, for cell population is injected through a needle that is smaller than 20 gauge. In certain cases, the formulation, composition, for cell population is injected through a needle that is smaller than 21 gauge. In certain cases, the formulation, composition, for cell population is injected through a needle that is smaller than 22 gauge. In certain cases, the formulation, composition, for cell population is injected through a needle that is smaller than 23 gauge. In certain cases, the formulation, composition, for cell population is injected through a needle that is smaller than 24 gauge. In certain cases, the formulation, composition, for cell population is injected through a needle that is smaller than 25 gauge. In certain cases, the formulation, composition, for cell population is injected through a needle that is smaller than 26 gauge. In certain cases, the formulation, composition, for cell population is injected through a needle that is smaller than 27 gauge. In certain cases, the formulation, composition, for cell population is injected through a needle that is smaller than 28 gauge. In certain cases, the formulation, composition, for cell population is injected through a needle that is smaller than 29 gauge. In certain cases, the formulation, composition, for cell population is injected through a needle that is about 20 gauge. In certain cases, the formulation, composition, for cell population is injected through a needle that is about 21 gauge.

In certain cases, the formulation, composition, for cell population is injected through a needle that is about 22 gauge. In certain cases, the formulation, composition, for cell population is injected through a needle that is about 23 gauge. In certain cases, the formulation, composition, for cell population is injected through a needle that is about 24 gauge. In certain cases, the formulation, composition, for cell population is injected through a needle that is about 25 gauge. In certain cases, the formulation, composition, for cell population is injected through a needle that is about 26 gauge. In certain cases, the formulation, composition, for cell population is injected through a needle that is about 27 gauge. In certain cases, the formulation, composition, for cell population is injected through a needle that is about 28 gauge. In certain cases, the formulation, composition, for cell population is injected through a needle that is about 29 gauge.

In certain cases, the inter diameter of the needle is less than 0.84 mm. In certain cases, the inter diameter of the needle is less than 0.61 mm. In certain cases, the inter diameter of the needle is less than 0.51 mm. In certain cases, the inter diameter of the needle is less than 0.41 mm. In certain cases, the inter diameter of the needle is less than 0.33 mm. In certain cases, the inter diameter of the needle is less than 0.25 mm. In certain cases, the inter diameter of the needle is less than 0.20 mm. In certain cases, the inter diameter of the needle is less than 0.15 mm. In certain cases, the outer diameter of the needle is less than 1.27 mm. In certain cases, the outer diameter of the needle is less than 0.91 mm. In certain cases, the outer diameter of the needle is less than 0.81 mm. In certain cases, the outer diameter of the needle is less than 0.71 mm. In certain cases, the outer diameter of the needle is less than 0.64 mm. In certain cases, the outer diameter of the needle is less than 0.51 mm. In certain cases, the outer diameter of the needle is less than 0.41 mm. In certain cases, the outer diameter of the needle is less than 0.30 mm. In cetain cases, a needle has one of the sizes in the following table:

| | ID Size | | OD Size | |
|---|---|---|---|---|
| Gauge | in | mm | in | mm |
| 14 | 0.060 | 1.55 | 0.072 | 1-83 |
| 15 | 0.054 | 1.37 | 0.065 | 1.65 |
| 16 | 0.047 | 1.19 | n/a | n/a |
| 18 | 0.033 | 0.84 | 0.050 | 1.27 |
| 20 | 0.023 | 0.61 | 0.036 | 0.91 |
| 21 | 0.020 | 0.51 | 0.032 | 0.81 |
| 22 | 0.016 | 0.41 | 0.028 | 0.71 |
| 23 | 0.013 | 0.33 | 0.025 | 0.64 |
| 25 | 0.010 | 0.25 | 0.020 | 0.51 |
| *27* | 0.008 | 0.20 | 0.016 | 0.41 |
| 30 | 0.006 | 0.15 | 0.012 | 0.30 |
| 32 | 0.004 | 0.10 | 0.009 | 0.23 |

### Secreted Products

In certain cases, the effect may be provided by the cells themselves and/or by products secreted from the cells. The regenerative effect may be characterized by one or more of the following: a reduction in epithelial-mesenchymal transition (which may be via attenuation of TGF-β signaling); a reduction in renal fibrosis; a reduction in renal inflammation; differential expression of a stem cell marker in the native kidney; migration of implanted cells and/or native cells to a site of renal injury, e.g., tubular injury, engraftment of implanted cells at a site of renal injury, e.g., tubular injury; stabilization of one or more indicators of kidney function (as described herein); de novo formation of S-shaped bodies/comma-shaped bodies associated with nephrogenesis, de novo formation of renal tubules or nephrons, restoration of erythroid homeostasis (as described herein); and any combination thereof (*see also* Basu et al., 2011. Functional evaluation of primary renal cell/biomaterial neo-kidney augment prototypes for renal tissue engineering. Cell Transplantation 20: 1771-90; Bruce et al., 2015. Selected renal cells modulate disease progression in rodent models of chronic kidney disease via NF-κB and TGF-β1 pathways. Regenerative Medicine 10: 815-839).

As an alternative to a tissue biopsy, a regenerative outcome in the subject receiving treatment can be assessed from examination of a bodily fluid, e.g., urine. It has been discovered that microvesicles obtained from subject-derived urine sources contain certain components including, without limitation, specific proteins and miRNAs that are ultimately derived from the renal cell populations impacted by treatment with the cell populations of the present disclosure. These components may include, without limitation, factors involved in stem cell replication and differentiation, apoptosis, inflammation and immuno-modulation, fibrosis, epithelial-mesenchymal transition, TGF-β signaling, and PAI-1 signaling. A temporal analysis of microvesicle-associated miRNA/protein expression patterns allows for continuous monitoring of regenerative outcomes within the kidney of subjects receiving the cell populations, admixtures, or constructs of the present disclosure.

The present disclosure provides methods of assessing whether a kidney disease (KD) patient is responsive to treatment with a therapeutic formulation. The method may include the step of determining or detecting the amount of vesicles or their luminal contents in a test sample obtained from a KD patient treated with the therapeutic, as compared to or relative to the amount of vesicles in a control sample (*e.g.,* a samplel derived from the same patient prior to treatment with the therapeutic), wherein a higher or lower amount of vesicles or their luminal contents in the test sample as compared to the amount of vesicles or their luminal contents in the control sample is indicative of the treated patient's responsiveness to treatment with the therapeutic.

These kidney-derived vesicles and/or the luminal contents of kidney derived vesicles may also be shed into the urine of a subject and may be analyzed for biomarkers indicative of regenerative outcome or treatment efficacy. The non-invasive prognostic methods may include the step of obtaining a urine sample from the subject before and/or after administration or implantation of a genetically modified bioactive renal cell population, admixture, or construct described herein. Vesicles and other secreted products may be isolated from the urine samples using standard techniques including without limitation, centrifugation to remove unwanted debris (Zhou et al. 2008. Kidney Int. 74(5):613-621; Skog et al. U.S. Published Patent Application No. 20110053157) precipitation to separate exosomes from urine, polymerase chain reaction and nucleic acid sequencing to identify specific nucleic acids and mass spectroscopy and/or 2D gel electrophoresis to identify specific proteins associated with regenerative outcomes.

### Methods and Routes of Administration

The bioactive cell formulations obtainable via the methods of the present invention can be administered alone or in combination with other bioactive components. The formulations are suitable for injection or implantation of incorporated tissue engineering elements to the interior of solid organs to regenerate tissue. In addition, the formulations are used for the injection or implantation of tissue engineering elements to the wall of hollow organs to regenerate tissue.

Described herein are methods of providing a bioactive cell formulation described herein to a subject in need. In certain cases, the source of the bioactive cell may be allogeneic or syngeneic, and any combination thereof. In certain cases, the methods may include the administration of an immunosuppressant agent. (see e.g. U.S. Patent No. 7,563,822).

The treatment methods of the disclosure involve the delivery of a bioactive cell formulation described herein. In certain cases, direct administration of cells to the site of intended benefit is preferred. A subject in need may also be treated by in vivo contacting of a native kidney with a bioactive cell formulation described herein together with products secreted from one or more enriched renal cell populations, and/or an admixture or construct containing the same. The step of *in vivo* contacting provides a regenerative effect to the native kidney.

A variety of means for administering compositions of selected renal cells to subjects will, in view of this specification, be apparent to those of skill in the art. Such methods include injection of the cells into a target site in a subject.

### Delivery Vehicles

Cells and/or secreted products can be inserted into a delivery device or vehicle, which facilitates introduction by injection or implantation into the subjects. In certain cases, the delivery vehicle can include natural materials. In certain other cases, the delivery vehicle can include synthetic materials. In certain cases, the delivery vehicle provides a structure to mimic or appropriately fit into the organ's architecture. In certain cases, the delivery vehicle is fluid-like in nature. Such delivery devices can include tubes, e.g., catheters, for injecting cells and fluids into the body of a recipient subject. In a preferred case, the tubes additionally have a needle, e.g., a syringe, through which the cells of the invention can be introduced into the subject at a desired location. In certain cases, mammalian kidney-derived cell populations are formulated for administration into a blood vessel via a catheter (where the term "catheter" is intended to include any of the various tube-like systems for delivery of substances to a blood vessel). Alternatively, the cells can be inserted into or onto a biomaterial or scaffold, including but not limited to textiles, such as weaves, knits, braids, meshes, and non-wovens, perforated films, sponges and foams, and beads, such as solid or porous beads, microparticles, nanoparticles, and the like (e.g., Cultispher-S gelatin beads - Sigma). The cells can be prepared for delivery in a variety of different forms. For example, the cells can be suspended in a solution or gel. Cells can be mixed with a pharmaceutically acceptable carrier or diluent in which the cells of the invention remain viable. Pharmaceutically acceptable carriers and diluents include saline, aqueous buffer solutions, solvents and/or dispersion media. The use of such carriers and diluents is well known in the art. The solution is preferably sterile and fluid, and will often be isotonic. Preferably, the solution is stable under the conditions of manufacture and storage and preserved against the contaminating action of microorganisms such as bacteria and fungi through the use of, for example, parabens, chlorobutanol, phenol, ascorbic acid, thimerosal, and the like. One of skill in the art will appreciate that the delivery vehicle used in the delivery of the cell populations and admixtures thereof of the instant invention can include combinations of the above-mentioned characteristics.

### Modes of Administration

Modes of administration of the formulations include, but are not limited to, systemic, intra-renal (e.g., parenchymal), intravenous or intra-arterial injection and injection directly into the tissue at the intended site of activity. Additional modes of administration to be used in accordance with the disclosure include single or multiple injection(s) via direct laparotomy, via direct laparoscopy, transabdominal, or percutaneous. Still yet additional modes of administration to be used in accordance with the present invention include, for example, retrograde and ureteropelvic infusion. Surgical means of administration include one-step procedures such as, but not limited to, partial nephrectomy and construct implantation, partial nephrectomy, partial pyelectomy, vascularization with omentum ± peritoneum, multifocal biopsy needle tracks, cone or pyramidal, to cylinder, and renal pole-like replacement, as well as two-step procedures including, for example, organoid-internal bioreactor for replanting. In certain cases, the formulations containing admixtures of cells are delivered via the same route at the same time. In certain cases, each of the cell compositions comprising the controlled admixture are delivered separately to specific locations or via specific methodologies, either simultaneously or in a temporally-controlled manner, by one or more of the methods described herein. In certain cases, the selected renal cells are percutaneously injected into the renal cortex of a kidney. In certain cases, a guiding cannula is inserted percutaneously and used to puncture the kidney capsule prior to injection of the composition into the kidney.

A laparoscopic or percutaneous technique may be used to access the kidney for injection of formulated BRC or SRC population. Use of laparoscopic surgical techniques allows for direct visualization of the kidney so that any bleeding or other adverse events can be spotted during injection and addressed immediately. Use of a percutaneous approach to the kidney has been in use for over a decade, primarily for ablating intrarenal masses. These procedures insert an electrode or cryogenic needle into a defined mass in the kidney, and remain in contact for (typically) 10 to 20 minutes while the lesion is ablated. For injection of the therapeutic formulation, the percutaneous instrumentation is no larger nor more complex, and this approach offers the safety advantages of no surgery (avoiding abdominal puncture wounds and inflation with gas) and minimal immobilization time. Furthermore, the access track can have hemostatic biodegradable material left in place, to further reduce any chance of significant bleeding.

According to one case of the delivery by injection, the therapeutic bioactive cell formulation is injected into the renal cortex. It is important to distribute the therapeutic formulation in the renal cortex as widely as possible, which can be achieved, for example, by entering the renal cortex at an angle allowing deposition of the therapeutic formulation in the renal cortex, distributed as widely as feasible. This could require imaging the kidney in a longitudinal or transverse approach using ultrasound guidance or with axial computed tomography (CT) imaging, depending upon individual patient characteristics. Ideally the injection will involve multiple deposits as the injection needle/cannula is gradually withdrawn. The full volume of the therapeutic formulation may be deposited at a single or multiple entry points. In certain cases, up to two entry points may be used to deposit the full volume of therapeutic formulation into the kidney. In certain cases, the injection may be administered to a single kidney, using one or more entry points, e.g. one or two entry points. In certain cases, the injection is made into both kidneys, in each kidney using one or more entry point, e.g. one or two entry points.

### EXAMPLES

### Example 1: Non-limiting Examples of Methods and Compositions for Producing SRCs

### Example 1.1 - Preparation of Solutions

This example section provides the compositions of the various media formulations and solutions used for the isolation and characterization of the heterogeneous renal cell population, and manufacture of the regenerative therapy product, in this example.

**Table 6: Culture Media and Solutions**

| **Material** | **Composition** | |
|---|---|---|
| Tissue Transport Medium | • Viaspan^{™} or HypoThermosol-FRS^{®} or DMEM | |
| | • Kanamycin: 100 µg/mL | |
| Renal Cell Growth Medium | • DMEM:KSFM (50:50) | |
| | • 5% FBS | |
| | • Growth Supplements: | |
| | | • HGF: 10 mg/L |
| | | • EGF: 2.5 µg/L |
| | | • Insulin: 10.0 mg/L, |
| | | • Transferrin: 5.5 mg/L |
| | | • Selenium: 670 µg/L |
| | | • Kanamycin: 100 µg/mL |
| Tissue Wash Solution | • DMEM | |
| | • Kanamycin: 100 µg/mL | |
| Digestion Solution | • Collagenase IV: 300 Units | |
| | • Dispase: 5 mg/mL | |
| | • Calcium Chloride: 5 mM | |
| Cell Dissociation Solution | • TrypLE^{™} | |
| Density Gradient Solution | • 7% OptiPrep | |
| | • OptiMEM | |
| Cryopreservation Solution | • DMEM or HypoThermosol-FRS | |
| | • 10% DMSO | |
| | • 10% FBS | |

Dulbecco's Phosphate Buffered Saline (DPBS) was used for all cell washes.

### Example 1.2 - Isolation of the Heterogeneous Unfractionated Renal Cell Population

This example section illustrates the isolation of an unfractionated (UNFX) heterogeneous renal cell population from human. Initial tissue dissociation was performed to generate heterogeneous cell suspensions from human kidney tissue.

Renal tissue via kidney biopsy provided the source material for a heterogeneous renal cell population. Renal tissue comprising one or more of cortical, corticomedullary junction or medullary tissue may be used. It is preferred that the corticomedullary junction tissue is used. Multiple biopsy cores (minimum 2), avoiding scar tissue, were required from a CKD kidney. Renal tissue was obtained by the clinical investigator from the patient at the clinical site approximately 4 weeks in advance of planned implantation of the final NKA. The tissue was transported in the Tissue Transport Medium of Example 1.1.

The tissue was then washed with Tissue Wash Solution of Example 1.1 in order to reduce incoming bioburden before processing the tissue for cell extractions.

Renal tissue was minced, weighed, and dissociated in the Digestion Solution of Example 1.1. The resulting cell suspension was neutralized in Dulbecco's Modified Eagle Medium (D-MEM)+10% fetal bovine serum (FBS) (Invitrogen, Carlsbad Calif.), washed, and resuspended in serum-free, supplement-free, Keratinocyte Media (KSFM) (Invitrogen). Cell suspensions were then centrifuged over a 15% (w/v) iodixanol (OptiPrep^{™}, Sigma) density boundary to remove red blood cells and debris prior to initiation of culture onto tissue culture treated polystyrene flasks or dishes at a density of 25,000 cells per cm² in Renal Cell Growth Medium of Example 1.1. For example, cells may be plated onto T500 Nunc flask at 25×10⁶ cells/flask in 150 ml of 50:50 media.

### Example 1.3 - Cell Expansion of the Isolated Renal Cell Population

Renal cell expansion is dependent on the amount of tissue received and on the success of isolating renal cells from the incoming tissue. Isolated cells can be cryopreserved, if required (*see* infra). Renal cell growth kinetics may vary from sample to sample due to the inherent variability of cells isolated from individual patients.

A defined cell expansion process was developed that accommodates the range of cell recoveries resulting from the variability of incoming tissue Table 7. Expansion of renal cells involves serial passages in closed culture vessels (*e.g*., T-flasks, Cell Factories, HyperStacks^{®}) in Renal Cell Growth Medium Table 6 using defined cell culture procedures.

A BPE-free medium was developed for human clinical trials to eliminate the inherent risks associated with the use of BPE. Cell growth, phenotype (CK18) and cell function (GGT and LAP enzymatic activity) were evaluated in BPE-free medium and compared to BPE containing medium used in the animal studies. Renal cell growth, phenotype and function were equivalent in the two media. (data not shown)

**Table 7 Cell Recovery from Human Kidney Biopsies**

| **Source** | **Renal cells** (cells/10 mg tissue) | |
|---|---|---|
| | (passage 0) | (passage 1) |
| Human Kidney Tissue Samples (n=7) | 1.44 - 10.80 × 10⁶ | 4.61- 23.10 × 10⁷ |

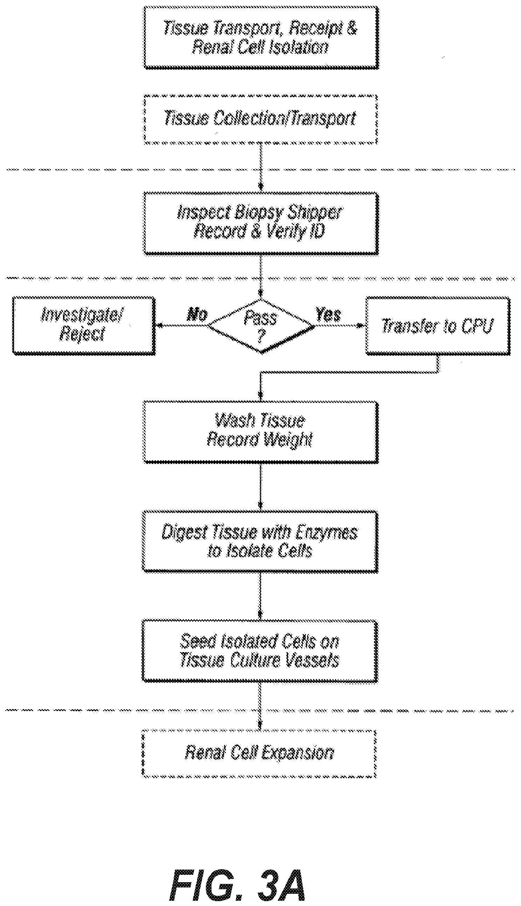

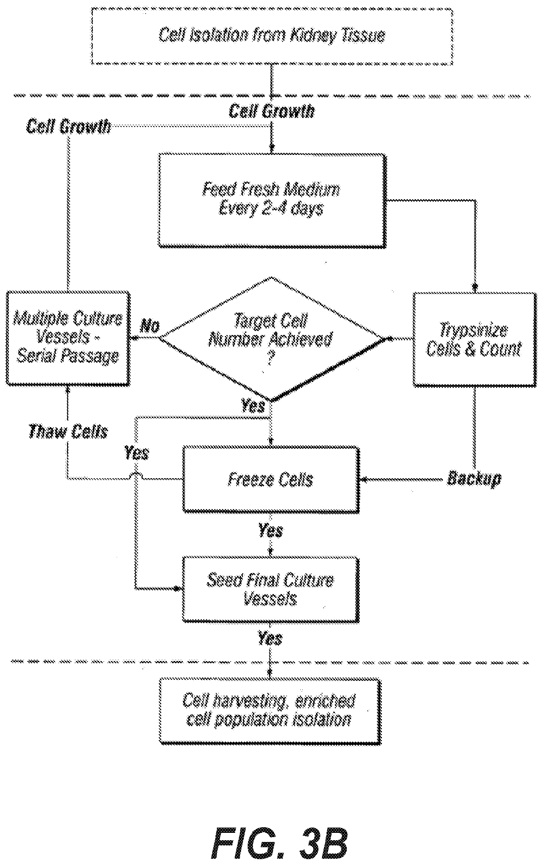

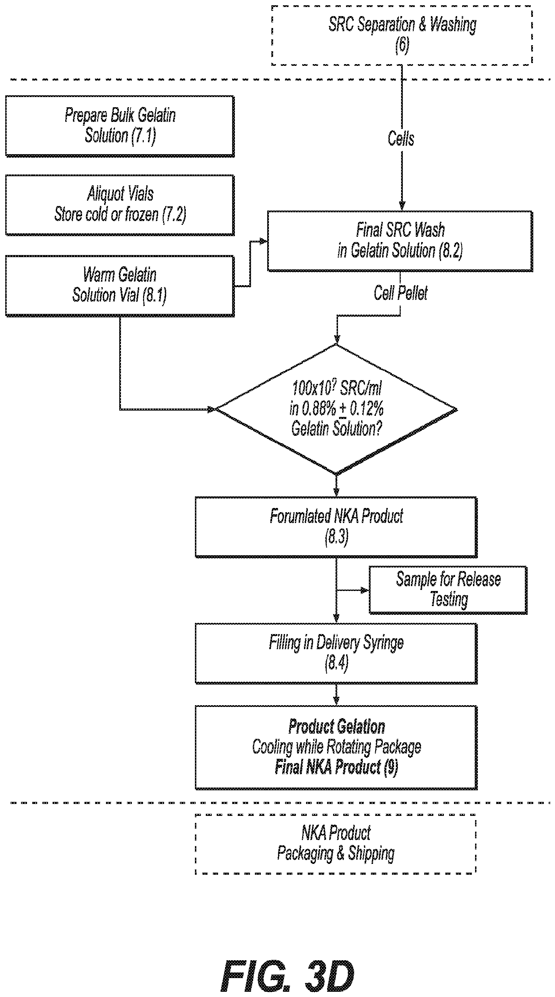

Once cell growth was observed in the initial T-flasks (passage 0) and there were no visual signs of contamination, culture medium was replaced and changed thereafter every 2-4 days (FIG. 3B). Cells were assessed to verify renal cell morphology by visual observation of cultures under the microscope. Cultures characteristically demonstrated a tight pavement or cobblestone appearance, due to the cells clustering together. These morphological characteristics vary during expansion and may not be present at every passage. Cell culture confluence was estimated at various levels of confluence in the culture vessels employed throughout cell expansions.

Renal cells were passaged by trypsinization when culture vessels are at least 50% confluent (FIG. 3B). Detached cells were collected into vessels containing Renal Cell Growth Medium, counted and cell viability calculated. At each cell passage, cells were seeded at 500-4000 cells/cm² in a sufficient number of culture vessels in order to expand the cell number to that required for formulation of NKA (FIG. 3B). Culture vessels were placed in a 37°C. incubator in a 5% CO₂ environment. As described above, cell morphology and confluence was monitored and tissue culture media was replaced every 2-4 days. Table 8 lists the viability of human renal cells observed during cell isolation and expansion of six kidney biopsies from human donors.

**Table 8 Cell Viability of Human Renal Cells in Culture**

| **Passage (n=6)** | **Cell Viability (Average %)** | **Range (%)** |
|---|---|---|
| P0 | 88 | 84-93 |
| P1 | 91 | 80-98 |
| P2 | 94 | 92-99 |
| P3 | 98 | 97-99 |

Inherent variability of tissue from different patients resulted in different cell yield in culture. Therefore, it is not practical to strictly define the timing of cell passages or number and type of culture vessels required at each passage to attain target cell numbers. Typically renal cells undergo 2 or 3 passages; however, duration of culture and cell yield can vary depending on the cell growth rate.

Cells were detached for harvest or passage with 0.25% Trypsin with EDTA (Invitrogen). Viability was assessed via Trypan Blue exclusion and enumeration was performed manually using a hemacytometer or using the automated Cellometer.RTM. counting system (Nexcelom Bioscience, Lawrence Mass.).

### Example 1.4 Cryopreservation of Cultured Cells

Expanded renal cells were routinely cryopreserved to accommodate for inherent variability of cell growth from individual patients and to deliver product on a pre-determined clinical schedule. Cryopreserved cells also provide a backup source of cells in the event that another NKA is needed (*e.g.,* delay due to patient sickness, unforeseen process events, *etc.*)*.* Conditions were established that have been used to cryopreserve cells and recover viable, functional cells upon thawing.

For cryopreservation, cells were suspended to a final concentration of about 50×10⁶ cells/mL in Cryopreservation Solution (see Example 1.1) and dispensed into vials. One ml vials containing about 50×10⁶ cells/mL were placed in the freezing chamber of a controlled rate freezer and frozen at a pre-programmed rate. After freezing, the cells were transferred to a liquid nitrogen freezer for in-process storage.

### Example 1.5 Preparation of SRC Cell Population

Selected Renal Cells (SRC) can be prepared from the final culture vessels that are grown from cryopreserved cells or directly from expansion cultures depending on scheduling (FIG. 3B).

If using cryopreserved cells, the cells were thawed and plated on tissue culture vessels for one final expansion step. When the final culture vessels were approximately 50-100% confluent cells were ready for processing for SRC separation. Media exchanges and final washes of NKA dilute any residual Cryopreservation Solution in the final product.

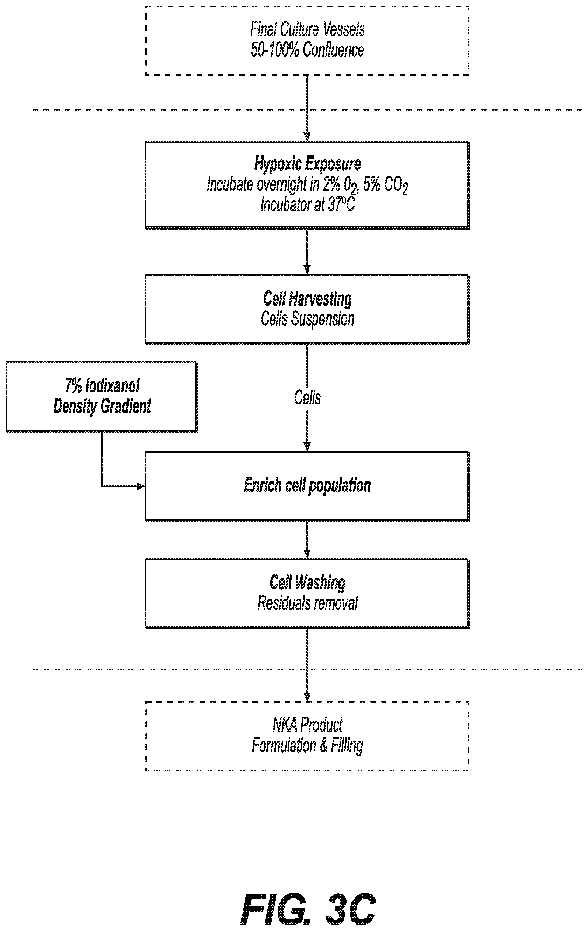

Once the final cell culture vessels have reached at least 50% confluence the culture vessels were transferred to a hypoxic incubator set for 2% oxygen in a 5% CO₂ environment at 37°C (FIG. 3C). and cultured overnight. Cells may be held in the oxygen-controlled incubator set to 2% oxygen for as long as 48 hours. Exposure to the more physiologically relevant low-oxygen (2%) environment improved cell separation efficiency and enabled greater detection of hypoxia-induced markers such as VEGF.

After the cells have been exposed to the hypoxic conditions for a sufficient time (*e.g.,* overnight to 48 hours), the cells were detached with 0.25% Trypsin with EDTA (Invitrogen). Viability was assessed via Trypan Blue exclusion and enumeration was performed manually using a hemacytometer or using the automated Cellometer^{®} counting system (Nexcelom Bioscience, Lawrence Mass.). Cells were washed once with DPBS and resuspended to about 850×10⁶ cells/mL in DPBS.

Centrifugation across a density boundary/interface was used to separate harvested renal cell populations based on cell buoyant density. Renal cell suspensions were separated by centrifugation over a 7% iodixanol Solution (OptiPrep; 60% (w/v) in OptiMEM; see Example 1.1).

The 7% OptiPrep density interface solution was prepared and refractive index indicative of desired density was measured (R.I. 1.3456+/-0.0004) prior to use. Harvested renal cells were layered on top of the solution. The density interface was centrifuged at 800 g for 20 min at room temperature (without brake) in either centrifuge tubes or a cell processor (e.g., COBE 2991). The cellular fraction exhibiting buoyant density greater than approximately 1.045 g/mL was collected after centrifugation as a distinct pellet. Cells maintaining a buoyant density of less than 1.045 g/mL were excluded and discarded.

The SRC pellet was re-suspended in DPBS (FIG. 3C). The carry-over of residual OptiPrep, FBS, culture medium and ancillary materials in the final product is minimized by 4 DPBS wash and 1 Gelatin Solution steps.

### Example 2: Preparation of Selected Renal Cells

Briefly, renal biopsies are dissociated enzymatically in a buffer containing 4.0 units/mL dispase (Stem Cell Technologies, Inc., Vancouver, BC, Canada) and 300 units/mL collagenase IV (Worthington Biochemical, Lakewood, NJ, USA). Red blood cells and debris are removed by centrifugation through 15% iodixanol (Optiprep^{®}, Axis Shield, Norton, MA, USA). Primary renal cells are seeded onto tissue culture treated polystyrene plates (NUNC, Rochester, NY, USA) and cultured in 50:50 media, a 1:1 mixture of high glucose Dulbecco's Modified Eagle Medium (DMEM): Keratinocyte Serum Free Medium (KSFM) containing 5% Fetal Bovine Serum (FBS), 1X ITS (insulin/transferrin/sodium selenite medium supplement), and antibiotic/antimycotic (all from Invitrogen, Carlsbad, CA, USA). Prior to post-culture cell separation, primary renal cell cultures are transferred from atmospheric oxygen conditions (21%) to a more physiologically relevant low-oxygen (2%) environment for 24 hours, to improve cell separation efficiency. Separation of primary renal cell cultures, prepared as 75 × 10⁶ cells in 2mL unsupplemented KSFM (uKSFM), is performed by centrifugation through a four-step iodixanol (OptiPrep; 60% w/v in uKSFM) density gradient (16%, 13%, 11%, and 7%) in 15 mL conical polypropylene tubes and centrifuged at 800g for 20 min at room temperature. After centrifugation all bands are washed 3 times with sterile phosphate buffered saline (PBS) prior to use.

In certain embodiments, cells are genomically modified to remove genes encoding for immunologically active cell surface antigens to render SRCs genomically modified and immunopriveleged in a way to reduce rejection by the recipient. In certain embodiments, cells are genetically modified to reduce the expression of genes (*e.g.,* using RNAi) encoding for immunologically active cell surface antigens to render SRCs genetically modified and immunoprivileged in a way to reduce rejection by the recipient.

Combining bioactive renal cells that exhibit a buoyant density greater than approximately 1.0419 g/mL from the density gradient centrifugation step, are used to produce therapeutically bioactive SRC. The preparation of selected renal cells is also described, for example, in Kelley et al. (Am J Physiol Renal Physiol 2010; 299: F1026-39), Kelley et al. (Cell Transplant 2013; 22:1023-39), Bruce et al. (Methods Mol Biol. 2013; 1001:53-64) and Basu et al. (Cell Transplant. 2011; 20:1171-901). Cell suspensions (100µL) are loaded into a 10cc syringe mixed with gelatin-based hydrogel biomaterial to formulate the therapeutic product.

Cell viability is measured at each culture passage and during formulation (Trypan Blue Exclusion). Assays of cell phenotype and potency are performed on the final product as previously described (Basu and Ludlow. Regen Med 2014; 9:497-512). All procedures are conducted in compliance with current Good Manufacturing Practices (cGMP) under the guidance of FDA and MPA QP.

### Example 3: General method to engineer human allogeneic renal cells for the treatment of chronic kidney disease

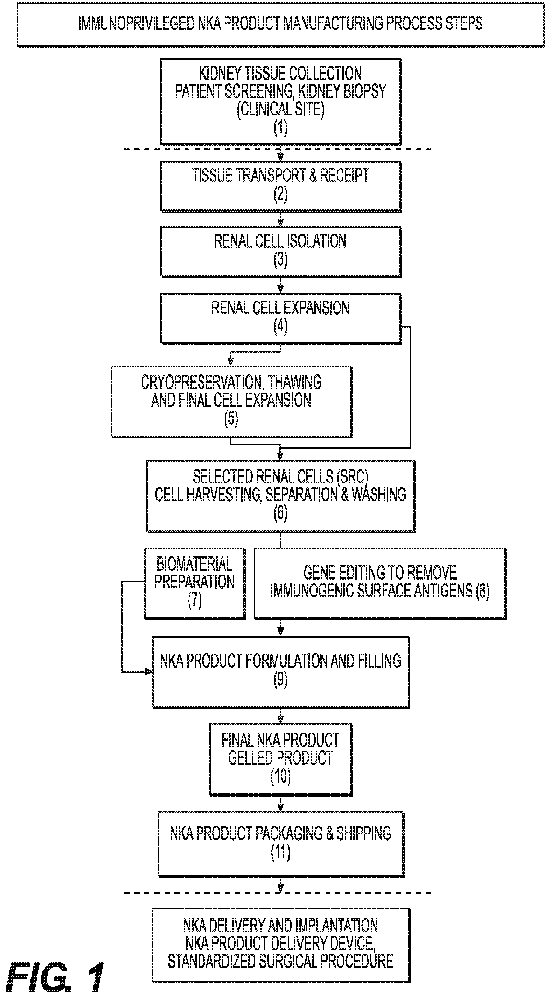

Treatment of patients using selected renal cells according to the present invention is based on using genomically modified immunoprivileged cells lacking surface antigens that render the cells capable of rejection . In certain embodiments, bioactive renal cells are recovered from the patient or a donor, selected ex vivo, cultivated in vitro in order to amplify the number of cells if necessary and finally infused into the patient. Each patient receives a treatment prepared using the genomically modified immunoprivileged bioactive renal cells that lack surface antigens leading to rejection renal cells (i.e. an immunopriveleged bioactive renal cell therapy). For a better understanding of the invention, a schematic of the manufacturing process steps for the immunoprivileged NKA product is illustrated in FIG. 1.

To increase the level of matching between a potential donor having an unsuitable level of HLA matching to a recipient targeted allele-specific gene editing is performed using Cas9 and specifically-identified gRNAs. As a result, the level of HLA matching between cells from the mismatched donor are made suitable (by reducing HLA mismatch) for transfer to a potential recipient patient through gene disruption. Multiplex HLA (MHC Class I and Class II) gene editing of primary human renal cells or enriched SRC from a potential allogeneic donor and recipient pair can be used to increase matching of donor SRC to recipient.

To decrease the likelihood of rejection of a transplanted HLA-mismatched allogeneic cell (e.g., bioactive renal cells), a recipient subject requiring transplantation is HLA typed (e.g., HLA- A, HLA-B and HLA- DRB 1 polymorphisms are determined) at the 6 HLA alleles (2 alleles each at HLA-A, HLA-B and HLA-DRB1). Ideally, the recipient genotype is matched with a donor having the same 6/6 HLA alleles since a 6/6 HLA allele match is associated with a reduced risk of immune rejection after transplantation. If no donor having a 6/6 allele match is available (e.g., from a bone marrow or cord blood HSC donor registry, or a related family member), but partially-matched donors having a 5/6, 4/6, 3/6 or 2/6 HLA allele match are available, the methods described herein may be used to reduce mismatching between the partially matched donor and recipient. As necessary, a single allele or multiple alleles (two, three, four, five, or six alleles) may be disrupted using the gene editing methods described herein to reduce the risk of developing immunosuppression and/or the severity of disease in the transplantation recipient.

The methods described herein may be used to modify donor renal cells (e.g., BRC and/or SRC) to generate immunoprivileged renal cells. For example, the methods may be used to disrupt (e.g., knockout) 1, 2 or 3 HLA alleles in a donor SRC to generate a cells matching HLA genotypes most frequently present in particular populations. For example, the most common 10 haplotypes for four ethnic groups in North America can be found, for example, in the National Marrow Donor Program HLA haplotype frequency data, available at https://bioinformatics.bethematchclinical.org/hla-resources/haplotype-frequencies/; Burdett et al. , Hum. Immunol. 64 (10 Suppl): S6 (2003)).

For each gRNA, a single oligonucleotide is designed and obtained. The U6 promoter and the gRNA scaffold (e.g. including everything except the targeting domain, e.g., including sequences derived from the crRNA and tracrRNA, e.g., including a first complementarity domain; a linking domain; a second complementarity domain; a proximal domain; and a tail domain) are separately PCR amplified and purified as dsDNA molecules. The gRNA-specific oligonucleotide is used in a PCR reaction to stitch together the U6 and the gRNA scaffold, linked by the targeting domain specified in the oligonucleotide. Resulting dsDNA molecule is purified for transfection. Alternate promoters may be used to drive in vivo transcription or for in vitro transcription. Any gRNA scaffold may be used to create gRNAs compatible with Cas9s from any bacterial species.

Each gRNA to be tested is transfected, along with a plasmid expressing Cas9 and a small amount of a GFP-expressing plasmid into human cells. In preliminary experiments, these cells can be immortalized human cell lines such as 293T, K562, or U20S. Alternatively, primary human cells may be used. In this case, cells may be relevant to the eventual therapeutic cell target (for example, an erythroid cell). The use of primary cells similar to the potential therapeutic target cell population may provide important information on gene targeting rates in the context of endogenous chromatin and gene expression.

Transfection may be performed using lipid transfection (such as Lipofectamine or Fugene) or by electroporation (such as Lonza Nucleofection^{™}). Following transfection, GFP expression can be determined either by fluorescence microscopy or by flow cytometry to confirm consistent and high levels of transfection. These preliminary transfections can comprise different gRNAs and different targeting approaches (17-mers, 20-mers, nuclease, dual-nickase, etc.) to determine which gRNAs/combinations of gRNAs give the greatest activity.

Efficiency of cleavage with each gRNA may be assessed by measuring NHEJ-induced indel formation at the target locus by a T7El-type assay or by sequencing. Alternatively, other mismatch- sensitive enzymes, such as Cell/Surveyor nuclease, may also be used.

For the T7E1 assay, PCR amplicons are approximately 500-700bp with the intended cut site placed asymmetrically in the amplicon. Following amplification, purification and size- verification of PCR products, DNA is denatured and re-hybridized by heating to 95°C and then slowly cooling. Hybridized PCR products are then digested with T7 Endonuclease I (or other mismatch-sensitive enzyme) that recognizes and cleaves non-perfectly matched DNA. If indels are present in the original template DNA, when the amplicons are denatured and re-annealed, this results in the hybridization of DNA strands harboring different indels and therefore lead to double-stranded DNA that is not perfectly matched. Digestion products may be visualized by gel electrophoresis or by capillary electrophoresis. The fraction of DNA that is cleaved (density of cleavage products divided by the density of cleaved and uncleaved) may be used to estimate a percent NHEJ using the following equation: %NHEJ = (1-(1 - fraction cleaved)'72). The T7E1 assay is sensitive down to about 2-5% NHEJ.

Sequencing may be used instead of, or in addition to, the T7E1 assay. For Sanger sequencing, purified PCR amplicons are cloned into a plasmid backbone, transformed, miniprepped and sequenced with a single primer. Sanger sequencing may be used for determining the exact nature of indels after determining the NHEJ rate by T7E1. Sequencing may also be performed using next generation sequencing techniques. When using next generation sequencing, amplicons may be 300-500bp with the intended cut site placed asymmetrically. Following PCR, next generation sequencing adapters and barcodes (for example Illumina multiplex adapters and indexes) may be added to the ends of the amplicon, e.g., for use in high throughput sequencing (for example on an Illumina MiSeq). This method allows for detection of very low NHEJ rates.

While the methods described herein are exemplified for identifying gRNAs for use with a CRISPR/Cas9 molecule, it will be readily apparent to any person skilled in the art that the gRNA identification methods described herein may be used to identify and select sequences that can be used with other nucleases (e.g., TALEN, Cpfl, and zinc finger nucleases). Various modifications to the example embodiments will be readily apparent to those skilled in the art, and the generic principles defined herein may be applied to other embodiments and applications without departing from the spirit and scope of the invention. Moreover, in the following description, numerous details are set forth for the purpose of explanation. However, one of ordinary skill in the art will realize that the invention may be practiced without the use of these specific details.

### Example 4: Immunogenicity and Potency Studies with genomically modified immunoprivileged SRC

In a first animal study genomically modified immunoprivileged SRC from a wild type rat are injected into the kidney of mutated Sprague Dawley (SD) rats with polycystic kidney disease (PCK). The primary end point to be evaluated is the number of wild type genomically modified immunoprivileged SRC that remain in the mutated kidney nephrons. The PCK SD rats having chronic kidney disease (CKD ) and injected with wild-type syngeneic SD rat SRC, will be evaluated using in situ hybridization and PCR of tissues, histological methods for qualitative characterization of localization and at the molecular level to quantify the extent of integration and retention of wild-type into diseased nephrons.

In a second study, human SRC gene editing will be evaluated by in vitro analysis using 4 potency assays to assess SRC function when genes controlling MHC class I, Class II, or Class I and II are removed. CRISPR/Cas9 will be introduced via electroporation and the SRC will be studied for chemotaxis affective, tubule formation, cytokine release and chemotaxis effective.

### Example 5: Suppression of MHC in SRCs

CRISPR/Cas9 allows for specific genome disruption and replacement in a flexible and simple system resulting in high specificity and low cell toxicity. The CRISPR/Cas9 genome editing system requires the co-expression of a Cas9 protein with a guide RNA vector expressed from the human U6 polymerase III promoter. With the protospacer-adjacent motif (PAM - the sequence NGG) present at the 3' end, Cas9 will unwind the DNA duplex and cleave both strands upon recognition of a target sequence by the guide RNA. The functional cassette synthesized in the rescue donor vector can then be inserted into the unwound DNA. The repaired genome will now express the desired sequence with or without tags. CRISPR was originally employed to "knock-out" target genes in various cell types and organisms, but modifications to the Cas9 enzyme have extended the application of CRISPR to selectively activate or repress target genes, purify specific regions of DNA, and even image DNA in live cells using fluorescence microscopy.

We chose to use CRISPR/Cas-9 gene editing for targeting the B2M or HLA-A (a subunit) component of MHC-I. Epigenomic modification at the targeted genomic locus may modify accessibility of the targeted DNA site to the cas protein. We have found that guide RNAs successfully knocking down expression of the B2M gene target near the transcription start site of the gene in a region of open chromatin characterized by extensive methylation of the H3K4 residue of the nucleosome. Publicly available software (CHOPCHOP, WU-CRISPR) was leveraged to design CRISPR targeting constructs (guide RNAs binding defined DNA sequences) and electroporation was used to transfect kidney cells. The target DNA sequence for the B2M gene that was successfully targeted to create FACS data is as follows:

### Target DNA Sequence

GAGTAGCGCGAGCACAGCTA (SEQ ID NO: 346)

### PAM Sequence

AGG

### Target locus

Chr.15: 44711563 - 44711585 on GRCh38 (*see* NM_004048.2 at chr15:45003685-45010357 on the University of California Santa Cruz genome browser genome assembly GRCh37/hg19)

### Strand

### Reverse

Electroporation was used to introduce the CRISPR/Cas-9 constructs into primary human kidney cells. Other methods including lipofection, transfection, micro-injection etc. known to those of skill in the art may be applied to deliver CRISPR/Cas-9 constructs. Although the results presented below focus on the B2M sub-unit, similar approaches may be applied to target the a polypeptide (HLA-A).

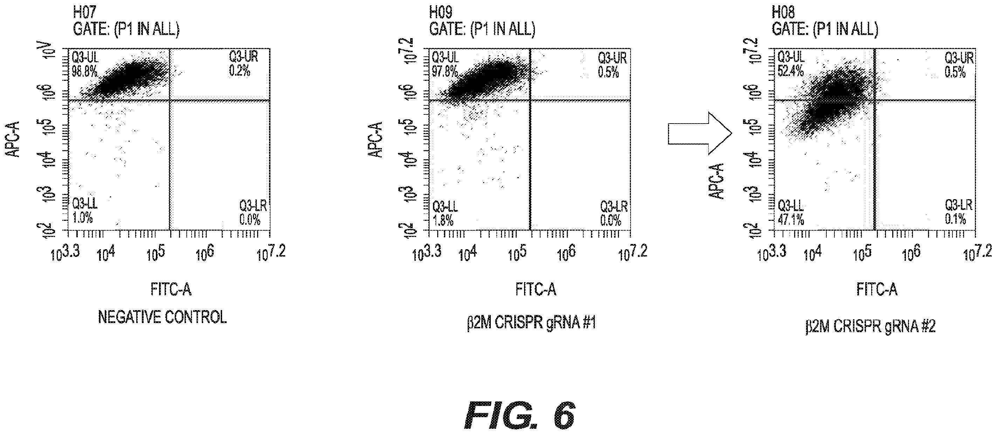

Human primary kidney cells (1×10⁶) were electroporated with a CRISPR/Cas9 construct specifically targeting the B2M component of the MHC-I complex. All work for FIGs. 6-8 was performed using human primary kidney cells cultured after enzymatic digestion of kidney tissue. These cells were not processed to obtain SRCs. Cells were allowed to recover 48 hours post-electroporation. Expression of B2M was evaluated by FACS. Electroporation was performed using a Gene pulser Xcell ^{™} electroporation system (Bio-Rad Laboratories, Inc., Hercules, California, USA), preset program #1: square wave pulse type, for 5 milliseconds, at 160volts, in a 0.2cm cuvette, 100µL total volume.

Separately, human primary kidney cells (1×10⁶) were electroporated with a CRISPR/Cas9 construct specifically targeting the B2M component of the MHC-I complex. Cells were allowed to recover 5 days post-electroporation. Expression of B2M was evaluated by FACS.

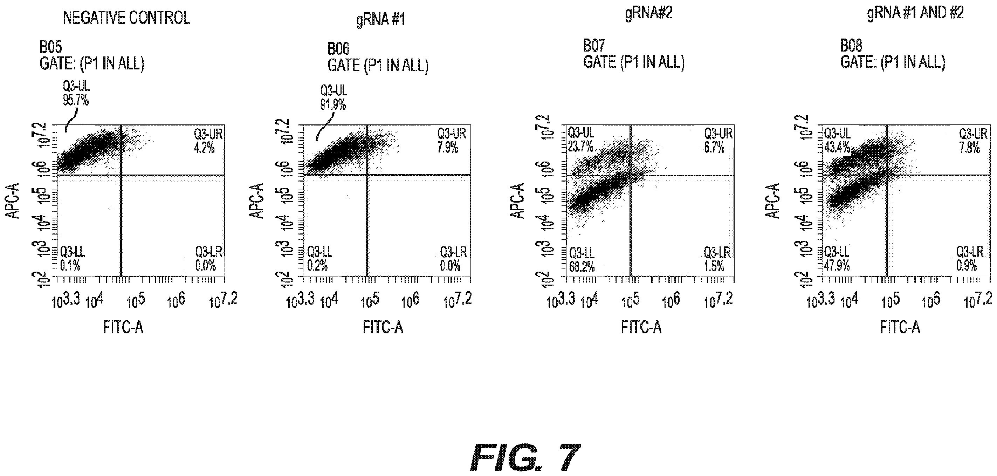

As shown in FIG. 6, we were able to decrease B2M expression by 47%, as determined by FACs 48 hr post-electroporation. A similar reduction was observed 5-days post electroporation, indicating that the transfection is stable. *See* FIG. 7.

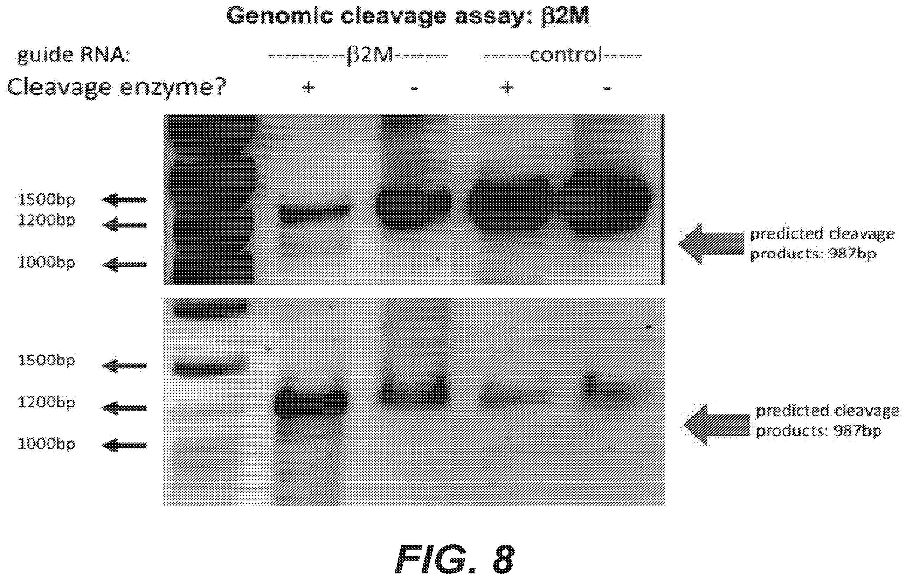

Position-wise, molecular analysis indicated that the B2M knock-out occurred at the correct, B2M locus. *See* FIG. 8. This is important for the safety of the cell product.

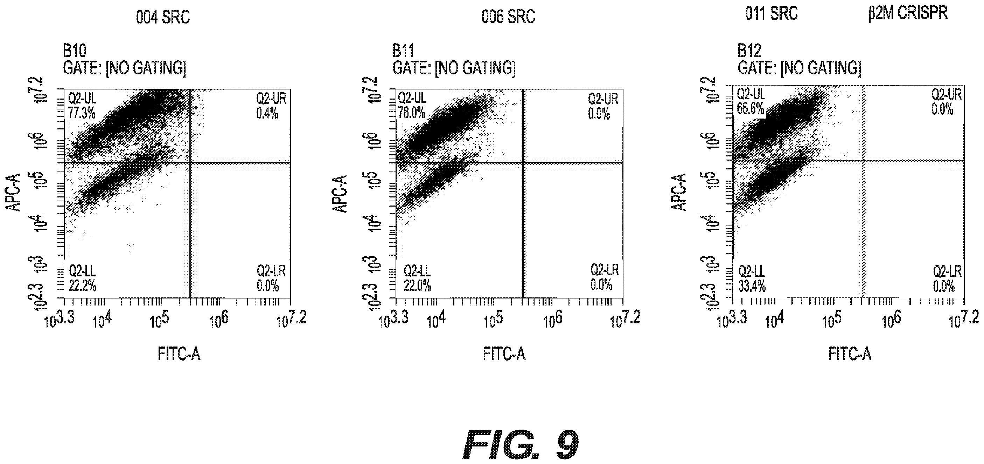

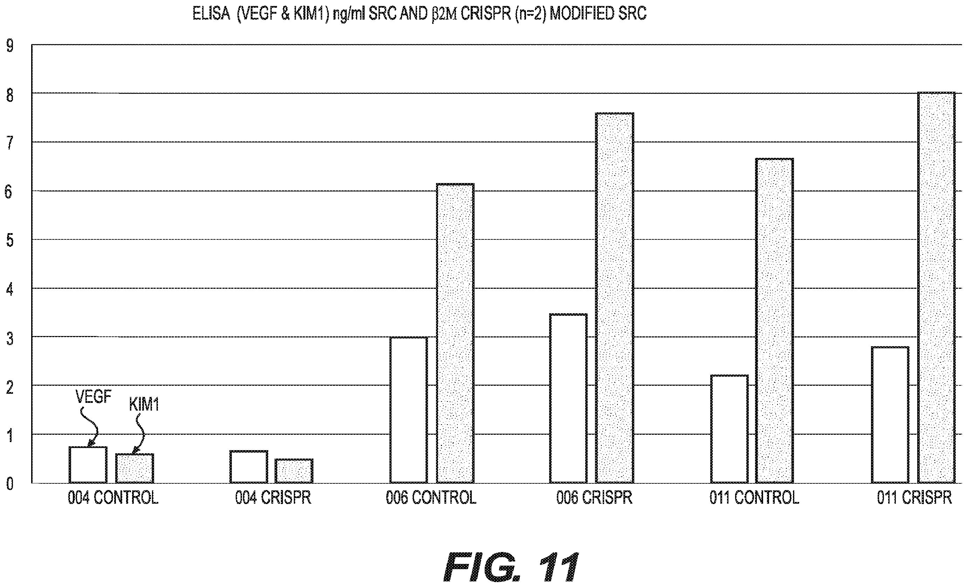

Regardless of the SRC donor-cell ABO haplotype, this approach successfully knock-out B2M, without affecting SRC function, as determined by VEGF and KIM1 secretion, which are potency assays for SRC. *See* FIGs. 9 and 11.

Mixed lymphocyte reaction (MLR) is a test used by pharmaceutical and biotech organizations to show the safety of a drug or implantable material. It is commonly used as part of the U.S. Food and Drug Administration (FDA) clearance process. Technically, it is an ex-vivo cellular immune assay that occurs between two allogeneic lymphocyte populations (same species but genetically distinct). In a one-way MLR, only one lymphocyte population can respond or proliferate. In a two-way MLR, both populations can proliferate. MLR's are performed to assess how T-cells react to external stimuli. T cells are a type of white blood cell that scans for cellular abnormalities and infections. They are essential to human immunity.

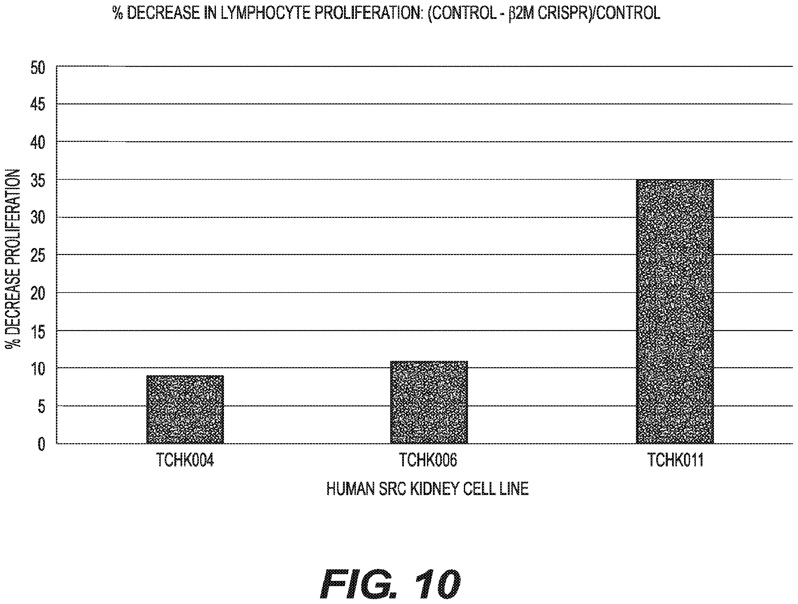

We performed a one-way MLR using control and CRISPER gene edited cells as the stimulator population. If B2M expression is successfully knocked-out or knocked-down, we would predict that there should be a reduction in lymphocyte cell proliferation. As shown in FIG. 10, testing 3 different SRC preps, each having a different level of B2M downregulation based on FACS, we observed a concomitant decrease in lymphocyte proliferation.

We have a system whereby we can demonstrate a tuned degree of gene knock-out and a correlation with an *in vitro* immune response. In so doing, a minimum to no use of immunosuppressive drugs, which have their own deleterious effects on the body, will be needed for recipients of this treatment.

### Example 6: Exemplary NKA Formulation Components

### 1. Cellular Components and Materials

SRC constitute the biologically active component of NKA. SRC are composed primarily of renal tubular epithelial cells that are well known for their regenerative potential. Other parenchymal (vascular), mesenchymal, endothelial and stromal (collecting duct) cells may be present in the autologous SRC population.

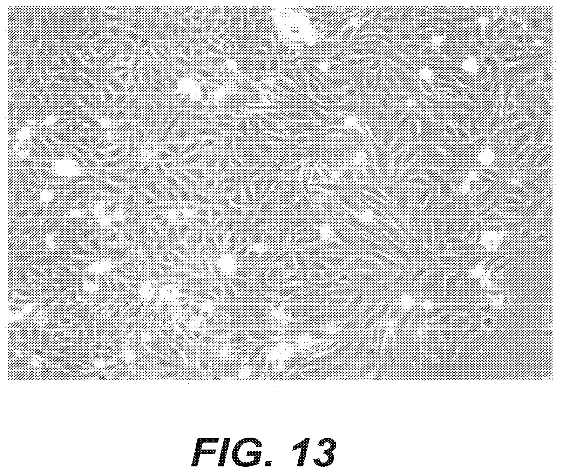

SRC are prepared from renal cortical tissue obtained using a standard-of-clinical-care kidney biopsy procedure to collect cores of kidney tissue. Renal cells are isolated from the kidney tissue by enzymatic digestion and expanded using standard cell culture techniques. Cells are assessed to verify renal cell morphology by visual observation of cultures under the microscope. Cultures characteristically demonstrate a tight pavement or cobblestone appearance, due to the cells clustering together (FIG. 13). SRC are obtained by separation of the isolated and expanded cells across a density boundary or density interface or single step discontinuous density gradient.

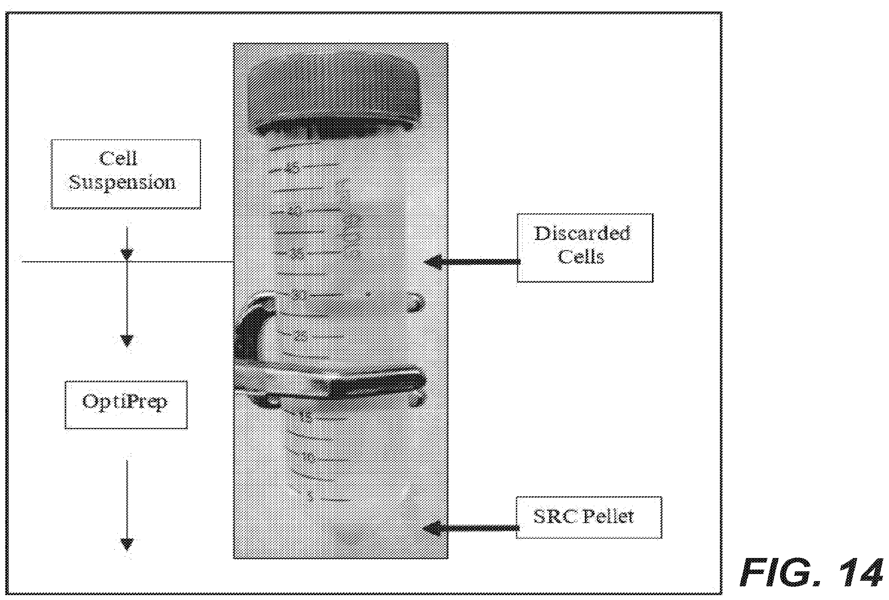

Centrifugation across a density boundary or interface is used to separate harvested renal cell populations based on cell buoyant density. Renal cell suspensions are separated over a solution of OptiPrep (7% iodixanol; 60% (w/v) in OptiMEM) medium. The cellular fraction exhibiting buoyant density greater than approximately 1.0419 g/mL is collected after centrifugation as a distinct pellet (FIG. 14). Cells maintaining a buoyant density of less than 1.0419 g/mL are excluded and discarded.

The SRC pellet is re-suspended in DPBS. The carry-over of residual OptiPrep, FBS, culture medium and ancillary materials in the final product is minimized by washing steps.

### 2. Biomaterial Components and Ancillary Materials

The following biomaterial components and ancillary materials are used for formulation of SRC into NKA:
1. Porcine gelatin - used to make the thermally responsive hydrogel.
2. Dulbecco's phosphate-buffered saline (DPBS) - used to dissolve the porcine gelatin. The buffer may be replaced or mixed with human plasma or human platelet lysate.

### Biomaterial Preparation

The biomaterial is a Gelatin Solution composed of porcine gelatin in DPBS. Gelatin is dissolved in DPBS or human plasma/human platelet lysate or a mixture of both to a specified concentration to form a Gelatin Solution of a thermally responsive hydrogel. The Gelatin Solution is filter sterilized through a 0.1 µm filter and stored refrigerated or frozen in single use aliquots ready for formulation.

The key property of the biomaterial is that it is a thermally responsive hydrogel such that it can gel and liquefy at different temperatures. Gelatin Solution used in NKA formulation is liquid at and above room temperature (22-28°C) and gels when cooled to refrigerated temperatures (2-8°C).

### Gelatin Solution Concentration

Gelatin concentration in the range of 0.5-1.0% was evaluated for gelation properties - ability to form a gel at refrigerated temperature (no flow when inverted) and to become fluid at room temperature (free flowing when inverted). Table 9 shows gelation properties of different concentration of Gelatin Solution.

**Table 9 - Gelation Properties of Gelatin Solution at Different Concentrations**

| **Gelatin Concentration** | **Gel (Refrigerated Temperature)** | **Flow (Room Temperature)** |
|---|---|---|
| 0.50% | +/- | + |
| 0.63% | + | + |
| 0.75% | + | + |
| 0.88% | + | + |
| 1.00% | + | + |

Since NKA formulated with gelatin solution of 0.63% and above were able to consistently meet the acceptance criteria, a range of 0.88 ± 0.12% was selected for gelatin concentration for NKA formulation. It is noted, however, that formulations comprising gelatin in the concentration range of about 0.63% to about 1% are also suitable.

### 3. NKA Formulation

SRC are formulated into NKA with Gelatin Solution, a gelatin-based thermally responsive hydrogel. The gelatin-based thermally responsive hydrogel provides improved stability of the cells thus extending product shelf life, stability during transport and delivery of SRC into the kidney cortex for clinical utility. Formulation development assessed composition, concentration and stability of Gelatin Solution.

Washed SRC are counted using Trypan Blue dye exclusion. Gelatin Solution is removed from cold storage and liquefied by warming to 26-30°C. A volume of SRC suspension containing the required number of cells is centrifuged and re-suspended in liquefied Gelatin Solution for a final wash step. This suspension is centrifuged and the SRC pellet is re-suspended in sufficient Gelatin Solution to achieve a resultant SRC concentration of 100×10⁶ cells/mL in the formulated NKA.

### NKA Filling and Gelation

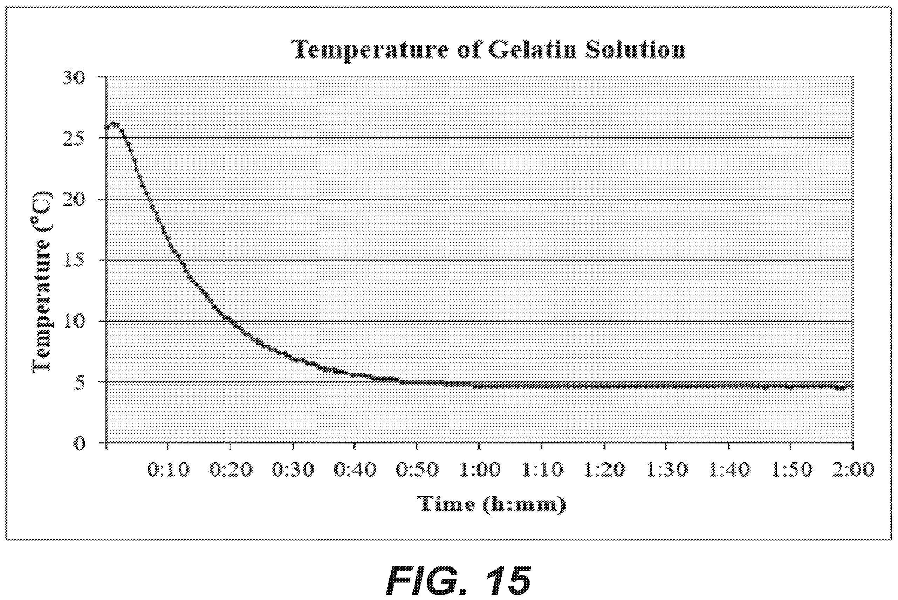

NKA product is aseptically filled into a syringe. Dynamic air sampling is performed for the duration of the filling process, including viable and non-viable sampling. The NKA package is rotated for a minimum of 2 hours to keep the cells in suspension while cooling to 2-8°C to form the final gelled NKA. Rapid cooling is required for gelation to take place so that cells do not settle in the Gelatin Solution. The temperature of the Gelatin Solution in a syringe was monitored as it was placed into refrigerated conditions. Rapid temperature drop is observed as shown in FIG. 15. After 1 hour, the temperature typically drops to within 0.3°C of the final temperature 4.4°C.

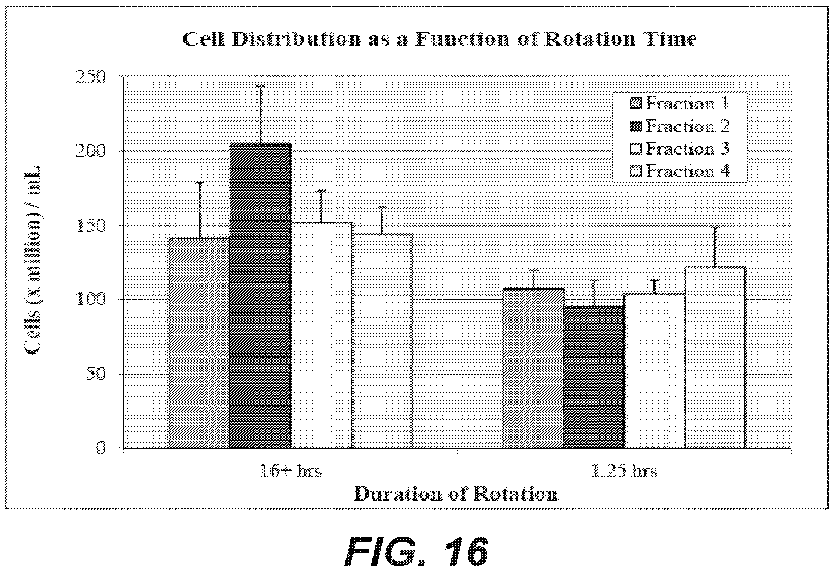

Cooling of the Gelatin Solution starts the gelation process but a finite amount to time is required for the formed gel to stabilize such that the SRC will remain suspended in the gel on storage. Syringes containing formulated NKA were rotated either overnight or for 1.25 hours and then held upright overnight. Subsequently, the contents were removed and cell concentration was measured in four different segments of the product. Analysis indicates that there is no difference among the four segments, thus no measurable cell settling occurs once NKA has rotated at cold temperature for a minimum of 1.25 hours (FIG. 16).

## Claims

1. A method of producing a genomically modified bioactive renal cell (BRC) comprising: genetically modifying at least one genomic immunogenicity gene in a BRC,
wherein the at least one gene encodes a protein within: a major histocompatibility complex (MHC) class I molecule, a MHC class II molecule, or a both a MHC class I and MHC class II molecule;
wherein genetically modifying the at least one gene reduces the amount of functional MHC class I, or MHC class II, or MHC class I and MHC class II on the surface of the cell; and
wherein the BRC is of a BRC population, wherein the BRC population is prepared from a kidney tissue; is hypoxia-resistant; and comprises tubule cells, glomerular cells, vascular cells and cells of the corticomedullary junction.

2. The method of claim 1, wherein the at least one gene comprises one or more of a B2M, HLA-A, HLA-B, HLA-C, HLA-DRA, HLA-DRB1, HLA-DRB3, HLA-DRB4, HLA-DRB5, HLA-DPA1, HLA-DPA2, HLA-DQA1, or HLA-DQB1 gene.

3. The method of claim 1 or 2, wherein genetically modifying the at least one gene comprises mutating the at least one gene.

4. The method of claim 3, wherein mutating the at least one gene comprises deleting the at least one gene or a portion thereof.

5. The method of any of claims 1-4, wherein the BRC is a selected renal cell (SRC), wherein the SRC is of a SRC population that: is selected from the BRC population by density gradient separation; comprises cells of a buoyant density greater than about 1.04 g/mL; and comprises cells that express CK18.

6. The method of claim 5, wherein the SRC is a tubule cell.

7. The method of claim 6, wherein the tubule cell is a tubule epithelial cell.

8. The method of claim 5, wherein the SRC population is further **characterized in that** it comprises:
iodixanol-resistant cells, or
cells that express hyaluronic synthase-2, or
cells that are capable of receptor-mediated albumin transport, or
CK18 and GGT1 expressing cells.

9. The method of any of claims 1-5, wherein genetically modifying the at least one gene comprises (i) expressing a gene editing protein in the BRC; or (ii) delivering a gene editing protein across the cell membrane of the BRC.

10. The method of claim 9, wherein the gene editing protein is a zinc finger nuclease (ZFN), a transcription activator-like effector nuclease (TALEN), a megaTAL, or an RNA-guided endonuclease.

11. The method of claim 10, wherein the RNA-guided endonuclease is a Cas protein.

12. The method of claim 11, wherein the Cas protein is a Cas9 protein.

13. The method of claim 10, wherein the gene editing protein is:
expressed from transfected mRNA; or
a recombinant protein and is complexed with a gRNA *ex vivo*; or
a recombinant protein and is complexed with a gRNA *ex vivo* and the protein/gRNA complex is delivered to the cell by transfection, lipfection, electroporation, or microinjection.

14. The method of claim 11 or claim 12, wherein the Cas protein is expressed from a DNA vector.

15. The method of claim 14, wherein the expression of the Cas protein is induced during at least part of the time that the gRNA is expressed in the cell.

16. The method of claim 14, wherein the DNA vector does not integrate in the genome, is a episomal vector or artificial chromosome, or is a transposon.

17. The method of claim 5, wherein the SRC is a glomerular cell.

18. A population of genomically modified BRCs obtainable by the method according to claim 1.

19. The population as recited in claim 18 for use in treating a kidney disease in a patient, the method comprising administering the population to the patient.

20. An injectable formulation comprising:
a) a temperature-sensitive cell-stabilizing biomaterial, and
b) the population of claim 18,
wherein the temperature-sensitive cell-stabilizing biomaterial is a hydrogel that
(i) maintains a substantially solid state at about 8°C or below, wherein the substantially solid state is a gel state,
(ii) maintains a substantially liquid state at about ambient temperature or above, and
(iii) has a solid-to-liquid transitional state between about 8°C and about ambient temperature or above.

## Patentansprüche

1. Verfahren zur Herstellung einer genomisch modifizierten bioaktiven Nierenzelle (BRC), umfassend:
genetisches Modifizieren von zumindest einem genomischen Immunogenitätsgen in einer BRC,
wobei das zumindest eine Gen für ein Protein innerhalb: eines Moleküls des Haupthistokompatibilitätskomplexes (MHC) Klasse I, eines MHC-Klasse-II-Moleküls oder sowohl eines MHC-Klasse-I- als auch eines MHC-Klasse-II-Moleküls kodiert;
wobei das genetische Modifizieren des zumindest einen Gens die Menge von funktionellem MHC Klasse I oder MHC Klasse II oder MHC Klasse I und MHC Klasse II auf der Oberfläche der Zelle reduziert; und
wobei die BRC aus einer BRC-Population stammt, wobei die BRC-Population aus Nierengewebe hergestellt ist; Hypoxie-resistent ist und Tubulus-Zellen, glomeruläre Zellen, vaskuläre Zellen und Zellen des kortikomedullären Übergangs umfasst.

2. Verfahren nach Anspruch 1, wobei das zumindest eine Gen ein oder mehrere aus einem B2M-, HLA-A-, HLA-B-, HLA-C-, HLA-DRA-, HLA-DRB1-, HLA-DRB3-, HLA-DRB4-, HLA-DRB5-, HLA-DPA1-, HLA-DPA2-, HLA-DQA1- oder HLA-DQB1-Gen umfasst.

3. Verfahren nach Anspruch 1 oder 2, wobei das genetische Modifizieren des zumindest einen Gens das Mutieren des zumindest einen Gens umfasst.

4. Verfahren nach Anspruch 3, wobei das Mutieren des zumindest einen Gens das Deletieren des zumindest einen Gens oder eines Teils davon umfasst.

5. Verfahren nach einem der Ansprüche 1 bis 4, wobei die BRC eine selektierte Nierenzelle (SRC) ist, wobei die SRC aus einer SRC-Population stammt, die: durch Dichtegradiententrennung aus der BRC-Population ausgewählt ist; Zellen mit einer Schwimmdichte von mehr als etwa 1,04 g/ml umfasst; und Zellen umfasst, die CK18 exprimieren.

6. Verfahren nach Anspruch 5, wobei die SRC eine Tubulus-Zelle ist.

7. Verfahren nach Anspruch 6, wobei die Tubulus-Zelle eine Tubulus-Epithelzelle ist.

8. Verfahren nach Anspruch 5, wobei die SRC-Population weiters **dadurch gekennzeichnet ist, dass** sie Folgendes umfasst:
lodixanol-resistente Zellen oder
Zellen, die Hyaluronsynthase-2 exprimieren, oder
Zellen, die zum Rezeptor-vermittelten Albumintransport in der Lage sind, oder
CK18- und GGT1-exprimierende Zellen.

9. Verfahren nach einem der Ansprüche 1 bis 5, wobei das genetische Modifizieren des zumindest einen Gens (i) das Exprimieren eines Genbearbeitungsproteins in der BRC; oder (ii) das Transportieren eines Genbearbeitungsproteins durch die Zellmembran der BRC umfasst.

10. Verfahren nach Anspruch 9, wobei das Genbearbeitungsprotein eine Zinkfingernuklease (ZFN), eine Transkriptionsfaktor-ähnliche Effektornuklease (TALEN), eine MegaTAL oder eine RNA-gelenkte Endonuklease ist.

11. Verfahren nach Anspruch 10, wobei die RNA-gelenkte Endonuklease ein Cas-Protein ist.

12. Verfahren nach Anspruch 11, wobei das Cas-Protein ein Cas9-Protein ist.

13. Verfahren nach Anspruch 10, wobei das Genbearbeitungsprotein Folgendes ist:
aus transfizierter mRNA exprimiert; oder
ein rekombinantes Protein und ex vivo mit einer gRNA komplexiert ist; oder
ein rekombinantes Protein und ex vivo mit einer gRNA komplexiert ist und der Protein/gRNA-Komplex der Zelle durch Transfektion, Lipofektion, Elektroporation oder Mikroinjektion zugeführt ist.

14. Verfahren nach Anspruch 11 oder Anspruch 12, wobei das Cas-Protein aus einem DNA-Vektor exprimiert wird.

15. Verfahren nach Anspruch 14, wobei die Expression des Cas-Proteins während zumindest eines Teils der Zeit ausgelöst wird, während der die gRNA in der Zelle exprimiert wird.

16. Verfahren nach Anspruch 14, wobei der DNA-Vektor sich nicht in das Genom integriert, ein episomaler Vektor oder ein künstliches Chromosom ist oder ein Transposon ist.

17. Verfahren nach Anspruch 5, wobei die SRC eine glomeruläre Zelle ist.

18. Population von genomisch modifizierten BRC, die durch ein Verfahren nach Anspruch 1 erhältlich sind.

19. Population nach Anspruch 18 zur Verwendung bei der Behandlung einer Nierenerkrankung in einem Patienten, wobei das Verfahren das Verabreichen der Population an den Patienten umfasst.

20. Injizierbare Formulierung, umfassend:
a) ein temperaturempfindliches zellstabilisierendes Biomaterial; und
b) eine Population nach Anspruch 18,
wobei das temperaturempfindliche zellstabilisierende Biomaterial ein Hydrogel ist, das:
(i) bei etwa 8 °C oder darunter einen im Wesentlichen festen Zustand beibehält, wobei der im Wesentlichen feste Zustand ein Gelzustand ist,
(ii) bei etwa Umgebungstemperatur oder darüber einen im Wesentlichen flüssigen Zustand beibehält und
(iii) zwischen etwa 8 °C und etwa Umgebungstemperatur oder darüber einen Fest-zu-Flüssig-Übergangszustand aufweist.

## Revendications

1. Procédé de production d'une cellule rénale bioactive (BRC) modifiée sur le plan génomique comprenant : la modification génétique d'au moins un gène d'immunogénicité génomique dans une BRC,
dans lequel ledit au moins un gène code pour une protéine dans : une molécule du complexe majeur d'histocompatibilité (MHC) de classe I, une molécule du MHC de classe II, ou à la fois une molécule du MHC de classe I et du MHC de classe II ;
dans lequel la modification génétique dudit au moins un gène réduit la quantité du MHC de classe I, ou du MHC de classe II, ou du MHC de classe I et du MHC de classe II fonctionnels sur la surface de la cellule; et
dans lequel la BRC est issue d'une population de BRC, dans lequel la population de BRC est préparée à partir d'un tissu rénal ; est résistante à une hypoxie ; et comprend des cellules tubulaires, des cellules glomérulaires, des cellules vasculaires et des cellules de la jonction cortico-médullaire.

2. Procédé selon la revendication 1, dans lequel ledit au moins un gène comprend un ou plusieurs gènes parmi un gène B2M, HLA-A, HLA-B, HLA-C, HLA-DRA, HLA-DRB1, HLA-DRB3, HLA-DRB4, HLA-DRB5, HLA-DPA1, HLA-DPA2, HLA-DQA1, ou HLA-DQB1.

3. Procédé selon la revendication 1 ou 2, dans lequel la modification génétique dudit au moins un gène comprend une mutation dudit au moins un gène.

4. Procédé selon la revendication 3, dans lequel la mutation dudit au moins un gène comprend la suppression dudit au moins un gène ou d'une partie de celui-ci.

5. Procédé selon l'une quelconque des revendications 1 à 4, dans lequel la BRC est une cellule rénale sélectionnée (SRC), dans lequel la SRC est issue d'une population de SRC qui : est sélectionnée parmi la population de BRC par séparation par gradient de densité ; comprend des cellules d'une densité de flottaison supérieure à environ 1,04 g/mL ; et comprend des cellules qui expriment CK18.

6. Procédé selon la revendication 5, dans lequel la SRC est une cellule tubulaire.

7. Procédé selon la revendication 6, dans lequel la cellule tubulaire est une cellule épithéliale tubulaire.

8. Procédé selon la revendication 5, dans lequel la population de SRC est en outre **caractérisée en ce qu'**elle comprend :
des cellules résistantes à l'iodixanol, ou
des cellules qui expriment la synthase-2 hyaluronique, ou
des cellules qui sont capables d'un transport d'albumine médié par récepteur, ou
des cellules exprimant CK18 et GGT1.

9. Procédé selon l'une quelconque des revendications 1 à 5, dans lequel la modification génétique dudit au moins un gène comprend (i) l'expression d'une protéine d'édition de gènes dans la BRC ; ou (ii) la libération d'une protéine d'édition de gènes à travers la membrane cellulaire de la BRC.

10. Procédé selon la revendication 9, dans lequel la protéine d'édition de gènes est une nucléase à doigts de zinc (ZFN), une nucléase effectrice de type activateur de transcription (TALEN), une mégaTAL, ou une endonucléase guidée par ARN.

11. Procédé selon la revendication 10, dans lequel l'endonucléase guidée par ARN est une protéine Cas.

12. Procédé selon la revendication 11, dans lequel la protéine Cas est une protéine Cas9.

13. Procédé selon la revendication 10, dans lequel la protéine d'édition de gènes est :
exprimée à partir d'ARNm transfecté ; ou
une protéine recombinante et est complexée avec un ARNg *ex vivo* ; ou
une protéine recombinante et est complexée avec un ARNg *ex vivo* et le complexe protéine/ARNg est délivré dans la cellule par transfection, lipofection, électroporation, ou micro-injection.

14. Procédé selon la revendication 11 ou 12, dans lequel la protéine Cas est exprimée à partir d'un vecteur d'ADN.

15. Procédé selon la revendication 14, dans lequel l'expression de la protéine Cas est induite pendant au moins une partie du temps pendant lequel l'ARNg est exprimé dans la cellule.

16. Procédé selon la revendication 14, dans lequel le vecteur d'ADN ne s'intègre pas dans le génome, est un vecteur épisomal ou un chromosome artificiel, ou est un transposon.

17. Procédé selon la revendication 5, dans lequel la SRC est une cellule glomérulaire.

18. Population de BRC génomiquement modifiées pouvant être obtenue par le procédé selon la revendication 1.

19. Population selon la revendication 18 pour une utilisation dans le traitement d'une maladie rénale chez un patient, le procédé comprenant l'administration de la population au patient.

20. Formulation injectable comprenant :
a) un biomatériel stabilisateur de cellules sensible à la température, et
b) la population selon la revendication 18,
dans laquelle le biomatériel stabilisateur de cellules sensible à la température est un hydrogel qui
(i) maintient un état sensiblement solide à environ 8 °C ou moins, dans laquelle l'état sensiblement solide est un état de gel,
(ii) maintient un état sensiblement liquide environ à la température ambiante ou plus, et
(iii) a un état de transition solide-liquide entre environ 8 °C et environ la température ambiante ou plus.
